# EUROPEAN PATENT APPLICATION

(11) **EP 4 755 908 A2**
(43) Date of publication of application: **10.06.2026**
(21) Application number: 26158770.3
(22) Date of filing: 18.01.2018
(51) Int. Cl.: C07K 14/715

(54) **PEPTIDE INHIBITORS OF INTERLEUKIN-23 RECEPTOR AND THEIR USE TO TREAT INFLAMMATORY DISEASES**

(30) Priority: 18.01.2017 US 201762447778 P
(62) Divisional of application: 18741939.5
(71) Applicant: Protagonist Therapeutics, Inc., Newark, CA 94560-1160 (US)
(72) Inventor: BHANDARI, Ashok, Pleasanton, 94588 (US); BOURNE, Gregory, Moggill, 4070 (AU)
(74) Representative: Carpmaels & Ransford LLP

(57) **Abstract**

The present invention provides novel peptide inhibitors of the interleukin-23 receptor, and related compositions and methods of using these peptide inhibitors to treat or prevent a variety of diseases and disorders, including inflammatory bowel diseases.

## Description

### CROSS-REFERENCE TO RELATED APPLICATIONS

This application claims priority to U.S. Provisional Application No. 62/447,778, filed on January 18, 2017, which is incorporated by reference herein in its entirety.

### STATEMENT REGARDING SEQUENCE LISTING

The Sequence Listing associated with this application is provided in text format in lieu of a paper copy, and is hereby incorporated by reference into the specification. The name of the text file containing the Sequence Listing is PRTH_027_01WO_ST25.txt. The text file is 255 KB, was created on January 18, 2018, and is being submitted electronically via EFS-Web.

### FIELD OF THE INVENTION

The present invention relates to novel peptide inhibitors of the interleukin-23 receptor, and their use to treat or prevent a variety of diseases and disorders, including inflammatory bowel disease, Crohn's disease and psoriasis.

### BACKGROUND

The interleukin-23 (IL-23) cytokine has been implicated as playing a crucial role in the pathogenesis of autoimmune inflammation and related diseases and disorders, such as multiple sclerosis, asthma, rheumatoid arthritis, psoriasis, and inflammatory bowel diseases (IBDs), e.g., ulcerative colitis and Crohn's disease. Studies in acute and chronic mouse models of IBD revealed a primary role of IL-23R and downstream effector cytokines in disease pathogenesis. IL-23R is expressed on various adaptive and innate immune cells including Th17 cells, γδ T cells, natural killer (NK) cells, dendritic cells, macrophages, and innate lymphoid cells, which are found abundantly in the intestine. At the intestine mucosal surface, the gene expression and protein levels of IL-23R are found to be elevated in IBD patients. It is believed that IL-23 mediates this effect by promoting the development of a pathogenic CD4⁺ T cell population that produces IL-6, IL-17, and tumor necrosis factor (TNF).

Production of IL-23 is enriched in the intestine, where it is believed to play a key role in regulating the balance between tolerance and immunity through T-cell-dependent and T-cell-independent pathways of intestinal inflammation through effects on T-helper 1 (Th1) and Th17-associated cytokines, as well as restraining regulatory T-cell responses in the gut, favoring inflammation. In addition, polymorphisms in the IL-23 receptor (IL-23R) have been associated with susceptibility to IBDs, further establishing the critical role of the IL-23 pathway in intestinal homeostasis.

Psoriasis, a chronic skin disease affecting about 2%-3% of the general population has been shown to be mediated by the body's T cell inflammatory response mechanisms. Il-23 has one of several interleukins implicated as a key player in the pathogenesis of psoriasis, purportedly by maintaining chronic autoimmune inflammation via the induction of interleukin-17, regulation of T memory cells, and activation of macrophages. Expression of IL-23 and IL-23R has been shown to be increased in tissues of patients with psoriasis, and antibodies that neutralize IL-23 showed IL-23-dependent inhibition of psoriasis development in animal models of psoriasis.

IL-23 is a heterodimer composed of a unique p19 subunit and the p40 subunit of IL-12, which is a cytokine involved in the development of interferon-y (IFN-y)-producing T helper 1 (T_{H}1) cells. Although IL-23 and IL-12 both contain the p40 subunit, they have different phenotypic properties. For example, animals deficient in IL-12 are susceptible to inflammatory autoimmune diseases, whereas IL-23 deficient animals are resistant, presumably due to a reduced number of CD4⁺ T cells producing IL-6, IL-17, and TNF in the CNS of IL-23-deficient animals. IL-23 binds to IL-23R, which is a heterodimeric receptor composed of IL-12Rβ1 and IL-23R subunits. Binding of IL-23 to IL-23R activates the Jak-stat signaling molecules. Jak2, Tyk2, and Stat1. Stat 3, Stat 4, and Stat 5, although Stat4 activation is substantially weaker and different DNA-binding Stat complexes form in response to IL-23 as compared with IL-12. IL-23R associates constitutively with Jak2 and in a ligand-dependent manner with Stat3. In contrast to IL-12, which acts mainly on naive CD4(+) T cells, IL-23 preferentially acts on memory CD4(+) T cells.

Efforts have been made to identify therapeutic moieties that inhibit the IL-23 pathway, for use in treating IL-23-related diseases and disorders. A number of antibodies that bind to IL-23 or IL-23R have been identified, including ustekinumab, a humanized antibody that binds IL-23, which has been approved for the treatment of psoriasis. More recently, polypeptide inhibitors that bind to IL-23R and inhibit the binding of IL-23 to IL-23R have been identified (see, e.g., US Patent Application Publication No. US2013/0029907). Clinical trials in Crohn's Disease or psoriasis with ustekinumab and briakinumab (which target the common p40 subunit) and tildrakizumab, guselkumab, MEDI2070, and BI-655066 (which target the unique p19 subunit of IL-23) highlight the potential of IL-23 signaling blockade in treatment of human inflammatory diseases. While these findings are promising, challenges remain with respect to identifying stable and selective agents that preferentially target the IL-23 pathway in the intestine, which can be used for the treatment of intestinal inflammation, such as intestinal bowel diseases, including Crohn's disease, ulcerative colitis and related disorders.

Clearly, there remains a need in the art for new therapeutics targeting the IL-23 pathway, which may be used to treat and prevent IL-23-asociated diseases, including those associated with autoimmune inflammation in the intestinal tract. In addition, compounds and methods for specific targeting of IL-23R from the luminal side of the gut may provide therapeutic benefit to IBD patients suffering from local inflammation of the intestinal tissue. The present invention addresses these needs by providing novel peptide inhibitors that bind IL-23R to inhibit IL-23 binding and signaling and which are suitable for oral administration.

### BRIEF SUMMARY OF THE INVENTION

The present invention provides *inter alia* novel peptide inhibitors of IL-23R and related methods of use.

In a first aspect, the present invention provides a peptide inhibitor of an interleukin-23 receptor, or a pharmaceutically acceptable salt or solvate thereof, wherein the peptide inhibitor comprises or consists of an amino acid sequence of Formula (II): 
X0-X1-X2-X3-X4-X5-X6-X7-X8-X9-X10-X11-X12-X13-X14-X15-X16-X17-X18-X19-
X20-X21-X22-X23 (II) (SEQ ID NO:237),
 wherein:
X0 is Gly, Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, or absent;
X1 is Gly, Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, or absent;
X2 is (D)Asp, Arg, (D)Arg, Phe, (D)Phe, 2-Nal, Thr, Leu, (D)Gln, (D)Asn, IsoGlu, Gly, Arg, Phe, Glu, Gln, Thr, (D)Glu, (D)Thr, (D)Leu, alpha-MeArg, alpha-MePhe, alpha-MeLeu, alpha-MeLys, alpha-MeAsn, alpha-MeTyr, or absent;
X3 is (D)Arg, (D)Tyr, Gly, alpha-MeArg, alpha-MePhe, alpha-MeLeu, alpha-MeLys, alpha-MeAsn, alpha-MeTyr, or absent;
X4 is Abu, Cys, (D)Cys, alpha-MeCys, (D)Abu, (D)Pen, or Pen;
X5 is Cit, Glu, Gly, Lys, Asn, Pro, alpha-MeGln, alpha-MeLys, alpha-MeLeu, alpha-MeAsn, Lys(Ac), alpha-MeLys(Ac), Dab(Ac), Dap(Ac), homo-Lys(Ac), or Gln;
X6 is Thr, Aib, Asp, Dab, Gly, Pro, Ser, alpha-MeGln, alpha-MeLys, alpha-MeLeu, alpha-MeAsn, alpha-MeThr, alpha-MeSer, or Val;
X7 is Trp, Trp(5-F), 1-Nal, 2-Nal, Phe(2-Me), Phe(3-Me), Phe(4-Me), Trp(7-Aza), or Phe(3,4-dimethoxy):
X8 is Gln, alpha-Me-Lys, alpha-MeLeu, alpha-MeLys(Ac), beta-homoGln, Cit, Glu, Phe, Asn, Thr, Val, Aib, alpha-MeGln, alpha-MeAsn, Lys(Ac), alpha-MeLys(Ac), Dab(Ac), Dap(Ac), homo-Lys(Ac),or Trp;
X9 is Cys, (D)Cys), alpha-MeCys, (D)Abu, (D)Pen, Pen or Abu;
X10 is Phe, Phe[4-(2-aminoethoxy)], Phe[4-(2-acetylaminoethoxy)], alpha-MeTyr, or Phe(4-CONH₂);
X11 is 2-Nal, Trp, Trp(5-F), Trp(7-Aza), Phe(2-Me), Phe(3-Me), Phe(4-Me), Phe(3,4-dimethoxy), or 1-Nal:
X12 is 4-amino-4-carboxy-tetrahydropyran (THP), alpha-MeLys, alpha-MeLeu, alpha-MeArg, alpha-MePhe, alpha-MeLeu, alpha-MeLys, alpha-MeAsn, alpha-MeTyr, or Aib:
X13 is Glu, Cit, Gln, alpha-MeArg, alpha-MeGlu, alpha-MeLeu, alpha-MeLys, alpha-Me-Asn, Lys(Ac), alpha-MeLys(Ac), Dab(Ac), Dap(Ac), homo-Lys(Ac), or Lys;
X14 is Asn, 2-Nap, Aib, Arg, Cit, Asp, Phe, Gly, Lys, Leu, Asn, n-Leu, Gln, Ser, Tic, Trp, alpha-MeGln, alpha-MeAsn, alpha-MeLys(Ac), Dab(Ac), Dap(Ac), homo-Lys(Ac),or Lys(Ac);
X15 is Asn, Aib, beta-Ala, Cit, Gln, Asp, alpha-MeGln, alpha-MeAsn, Lys(Ac), alpha-MeLys(Ac), Dab(Ac), Dap(Ac), homo-Lys(Ac),or absent;
X16 is Glu, Phe, Lys, Asn, Trp, Gly, Thr, Pro, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent;
X17 is Lys, Gly, Pro, The, Phe, Trp, Gln, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent;
X18 is Gly, Lys, Glu, Phe, Thr, Arg, Gln, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent;
X19 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent;
X20 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent;
X21 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent;
X22 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent: and
X23 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent,
wherein the peptide inhibitor is cyclized via a bond between X4 and X9, and
wherein the peptide inhibitor inhibits the binding of an interleukin-23 (IL-23) to an IL-23 receptor.

In a second aspect, the present invention provides a peptide inhibitor of an interleukin-23 receptor, or a pharmaceutically acceptable salt or solvate thereof, wherein the peptide inhibitor comprises or consists of an amino acid sequence of Formula (V): 
X0-X1-X2-X3-X4-X5-X6-X7-X8-X9-X10-X11-X12-X13-X14-X15-X16-X17-X18-X19-
X20-X21-X22-X23 (V) (SEQ ID NO:238)
 wherein
X0 is Gly, Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, or absent;
X1 is Gly, Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, or absent;
X2 is (D)Asp, Arg, (D)Arg, Phe, (D)Phe, 2-Nal, Thr, Leu, (D)Gln, (D)Asn, IsoGlu, Gly, Arg, Phe, Glu, Gln, Thr, (D)Glu, (D)Thr, (D)Leu, alpha-MeArg, alpha-MePhe, alpha-MeLeu, alpha-MeLys, alpha-MeAsn, alpha-MeTyr, or absent;
X3 is (D)Arg, (D)Tyr, Gly, alpha-MeArg, alpha-MePhe, alpha-MeLeu, alpha-MeLys, alpha-MeAsn, alpha-MeTyr, Lys(Ac), Lys(Y1-Ac), or absent, wherein Y1 is an amino acid;
X4 is Abu, Cys, (D)Cys), alpha-MeCys, (D)Abu, (D)Pen, Pen, or Pen(sulfoxide);
X5 is Cit, Glu, Gly, Lys, Asn, Pro, alpha-MeGln, alpha-MeLys, alpha-MeLeu, alpha-MeAsn, Lys(Ac), alpha-MeLys(Ac), Dab(Ac), Dap(Ac), homo-Lys(Ac), Gln, Asp, or Cys;
X6 is Thr, Aib, Asp, Dab, Gly, Pro, Ser, alpha-MeGln, alpha-MeLys, alpha-MeLeu, alpha-MeAsn, alpha-MeThr, alpha-MeSer, or Val;
X7 is Trp, Trp(5-F), 1-Nal, 2-Nal, Phe(2-Me), Phe(3-Me), Phe(4-Me), Trp(7-Aza), or Phe(3,4-dimethoxy);
X8 is Gln, alpha-Me-Lys, alpha-MeLeu, alpha-MeLys(Ac), beta-homoGln, Cit, Glu, Phe, Asn, Thr, Val, Aib, alpha-MeGln, alpha-MeAsn, Lys(Ac), alpha-MeLys(Ac), Dab(Ac), Dap(Ac), homo-Lys(Ac), 1-Nal, 2-Nal, or Trp;
X9 is Cys, (D)Cys), alpha-MeCys, (D)Abu, (D)Pen, Pen, or Abu;
X10 is Phe, Phe[4-(2-aminoethoxy)], Phe[4-(2-acetylaminoethoxy)], alpha-MeTyr, or Phe(4-CONH₂);
X11 is 2-Nal, Trp, Trp(5-F), Trp(7-Aza), Phe(2-Me), Phe(3-Me), Phe(4-Me), Phe(3,4-dimethoxy), or 1-Nal:
X12 is 4-amino-4-carboxy-tetrahydropyran (THP), alpha-MeLys, alpha-MeLeu, alpha-MeArg, alpha-MePhe, alpha-MeLeu, alpha-MeLys, alpha-MeAsn, alpha-MeTyr, Ala, cyclohexylAla, Lys, or Aib;
X13 is Glu, Cit, Gln, Lys(Ac), alpha-MeArg, alpha-MeGlu, alpha-MeLeu, alpha-MeLys, alpha-Me-Asn, alpha-MeLys(Ac), Dab(Ac), Dap(Ac), homo-Lys(Ac), Lys, pegylated Lys, b-homoGlu, or Lys(Y2-Ac), wherein Y2 is an amino acid;
X14 is Asn, 2-Nap, Aib, Arg, Cit, Asp, Phe, Gly, Lys, Leu, Asn, n-Leu, Gln, Ser, Tic, Trp, alpha-MeGln, alpha-MeAsn, alpha-MeLys(Ac), Dab(Ac), Dap(Ac), homo-Lys(Ac), or Lys(Ac);
X15 is Asn, Aib, beta-Ala, Cit, Gln, Asp, alpha-MeGln, alpha-MeAsn, Lys(Ac), alpha-MeLys(Ac), Dab(Ac), Dap(Ac), homo-Lys(Ac), or absent;
X16 is Glu, Phe, Lys, Asn, Trp, Gly, Thr, Pro, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, Ala, Asp, Tyr, Arg, Leu, Gln, Ser, Ile, 1-Nal, 2-Nal, (D)Ala, (D)Asp, (D)Tyr, (D)Arg, (D)Leu, (D)Ser, (D)Ile, or absent;
X17 is Lys, Gly, Pro, The, Phe, Trp, Gln, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent;
X18 is Gly, Lys, Glu, Phe, Thr, Arg, Gln, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent;
X19 is Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent;
X20 is Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent:
X21 is Arg, Phe, Glu, Gln; Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent:
X22 is Arg, Phe, Glu, Gln; Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent: and
X23 is Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent,
wherein the peptide inhibitor is cyclized via a bond between X4 and X9, and wherein the peptide inhibitor inhibits the binding of an interleukin-23 (IL-23) to an IL-23 receptor.

In certain embodiments, X3 is (D)Arg, (D)Tyr, Gly, alpha-MeArg, alpha-MePhe, alpha-MeLeu, alpha-MeLys, alpha-MeAsn, alpha-MeTyr, or absent. In certain embodiments, X3 is Lys(Ac) or Lys(Y1-Ac), wherein Y1 is an amino acid.

In certain embodiments, X4 is Abu, Cys, (D)Cys), alpha-MeCys, (D)Abu, (D)Pen, or Pen. In certain embodiments, X4 is Pen(sulfoxide).

In certain embodiments, X5 is Cit, Glu, Gly, Lys, Asn, Pro, alpha-MeGln, alpha-MeLys, alpha-MeLeu, alpha-MeAsn, Lys(Ac), alpha-MeLys(Ac), Dab(Ac), Dap(Ac), homo-Lys(Ac), or Gln. In certain embodiments, X5 is Asp or Cys.

In certain embodiments, X8 is Gln, alpha-Me-Lys, alpha-MeLeu, alpha-MeLys(Ac), beta-homoGln, Cit, Glu, Phe, Asn, Thr, Val, Aib, alpha-MeGln, alpha-MeAsn, Lys(Ac), alpha-MeLys(Ac), Dab(Ac), Dap(Ac), homo-Lys(Ac), or Trp. In certain embodiments, X8 is 1-Nal or 2-Nal.

In certain embodiments, X12 is 4-amino-4-carboxy-tetrahydropyran (THP), alpha-MeLys, alpha-MeLeu, alpha-MeArg, alpha-MePhe, alpha-MeLeu, alpha-MeLys, alpha-MeAsn, alpha-MeTyr, Ala, cyclohexylAla, Lys, or Aib. In certain embodiments, X12 is Ala, cyclohexylAla, or Lys.

In certain embodiments, X13 is Glu, Cit, Gln, Lys(Ac), alpha-MeArg, alpha-MeGlu, alpha-MeLeu, alpha-MeLys, alpha-Me-Asn, alpha-MeLys(Ac), Dab(Ac), Dap(Ac), homo-Lys(Ac), or Lys. In certain embodiments, X13 is Lys, pegylated Lys, b-homoGlu, or Lys(Y2-Ac), wherein Y2 is an amino acid:

In certain embodiments, X16 is Glu, Phe, Lys, Asn, Trp, Gly, Thr, Pro, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent. In certain embodiments, X16 is Ala, Asp, Tyr, Arg, Leu, Gln, Ser, Ile, 1-Nal, 2-Nal, (D)Ala, (D)Asp, (D)Tyr, (D)Arg, (D)Leu, (D)Ser, or (D)Ile.

In particular embodiments of any of the peptide inhibitors disclosed herein, including peptide inhibitors comprising an amino acid sequence of Formula (I), Formula (II), or Formula (V), X4 is Pen and X9 is Pen, and the bond is a disulfide bond. In particular embodiments of any of the peptide inhibitors, X4 and X9 are Pen. In particular embodiments, X4 and X9 form a disulfide bond.

In certain embodiments of any of the peptide inhibitors disclosed herein, including peptide inhibitors comprising an amino acid sequence of Formula (I), Formula (II), or Formula (V), X4 is Abu: and X9 is Cys. In particular embodiments, X4 is Abu and X9 is Cys. In particular embodiments, X4 and X9 form a thioether bond.

In particular embodiments, any of the peptide inhibitors described herein comprise one or more half-life extension moiety and/or one or more linker moiety conjugated to the peptide inhibitor. In particular embodiments, the half-life extension moiety is conjugated to the peptide inhibitor via one or more linker moieties.

In certain embodiments, any of the peptide inhibitors described herein further comprises a conjugated chemical substituent. In particular embodiments, the conjugated chemical substituent is a lipophilic substituent or a polymeric moiety, e.g., Ac, Palm, gamaGlu-Palm, isoGlu-Palm, PEG2-Ac, PEG4-isoGlu-Palm, (PEG)s-Palm, succinic acid, glutaric acid, pyroglutaric acid, benzoic acid, IVA, octanoic acid, 1,4 diaminobutane, isobutyl, Alexa488, Alexa647, or biotin. In certain embodiments, the conjugated chemical substituent is a polyethylene glycol with a molecular mass of 400 Da to 40,000 Da. In particular embodiments, the peptide is conjugated at X8. In another particular embodiment, the peptide is conjugated at X9. In a more particular embodiment, the peptide is conjugated at X10.

In another aspect, the present invention includes peptide inhibitors comprising the structure of Formula Z:

R¹-X-R² (Z)

or a pharmaceutically acceptable salt or solvate thereof, wherein
R¹ is a bond, hydrogen, a C1-C6 alkyl, a C6-C12 aryl, a C6-C12 aryl, a C1-C6 alkyl, a C1-C20 alkanoyl, and including PEGylated versions alone or as spacers of any of the foregoing:
   R² is a bond, OH or NH₂; and
   X is a peptide comprising any of the amino acid sequences set forth herein, including a peptide comprising an amino acid sequence of Formula (I), Formula (II), Formula (IIIa), (IVa), Formula (V), Formula (XII)-(XVIIIh), or any of the peptide sequences set forth in the tables herein.

In a related aspect, the present invention includes a peptide dimer inhibitor of an interleukin-23 receptor, wherein the peptide dimer inhibitor comprises two peptide monomer subunits connected via one or more linker moieties, wherein each peptide monomer subunit comprises a sequence of Formula (I), Formula (II), Formula (V), or any other sequence or structure set forth herein. In certain embodiments, one or both peptide monomer subunit is cyclized via an intramolecular bond between X4 and X9. In certain embodiments, one or both intramolecular bond is a disulfide bond or a thioether bond. In certain embodiments, the linker is any of those shown in Table 2 or described herein. In certain embodiments, the linker moiety is a diethylene glycol linker, an iminodiacetic acid (IDA) linker, a β-Ala-iminodiaceticacid (β-Ala-IDA) linker, or a PEG linker. In particular embodiments, the N-terminus of each peptide monomer subunit is connected by the linker moiety. In particular embodiments, the C-terminus of each peptide monomer subunit is connected by the linker moiety. In certain embodiments, the linker connects an internal amino acid residue of at least one of the peptide monomer subunits to the N-terminus, C-terminus, or an internal amino acid residue of the other peptide monomer subunit.

In a further related aspect, the present invention includes a polynucleotide comprising a sequence encoding a peptide inhibitor of the present invention or one or both peptide monomer subunit of a peptide dimer inhibitor of the present invention. The present invention also includes a vector comprising the polynucleotide.

In another aspect, the present invention includes a pharmaceutical composition comprising a peptide inhibitor or a peptide dimer inhibitor of the present invention, and a pharmaceutically acceptable carrier, excipient, or diluent. In particular embodiments, the pharmaceutical composition comprises an enteric coating. In certain embodiments, the enteric coating protects and releases the pharmaceutical composition within a subject's lower gastrointestinal system.

In another aspect, the present invention includes a method for treating or preventing a disease associated with IL-23 signalling, including but not limited to an Inflammatory Bowel Disease (IBD), ulcerative colitis, Crohn's disease, Celiac disease (*nontropical Sprue)*, enteropathy associated with seronegative arthropathies, microscopic colitis, collagenous colitis, eosinophilic gastroenteritis, colitis associated with radio- or chemo-therapy, colitis associated with disorders of innate immunity as in leukocyte adhesion deficiency-1, chronic granulomatous disease, glycogen storage disease type 1b, Hermansky-Pudlak syndrome, Chediak-Higashi syndrome, and Wiskott-Aldrich Syndrome, pouchitis resulting after proctocolectomy and ileoanal anastomosis, gastrointestinal cancer, pancreatitis, insulin-dependent diabetes mellitus, mastitis, cholecystitis, cholangitis, pericholangitis, chronic bronchitis, chronic sinusitis, asthma, psoriasis, or graft versus host disease in a subject, comprising providing to the subject an effective amount of a peptide inhibitor or pharmaceutical composition of the present invention. In certain embodiments, the inflammatory bowel disease is ulcerative colitis or Crohn's disease. In particular embodimnts, the peptide inhibitor or the peptide dimer inhibitor inhibits binding of an interleukin-23 (IL-23) to the interleukin-23 receptor (IL-23R). In certain embodiments, the pharmaceutical composition is provided to the subject by an oral, intravenous, peritoneal, intradermal, subcutaneous, intramuscular, intrathecal, inhalation, vaporization, nebulization, sublingual, buccal, parenteral, rectal, intraocular, inhalation, vaginal, or topical route of administration. In particular embodiments, the pharmaceutical composition is provided orally for treating Inflammatory Bowel Disease (IBD), ulcerative colitis, Crohn's disease. In certain embodiments, the pharmaceutical composition is provided to the subject topically, parenterally, intravenously, subcutaneously, peritonealy, or intravenously for treating psoriasis.

### DETAILED DESCRIPTION OF THE INVENTION

Unless otherwise defined herein, scientific and technical terms used in this application shall have the meanings that are commonly understood by those of ordinary skill in the art. Generally, nomenclature used in connection with, and techniques of, chemistry, molecular biology, cell and cancer biology, immunology, microbiology, pharmacology, and protein and nucleic acid chemistry, described herein, are those well-known and commonly used in the art.

As used herein, the following terms have the meanings ascribed to them unless specified otherwise.

Throughout this specification, the word "comprise" or variations such as "comprises" or "comprising" will be understood to imply the inclusion of a stated integer (or components) or group of integers (or components), but not the exclusion of any other integer (or components) or group of integers (or components).

The singular forms "a," "an," and "the" include the plurals unless the context clearly dictates otherwise.

The term "including" is used to mean "including but not limited to." "Including" and "including but not limited to" are used interchangeably.

The terms "patient," "subject," and "individual" may be used interchangeably and refer to either a human or a non-human animal. These terms include mammals such as humans, primates, livestock animals (e.g., bovines, porcines), companion animals (e.g., canines, felines) and rodents (e.g., mice and rats).

The term "peptide," as used herein, refers broadly to a sequence of two or more amino acids joined together by peptide bonds. It should be understood that this term does not connote a specific length of a polymer of amino acids, nor is it intended to imply or distinguish whether the polypeptide is produced using recombinant techniques, chemical or enzymatic synthesis, or is naturally occurring.

The recitations "sequence identity", "percent identity", "percent homology", or, for example, comprising a "sequence 50% identical to," as used herein, refer to the extent that sequences are identical on a nucleotide-by-nucleotide basis or an amino acid-by-amino acid basis over a window of comparison. Thus, a "percentage of sequence identity" may be calculated by comparing two optimally aligned sequences over the window of comparison, determining the number of positions at which the identical nucleic acid base (e.g., A, T, C, G, I) or the identical amino acid residue (e.g., Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (i.e., the window size), and multiplying the result by 100 to yield the percentage of sequence identity.

Calculations of sequence similarity or sequence identity between sequences (the terms are used interchangeably herein) can be performed as follows. To determine the percent identity of two amino acid sequences, or of two nucleic acid sequences, the sequences can be aligned for optimal comparison purposes (e.g., gaps can be introduced in one or both of a first and a second amino acid or nucleic acid sequence for optimal alignment and non-homologous sequences can be disregarded for comparison purposes). In certain embodiments, the length of a reference sequence aligned for comparison purposes is at least 30%, preferably at least 40%, more preferably at least 50%, 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position.

The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In some embodiments, the percent identity between two amino acid sequences is determined using the Needleman and Wunsch, (1970, J. Mol. Biol. 48: 444-453) algorithm which has been incorporated into the GAP program in the GCG software package, using either a Blossum 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package, using an NWSgapdna.CMP matrix and a gap weight of 40, 50, 60, 70, or 80 and a length weight of 1, 2, 3, 4, 5, or 6. Another exemplary set of parameters includes a Blossum 62 scoring matrix with a gap penalty of 12, a gap extend penalty of 4, and a frameshift gap penalty of 5. The percent identity between two amino acid or nucleotide sequences can also be determined using the algorithm of E. Meyers and W. Miller (1989, Cabios, 4: 11-17) which has been incorporated into the ALIGN program (version 2.0), using a PAM120 weight residue table, a gap length penalty of 12 and a gap penalty of 4.

The peptide sequences described herein can be used as a "query sequence" to perform a search against public databases to, for example, identify other family members or related sequences. Such searches can be performed using the NBLAST and XBLAST programs (version 2.0) of Altschul, et al., (1990, J. Mol. Biol, 215: 403-10). BLAST nucleotide searches can be performed with the NBLAST program, score = 100, wordlength = 12 to obtain nucleotide sequences homologous to nucleic acid molecules of the invention. BLAST protein searches can be performed with the XBLAST program, score = 50, wordlength = 3 to obtain amino acid sequences homologous to protein molecules of the invention. To obtain gapped alignments for comparison purposes, Gapped BLAST can be utilized as described in Altschul et al. (Nucleic Acids Res. 25:3389-3402, 1997). When utilizing BLAST and Gapped BLAST programs, the default parameters of the respective programs (e.g., XBLAST and NBLAST) can be used.

The term "conservative substitution" as used herein denotes that one or more amino acids are replaced by another, biologically similar residue. Examples include substitution of amino acid residues with similar characteristics, e.g., small amino acids, acidic amino acids, polar amino acids, basic amino acids, hydrophobic amino acids and aromatic amino acids. See, for example, the table below. In some embodiments of the invention, one or more Met residues are substituted with norleucine (Nle) which is a bioisostere for Met, but which, as opposed to Met, is not readily oxidized. Another example of a conservative substitution with a residue normally not found in endogenous, mammalian peptides and proteins is the conservative substitution of Arg or Lys with, for example, ornithine, canavanine, aminoethylcysteine or another basic amino acid. In some embodiments, one or more cysteines of a peptide analogue of the invention may be substituted with another residue, such as a serine. For further information concerning phenotypically silent substitutions in peptides and proteins, see, for example, Bowie et.al. Science 247, 1306-1310, 1990. In the scheme below, conservative substitutions of amino acids are grouped by physicochemical properties. I: neutral, hydrophilic, II: acids and amides, III: basic, IV: hydrophobic, V: aromatic, bulky amino acids.

| **I** | **II** | **III** | **IV** | **V** |
|---|---|---|---|---|
| A | N | H | M | F |
| S | D | R | L | Y |
| T | E | K | I | W |
| P | Q | | V | |
| G | | | C | |

In the scheme below, conservative substitutions of amino acids are grouped by physicochemical properties. VI: neutral or hydrophobic, VII: acidic, VIII: basic, IX: polar, X: aromatic.

| **VI** | **VII** | **VIII** | **IX** | **X** |
|---|---|---|---|---|
| A | E | H | M | F |
| L | D | R | S | Y |
| I | | K | T | W |
| P | | | C | |
| G | | | N | |
| V | | | Q | |

The term "amino acid" or "any amino acid" as used here refers to any and all amino acids, including naturally occurring amino acids (e.g., a-amino acids), unnatural amino acids, modified amino acids, and non-natural amino acids. It includes both D- and L-amino acids. Natural amino acids include those found in nature, such as, e.g., the 23 amino acids that combine into peptide chains to form the building-blocks of a vast array of proteins. These are primarily L stereoisomers, although a few D-amino acids occur in bacterial envelopes and some antibiotics. The 20 "standard," natural amino acids are listed in the above tables. The "non-standard," natural amino acids are pyrrolysine (found in methanogenic organisms and other eukaryotes), selenocysteine (present in many noneukaryotes as well as most eukaryotes), and N-formylmethionine (encoded by the start codon AUG in bacteria, mitochondria and chloroplasts). "Unnatural" or "non-natural" amino acids are non-proteinogenic amino acids (i.e., those not naturally encoded or found in the genetic code) that either occur naturally or are chemically synthesized. Over 140 unnatural amino acids are known and thousands of more combinations are possible. Examples of "unnatural" amino acids include β-amino acids (β³ and β²), homo-amino acids, proline and pyruvic acid derivatives, 3-substituted alanine derivatives, glycine derivatives, ring-substituted phenylalanine and tyrosine derivatives, linear core amino acids, diamino acids, D-amino acids, alpha-methyl amino acids and N-methyl amino acids. Unnatural or non-natural amino acids also include modified amino acids. "Modified" amino acids include amino acids (e.g., natural amino acids) that have been chemically modified to include a group, groups, or chemical moiety not naturally present on the amino acid. According to certain embodiments, a peptide inhibitor comprises an intramolecular bond between two amino acid residues present in the peptide inhibitor. It is understood that the amino acid residues that form the bond will be altered somewhat when bonded to each other as compared to when not bonded to each other. Reference to a particular amino acid is meant to encompass that amino acid in both its unbonded and bonded state. For example, the amino acid residue homoSerine (hSer) or homoSerine(Cl) in its unbonded form may take the form of 2-aminobutyric acid (Abu) when participating in an intramolecular bond according to the present invention. The present invention inclues both peptide inhibitors containing cross-links between X4 and X9, as well as the peptide inhibitors that do not contain cross-links between X4 and X9, e.g., before cross-link formation. As such, the names hSer and Abu are intended to indicate the same amino acids and are used interchangeably.

For the most part, the names of naturally occurring and non-naturally occurring aminoacyl residues used herein follow the naming conventions suggested by the IUPAC Commission on the Nomenclature of Organic Chemistry and the IUPAC-IUB Commission on Biochemical Nomenclature as set out in "Nomenclature of α-Amino Acids (Recommendations, 1974)" Biochemistry, 14(2), (1975). To the extent that the names and abbreviations of amino acids and aminoacyl residues employed in this specification and appended claims differ from those suggestions, they will be made clear to the reader. Some abbreviations useful in describing the invention are defined below in the following Table 1.

**Table 1. Abbreviations of Non-Natural Amino Acids and Chemical Moieties (for amino acid derivatives, all L unless stated**

| Abbreviation | Definition |
|---|---|
| Ac- | Acetyl |
| Hy | Hydrogen (Free N-terminal) |
| Dap | L-Diaminopropionic acid |
| Dab | L-Diaminobutyric acid |
| Orn | L-Omathine |
| Pen | L-Penicillamine |
| Sarc | Sarcosine |
| Cit | L-Citrulline |
| Cav | L-Cavanine |
| Phe-(4-Guanidino) | 4-Guanidine-L-Phenylalanine |
| N-MeArg | N-Methyl-L-Arginine |
| N-MeTrp | N-Methyl-L-Tryptophan |
| N-MeGln | N-Methyl-L-Glutamine |
| N-MeAla | N-Methyl-L-Alanine |
| N-MeLys | N-Methyl-Lysine |
| N-MeAsn | N-Methyl-L-Asparagine |
| 6-ChloroTrp | 6-Chloro-L-Tryptophan |
| 5-HydroxyTrp | 5-Hydroxy-L-Tryptophan |
| 1,2,3,4-tetrahy dro-norharman | L-1,2,3,4-tetrahydro-norharman |
| 2-Nal (also referred to as 2-Nap) | L-2-Napthylalanine |
| 1-Nal (also referred to as 1-Nap) | L-1-Napthylalanine |
| Phe(4-OMe) | 4-Methoxy-L-phenylalanine |
| Abu | 2-Aminobutyric acid |
| Bip | L-4,4'-Biphenylalanine |
| βAla | beta-Alanine |
| βhTyr | beta homo-L-Tyrosine |
| βhTrp | beta homo-L-Trptophan |
| βhAla | beta homo-L-Alanine |
| βhLeu, | beta homo-L-Leucine |
| βhVal | beta homo-L-Valine |
| Aib | 2-aminoisobutyric acid |
| Azt | L-azetidine-2-carboxylic acid |
| Tic | (3S)-1,2,3,4-Tetrahydroisoquinoline-7-hydroxy-3-carboxylic Acid |
| Phe(4-OMe) | 4-methoxy-L-phenylalanine |
| N-Me-Lys | N-Methyl-L-Lysine |
| N-Me-Lys(Ac) | N-ε-Acetyl-D-lysine |
| CONH₂ | Carboxamide |
| COOH | Acid |
| 3-Pal | L-3-Pyridylalanine |
| Phe(4-F) | 4-Fluoro-L-Phenylalanine |
| DMT | 2,6-DimethylTyrosine |
| Phe(4-OMe) | 4-Methoxyphenylalanine |
| hLeu | L-homoLeucine |
| hArg | L-homoArginine |
| α-MeLys | alpha-methyl-L-Lysine |
| α-MeOrn | alpha-methyl-L-Ornathine |
| α-MeLeu | alpha-methyl-L-Leucine |
| α-MeTrp | alpha-methyl-L-Tryptophan |
| α-MePhe | alpha-methyl-L-Phenylalanine |
| α-MeTyr | alpha-methyl-L-Tyrosine |
| α-DiethylGly | α-DiethylGlycine |
| Lys(Ac) | N-ε-acetyl-L-Lysine |
| DTT | Dithiothreotol |
| Nle | L-Norleucine |
| βhTrp | L-β-homoTrypophan |
| βhPhe | L-β-homophenylalanine |
| βhPro | L-B-homoproline |
| Phe(4-CF₃) | 4-Trifluoromethyl-L-Phenylalanine |
| β-Glu | L-β-Glutamic acid |
| βhGlu | L-β-homoglutamic acid |
| 2-2-Indane | 2-Aminoindane-2-carboxylic acid |
| 1-1-Indane | 1-Aminoindane-1-carboxylic acid |
| hCha | L-homocyclohexylalanine |
| Cyclobutyl | L-cyclobutylalanine |
| βhPhe | L-β-homo-phenylalanine |
| Gla | Gama-Carboxy-L-Glutamic acid |
| Cpa | Cyclopentyl-L-alanine |
| Cha | Cyclohexyl-L-alanine |
| Octgly | L-Octylglycine |
| *t*-butyl-Ala | 3-(*tert*-butyl)-L-Ala-OH |
| *t*-butyl-Gly | *tert*-butyl-glycine |
| AEP | 3-(2-aminoethoxy)propanoic acid |
| AEA | (2-aminoethoxy)acetic acid |
| Phe(4-Phenoxy)] | 4-Phenoxy-L-phenylalanine |
| Phe(4-OBzl) | *O*-*Benzyl*-L-tyrosine |
| Phe(4-CONH₂) or Phe(Cmd) | 4-Carbamoyl-L-phenylalanine |
| Phe(4-CO₂H) | 4-Carboxy-L-phenylalanine |
| Phe(3,4-Cl₂) | 3,4 dichloro-L-phenylalanine |
| Tyr(3-t-Bu) | 3-*t*-butyl-L-tyrosine |
| Phe(t-Bu) | *t*-butyl-L-phenylalanine |
| Phe[4-(2-aminoethoxy)] | 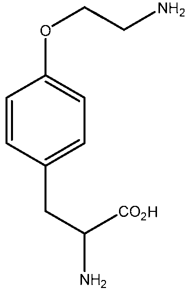 4-(2-aminoethoxy)-L-phenylalanine |
| Phe(4-CN) | 4-cyano-L-phenylalanine |
| Phe(4-Br) | 4-bromo-L-phenylalanine |
| Phe(4-NH₂) | 4-amino-L-phenylalanine |
| Phe(4-Me) | 4-methyl-L-phenylalanine |
| 4-Pyridylalanine | 4-L-Pyridylalanine |
| 4-amino-4-carboxy-piperidine | 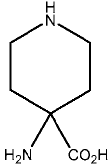 4-amino-4-carboxy-piperidine |
| hPhe(3,4-dimethoxy) | 3,4-dimethoxy-L-homophenylalanine |
| Phe(2,4-Me₂) | 2,4-dimethyl-L-phenylalanine |
| Phe(3,5-F₂) | 3,5-difluoro-L-phenylalanine |
| Phe(penta-F) | pentafluoro-L-phenylalanine |
| 2,5,7-tert butyl Trp | 2,5,7-Tris-tert-butyl-L-tryptophan |
| Tic | 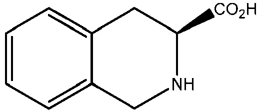 L-1,2,3,4,-tetrahydro-isoquinoline-3-carboxylic acid |
| Phe(4-OAllyl) | O-*Allyl*-L-Tyrosine |
| Phe(4-N₃) | 4-azidophenylalanine |
| Ache | 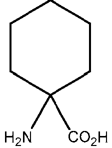 1-aminocyclohexanecarboxylic acid |
| Acvc | 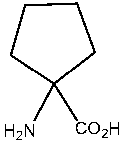 1-aminocyclopentanecarboxylic acid |
| Acbc | 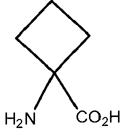 1-aminocyclobutanecarboxylic acid |
| Acpc | 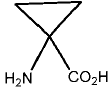 1-aminocyclopropylcarboxylic acid |
| 4-amino-4-carboxy-tetrahydropyran (also referred as THP) | 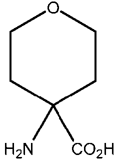 4-amino-4-carboxy-tetrahydropyran |
| Ahx | 6-aminohexanoic acid |

Throughout the present specification, unless naturally occurring amino acids are referred to by their full name (e.g., alanine, arginine, etc.), they are designated by their conventional three-letter or single-letter abbreviations (e.g., Ala or A for alanine, Arg or R for arginine, etc.). Unless otherwise indicated, three-letter and single-letter abbreviations of amino acids refer to the L-isomeric form of the amino acid in question. The term "L-amino acid," as used herein, refers to the "L" isomeric form of a peptide, and conversely the term "D-amino acid" refers to the "D" isomeric form of a peptide (e.g., Dasp, (D)Asp or D-Asp; Dphe, (D)Phe or D-Phe). Amino acid residues in the D isomeric form can be substituted for any L-amino acid residue, as long as the desired function is retained by the peptide. D-amino acids may be indicated as customary in lower case when referred to using single-letter abbreviations.

In the case of less common or non-naturally occurring amino acids, unless they are referred to by their full name (e.g. sarcosine, ornithine, etc.), frequently employed three- or four-character codes are employed for residues thereof, including, Sar or Sarc (sarcosine, i.e. N-methylglycine), Aib (α-aminoisobutyric acid), Dab (2,4-diaminobutanoic acid), Dapa (2,3-diaminopropanoic acid), γ-Glu (y-glutamic acid), Gaba (y-aminobutanoic acid), β-Pro (pyrrolidine-3-carboxylic acid), and 8Ado (8-amino-3,6-dioxaoctanoic acid), Abu (2-amino butyric acid), βhPro (β-homoproline), βhPhe (β-homophenylalanine) and Bip (β,β diphenylalanine), and Ida (Iminodiacetic acid).

As is clear to the skilled artisan, the peptide sequences disclosed herein are shown proceeding from left to right, with the left end of the sequence being the N-terminus of the peptide and the right end of the sequence being the C-terminus of the peptide. Among sequences disclosed herein are sequences incorporating a "Hy-"" moiety at the amino terminus (N-terminus) of the sequence, and either an "-OH" moiety or an "-NH₂" moiety at the carboxy terminus (C-terminus) of the sequence. In such cases, and unless otherwise indicated, a "Hy-" moiety at the N-terminus of the sequence in question indicates a hydrogen atom, corresponding to the presence of a free primary or secondary amino group at the N-terminus, while an "-OH" or an "-NH₂" moiety at the C-terminus of the sequence indicates a hydroxy group or an amino group, corresponding to the presence of an amido (CONH₂) group at the C-terminus, respectively. In each sequence of the invention, a C-terminal "-OH" moiety may be substituted for a C-terminal "-NH₂" moiety, and vice-versa.

One of skill in the art will appreciate that certain amino acids and other chemical moieties are modified when bound to another molecule. For example, an amino acid side chain may be modified when it forms an intramolecular bridge with another amino acid side chain, e.g., one or more hydrogen may be removed or replaced by the bond. Accordingly, as used herein, reference to an amino acid or modified amino acid present in a peptide dimer of the present invention (e.g., at position X4 or position X9) is meant to include the form of such amino acid or modified amino acid present in the peptide both before and after forming the intramolecular bond.

The term "dimer," as used herein, refers broadly to a peptide comprising two or more monomer subunits. Certain dimers comprise two monomer subunits comprising a sequence of Formula (I) or set forth herein. Dimers of the present invention include homodimers and heterodimers. A monomer subunit of a dimer may be linked at its C- or N-terminus, or it may be linked via internal amino acid residues. Each monomer subunit of a dimer may be linked through the same site, or each may be linked through a different site (e.g., C-terminus, N-terminus, or internal site).

The term "NH₂," as used herein, can refer to a free amino group present at the amino terminus of a polypeptide. The term "OH," as used herein, can refer to a free carboxy group present at the carboxy terminus of a peptide. Further, the term "Ac," as used herein, refers to Acetyl protection through acylation of the C- or N-terminus of a polypeptide. In certain peptides shown herein, the NH₂ locates at the C-terminus of the peptide indicates an amino group.

The term "carboxy," as used herein, refers to -CO₂H.

The term "isostere replacement," as used herein, refers to any amino acid or other analog moiety having chemical and/or structural properties similar to a specified amino acid. In certain embodiments, an isostere replacement is a conservative substitution or an analog of a specified amino acid.

The term "cyclized," as used herein, refers to one part of a polypeptide molecule being linked to another part of the polypeptide molecule to form a closed ring, such as by forming a disulfide bridge or thioether bond.

The term "subunit," as used herein, refers to one of a pair of polypeptide monomers that are joined to form a dimer peptide composition.

The term "linker moiety," as used herein, refers broadly to a chemical structure that is capable of linking or joining together two peptide monomer subunits to form a dimer.

The term "pharmaceutically acceptable salt," as used herein, represents salts or zwitterionic forms of the peptides or compounds of the present invention which are water or oil-soluble or dispersible, which are suitable for treatment of diseases without undue toxicity, irritation, and allergic response; which are commensurate with a reasonable benefit/risk ratio, and which are effective for their intended use. The salts can be prepared during the final isolation and purification of the compounds or separately by reacting an amino group with a suitable acid. Representative acid addition salts include acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, formate, fumarate, hydrochloride, hydrobromide, hydroiodide, 2-hydroxyethansulfonate (isethionate), lactate, maleate, mesitylenesulfonate, methanesulfonate, naphthylenesulfonate, nicotinate, 2-naphthalenesulfonate, oxalate, pamoate, pectinate, persulfate, 3-phenylproprionate, picrate, pivalate, propionate, succinate, tartrate, trichloroacetate, trifluoroacetate, phosphate, glutamate, bicarbonate, para-toluenesulfonate, and undecanoate. Also, amino groups in the compounds of the present invention can be quaternized with methyl, ethyl, propyl, and butyl chlorides, bromides, and iodides; dimethyl, diethyl, dibutyl, and diamyl sulfates; decyl, lauryl, myristyl, and steryl chlorides, bromides, and iodides; and benzyl and phenethyl bromides. Examples of acids which can be employed to form therapeutically acceptable addition salts include inorganic acids such as hydrochloric, hydrobromic, sulfuric, and phosphoric, and organic acids such as oxalic, maleic, succinic, and citric. A pharmaceutically acceptable salt may suitably be a salt chosen, e.g., among acid addition salts and basic salts. Examples of acid addition salts include chloride salts, citrate salts and acetate salts. Examples of basic salts include salts where the cation is selected among alkali metal cations, such as sodium or potassium ions, alkaline earth metal cations, such as calcium or magnesium ions, as well as substituted ammonium ions, such as ions of the type N(R1)(R2)(R3)(R4)+, where R1, R2, R3 and R4 independently will typically designate hydrogen, optionally substituted C1-6-alkyl or optionally substituted C2-6-alkenyl. Examples of relevant C 1-6-alkyl groups include methyl, ethyl, 1-propyl and 2-propyl groups. Examples of C2-6-alkenyl groups of possible relevance include ethenyl, 1-propenyl and 2-propenyl. Other examples of pharmaceutically acceptable salts are described in "Remington's Pharmaceutical Sciences", 17th edition, Alfonso R. Gennaro (Ed.), Mark Publishing Company, Easton, PA, USA, 1985 (and more recent editions thereof), in the "Encyclopaedia of Pharmaceutical Technology", 3rd edition, James Swarbrick (Ed.), Informa Healthcare USA (Inc.), NY, USA, 2007, and in J. Pharm. Sci. 66: 2 (1977). Also, for a review on suitable salts, see Handbook of Pharmaceutical Salts: Properties. Selection, and Use by Stahl and Wermuth (Wiley-VCH, 2002). Other suitable base salts are formed from bases which form non-toxic salts. Representative examples include the aluminum, arginine, benzathine, calcium, choline, diethylamine, diolamine, glycine, lysine, magnesium, meglumine, olamine, potassium, sodium, tromethamine, and zinc salts. Hemisalts of acids and bases may also be formed, e.g., hemisulphate and hemicalcium salts.

The term "N(alpha)Methylation", as used herein, describes the methylation of the alpha amine of an amino acid, also generally termed as an N-methylation.

The term "sym methylation" or "Arg-Me-sym", as used herein, describes the symmetrical methylation of the two nitrogens of the guanidine group of arginine. Further, the term "asym methylation" or "Arg-Me-asym" describes the methylation of a single nitrogen of the guanidine group of arginine.

The term "acylating organic compounds", as used herein refers to various compounds with carboxylic acid functionality that are used to acylate the N-terminus of an amino acid or a monomer or dimer, e.g., a monomer subunit prior to forming a C-terminal dimer. Nonlimiting examples of acylating organic compounds include cyclopropylacetic acid, 4-Fluorobenzoic acid, 4-fluorophenylacetic acid, 3-Phenylpropionic acid, Succinic acid, Glutaric acid, Cyclopentane carboxylic acid, 3,3,3-trifluoropropeonic acid, 3-Fluoromethylbutyric acid, Tetrahedro-2H-Pyran-4-carboxylic acid.

The term "alkyl" includes a straight chain or branched, noncyclic or cyclic, saturated aliphatic hydrocarbon containing from 1 to 24 carbon atoms. Representative saturated straight chain alkyls include, but are not limited to, methyl, ethyl, *n*-propyl, *n*-butyl, *n*-pentyl, *n*-hexyl, and the like, while saturated branched alkyls include, without limitation, isopropyl, *sec*-butyl, isobutyl, *tert*-butyl, isopentyl, and the like. Representative saturated cyclic alkyls include, but are not limited to, cyclopropyl, cyclobutyl, cyclopentyl, cyclohexyl, and the like, while unsaturated cyclic alkyls include, without limitation, cyclopentenyl, cyclohexenyl, and the like.

The term "mammal" refers to any mammalian species such as a human, mouse, rat, dog, cat, hamster, guinea pig, rabbit, livestock, and the like.

As used herein, a "therapeutically effective amount" of the peptide inhibitor of the invention is meant to describe a sufficient amount of the peptide inhibitor to treat an IL-23/IL-23R-related disease, including but not limited to any of the diseases and disorders described herein (for example, to reduce inflammation associated with IBD). In particular embodiments, the therapeutically effective amount will achieve a desired benefit/risk ratio applicable to any medical treatment.

An "analog" of an amino acid, e.g., a "Phe analog" or a "Tyr analog" means an analog of the referenced amino acid. A variety of amino acid analogs are known and available in the art, including Phe and Tyr analogs. In certain embodiments, an amino acid analog, e.g., a Phe analog or a Tyr analog comprises one, two, three, four or five substitutions as compared to Phe or Tyr, respectively. In certain embodiments, the substitutions are present in the side chains of the amino acids. In certain embodiments, a Phe analog has the structure Phe(R²), wherein R² is a Hy, OH, CH₃, CO₂H, CONH₂, CONH₂OCH₂CH₂NH₂, t-Bu, OCH₂CH₂NH₂, phenoxy, OCH₃, OAllyl, Br, Cl, F, NH₂, N3, or guanadino. In certain embodiments, R² is CONH₂OCH₂CH₂NH₂, OCH₃, CONH₂, OCH₃ or CO₂H. Examples of Phe analogs include, but are not limited to: hPhe, Phe(4-OMe), α-Me-Phe, hPhe(3,4-dimethoxy), Phe(4-CONH₂), Phe(4-phenoxy), Phe(4-guanadino), Phe(4-tBu), Phe(4-CN), Phe(4-Br), Phe(4-OBzl), Phe(4-NH₂), BhPhe(4-F), Phe(4-F), Phe(3,5 DiF), Phe(CH₂CO₂H), Phe(penta-F), Phe(3,4-Cl₂), Phe (3,4-F₂), Phe(4-CF₃), ββ-diPheAla, Phe(4-N₃), Phe[4-(2-aminoethoxy)], 4-Phenylbenzylalanine, Phe(4-CONH₂), Phe(3,4-Dimethoxy), Phe(4-CF₃), Phe(2,3-Cl₂), and Phe(2,3-F₂). Examples of Tyr analogs include, but are not limited to: hTyr, N-Me-Tyr, Tyr(3-tBu), Tyr(4-N₃) and βhTyr.

### Peptide Inhibitors of IL-23R

Genome-wide association studies (GWAS) have demonstrated significant association of the IL-23 receptor (IL-23R) gene with inflammatory bowel disease (IBD), suggesting that perturbation of IL-23 signaling could be relevant to the pathogenesis of this disease and other inflammatory diseases and disorders. The present invention provides compositions and methods to modulate the IL-23 pathway through antagonism of IL-23R.

The present invention relates generally to peptides that have IL-23R antagonist activity, including both peptide monomers and peptide dimers. In certain embodiments, this invention demonstrates a new paradigm for treatment of IBD and other diseases and disorders by oral delivery of antagonists of IL-23. IBD represents a local inflammation of the intestinal tissue: therefore, advantageous therapeutic agents act from the luminal side of the intestine, yielding high drug concentrations in diseased tissue, minimizing systemic availability and resulting in improved efficacy and safety when compared to systemic approaches. Oral administration of the compounds of the present invention is expected to maximize drug levels in diseased intestinal tissues while limiting drug concentrations in circulation, thereby providing efficacious, safe, and durable delivery for life-long treatment of IBD and other diseases and disorders.

In certain embodiments, the present invention relates to various peptides, or peptide dimers comprising hetero- or homo-monomer subunits, that form cyclized structures through disulfide or other bonds. In certain embodiments, the disulfide or other bonds are intramolecular bonds. The cyclized structure of the peptide monomer inhibitors and the monomer subunits of the peptide dimer inhibitors has been shown to increase potency and selectivity of the peptide inhibitors. In certain embodiments, a peptide dimer inhibitor may include one or more intermolecular bonds linking the two monomer peptide subunits within the peptide dimer inhibitor, e.g., an intermolecular bridge between two Pen residues, one in each peptide monomer subunit.

The present invention provides peptide inhibitors that bind to IL-23R, which may be monomers or dimers. In particular embodiments, the peptide inhibitors inhibit the binding of IL-23 to IL-23R. In certain embodiments, the IL-23R is human IL-23R, and the IL-23 is human IL-23. In certain embodiments, a peptide inhibitor of the present invention reduces IL-23 binding to IL-23R by at least 20%, at least 30%, at least 40%, at least 50%, at least 60%, at least 70%, at least 80%, or at least 90% as compared to a negative control peptide. Methods of determining binding are known in the art and include ELISA assays, as described in the accompanying Examples.

In certain embodiments, a peptide inhibitor of the present invention has an IC50 of > 1 mM, < 1 mM, 500 nM to 1000 nM, < 500 nM, < 250 nM, < 100 nM, < 50 nM, < 25 nM, < 10 nM, < 5 nM, < 2 nM, < 1 nM, or < 5 mM, e.g., for inhibiting binding of IL-23 to IL-23R (e.g., human IL-23 and human IL-23R). Methods of determining activity are known in the art and include any of those described in the accompanying Examples.

In certain embodiments, a peptide inhibitor of the present invention has increased stability, increased gastrointestinal stability, or increased stability in stimulated intestinal fluid (SIF) or simulated gastric fluid (SGF), and/or under redox conditions (e.g., DTT) as compared to a control peptide. In certain embodiments, a control peptide is an unrelated peptide of the same or similar length. In particular embodiments, a control peptide is a peptide having the identical or a highly related amino acid sequence (e.g., > 90% sequence identity) as the peptide inhibitor. In particular embodiments, a control peptide is a peptide having the identical or a highly related amino acid sequence (e.g., > 90% sequence identity) as the peptide inhibitor, but which does not have a cyclized structure, e.g., through an intramolecular bond between two amino acid residues within the control peptide, or which is not dimerized, or which does not comprise a conjugate for stabilization. In particular embodiments, the only difference between the peptide inhibitor and the control peptide is that the peptide inhibitor comprises one or more amino acid substitutions that introduce one or more amino acid residues into the peptide inhibitor, wherein the introduced amino residue(s) forms an intrasulfide disulfide or thioether bond with another amino acid residue in the peptide inhibitor. One example of a control for a peptide dimer inhibitor is a monomer having the same sequence as one of the monomer subunits present in the peptide dimer inhibitor. One example of a control for a peptide inhibitor comprising a conjugate is a peptide having the same sequence but not including the conjugated moiety. In certain embodiments, a control peptide is a peptide (e.g., a naturally-occurring peptide) corresponding to a region of IL-23 that binds to IL-23R.

Methods of determining the stablity of a peptide are known in the art. In certain embodiments, the stability of a peptide inhibitor is determined using an SIF assay, e.g., as described in Example 3. In certain embodiments, the stability of a peptide inhibitor is determined using an SGF assay, e.g., as described in Example 3. In particular embodiments, a peptide inhibitor has a half-life (e.g., in SIF or SGF or DTT) under a given set of conditions (e.g., temperature) of greater than 1 minute, greater than 10 minutes, greater than 20 minutes, greater than 30 minutes, greater than 60 minutes, greater than 90 minutes, greater than 120 minutes, greater than 3 hours, or greater than four hours when exposed to SIF or SGF or DTT. In certain embodiments, the temperature is about 25 °C, about 4 °C, or about 37 °C, and the pH is a physiological pH, or a pH about 7.4.

In some embodiments, the half-life is measured in vitro using any suitable method known in the art, e.g., in some embodiments, the stability of a peptide of the present invention is determined by incubating the peptide with pre-warmed human serum (Sigma) at 37 ° C. Samples are taken at various time points, typically up to 24 hours, and the stability of the sample is analyzed by separating the peptide or peptide dimer from the serum proteins and then analyzing for the presence of the peptide or peptide dimer of interest using LC-MS.

In some embodiments, a peptide inhibitor of the present invention exhibits improved solubility or improved aggregation characteristics as compared to a control peptide. Solubility may be determined via any suitable method known in the art. In some embodiments, suitable methods known in the art for determining solubility include incubating peptides in various buffers (Acetate pH4.0, Acetate pH5.0, Phos/Citrate pH5.0, Phos Citrate pH6.0, Phos pH 6.0, Phos pH 7.0, Phos pH7.5, Strong PBS pH 7.5, Tris pH7.5, Tris pH 8.0, Glycine pH 9.0, Water, Acetic acid (pH 5.0 and other known in the art) and testing for aggregation or solubility using standard techniques. These include, but are not limited to, visual precipitation, dynamic light scattering, Circular Dichroism and fluorescent dyes to measure surface hydrophobicity, and detect aggregation or fibrillation, for example. In some embodiments, improved solubility means the peptide is more soluble in a given liquid than is a control peptide. In some embodiments, improved aggregation means the peptide has less aggregation in a given liquid under a given set of conditions than a control peptide.

In certain embodiments advantageous for achieving high compound concentrations in intestinal tissues when delivered orally, peptide inhibitors of the present invention are stable in the gastrointestinal (GI) environment. Proteolytic metabolism in the GI tract is driven by enzymes (including pepsins, trypsin, chymotrypsin, elastase, aminopeptidases, and carboxypeptidase A/B) that are secreted from the pancreas into the lumen or are produced as brush border enzymes. Proteases typically cleave peptides and proteins that are in an extended conformation. In the reducing environment of intestinal fluids, disulfide bonds may be broken, resulting in a linear peptide and rapid proteolysis. This luminal redox environment is largely determined by the Cys/CySS redox cycle. In enterocytes, relevant activities include numerous digestive enzymes such as CYP450 and UDP-glucuronsyl-transferase. Finally, bacteria, present in the large intestine at concentration ranging from 10¹⁰ to 10¹² CFU/ml, constitute another metabolic barrier. In certain embodiments, the peptide inhibitors are stable to various pHs that range from strongly acidic in the stomach (pH 1.5-1.9), trending towards basic in the small intestine (pH 6-7.5), and then weakly acidic in the colon (pH 5-7). Such peptide inhibitors are stable during their transit through the various GI compartments, a process that has been estimated to take 3-4 h in the intestine and 6-48 h in the colon.

In some embodiments, the peptide inhibitors of the present invention have less degradation, e.g., over a period of time (i.e., more degradation stability), e.g., greater than or about 10% less, greater than or about 20% less, greater than or about 30% less, greater than or about 40 less, or greater than or about 50% less degradation than a control peptide. In some embodiments, degradation stability is determined via any suitable method known in the art. In some embodiments, the degradation is enzymatic degradation. For example, in certain embodiments, the peptide inhibitors have reduced susceptibility to degradation by trypsin, chhrmotrypsin or elastase. In some embodiments, suitable methods known in the art for determining degradation stability include the method described in Hawe et al., J Pharm Sci, VOL. 101, No. 3, 2012, p 895-913, incorporated herein in its entirety. Such methods are in some embodiments used to select potent peptide sequences with enhanced shelf lifes. In particular embodiments, peptide stability is determined using a SIF assay or SGF assay, e.g., as described in PCT Publication No. WO 2016/011208.

In certain embodiments, peptide inhibitors of the present invention inhibit or reduce IL-23-mediated inflammation. In related embodiments, peptide inhibibitors of the present invention inhibit or reduce IL-23-mediated secretion of one or more cytokines, e.g., by binding to IL-23R on the cell surface, thus inhibiting IL-23 binding to the cell. In particular embodiments, peptide inhibitors of the present invention inhibit or reduce IL-23-mediated activation of Jak2, Tyk2, Stat1, Stat3, Stat4, or Stat5. Methods of determining inhibition of cytokine secretion and inhibition of signaling molecules are known in the art. For example, inhibiton of IL-23/IL-23R signaling may be determined by measuring inhibition of phospho-Stat3 levels in cell lysates, e.g., as described in PCT Publication No. WO 2016/011208.

In certain embodiments, peptide inhibitors have increased redox stability as compared to a control peptide. A variety of assays that may be used to determine redox stability are known and available in the art. Any of these may be used to determine the redox stability of peptide inhibitors of the present invention.

In certain embodiments, the present invention provides various peptide inhibitors that bind or associate with the IL-23R, *in vitro* or *in vivo*, to disrupt or block binding between IL-23 and IL-23R. In certain embodiments, the peptide inhibitors bind and/or inhibit human IL-23R. In certain embodiments, the peptide inhibitors bind and/or inhibit both human and rodent IL-23R. In certain embodiments, the peptide inhibitors bind and/or inhibit both human and rat IL-23R. In particular embodiments, the peptide inhibitors inhibit rat IL-23R at least 50%, at least 60%, at least 70%, at least 80%, at least 90%, or at least 95% as well as they bind or inhibit human IL-23R, e.g., as determined by an assay described herein. In certain embodiments, the peptide inhibitors preferentially bind and/or inhibit human and/or rat IL-23R as compared to mouse IL-23R. In particular embodiments, the peptide inhibitors preferentially bind to rat IL-23R as compared to mouse IL-23R. In particular embodiments, the peptide inhibitors preferentially bind to human IL-23R as compared to mouse IL-23R. In certain embodiments, binding of a peptide inhibitor to mouse IL-23R is less than 75%, less than 50%, less than 40%, less than 30%, less than 20%, or less than 10% of binding of the same peptide inhibitor to human IL-23R and/or rat IL-23R. In certain embodiments of peptide inhibitors that preferentially bind and/or inhibit human IL-23R and/or rat IL-23R as compared to mouse IL-23R, the peptide inhibitor binds to a region of IL-23R that is disrupted by the presence of additional amino acids present in mouse IL-23R but not human IL-23R or rat IL-23. In one embodiment, the additional amino acids present in the mouse IL-23R are in the region corresponding to about amino acid residue 315 to about amino acid residue 340 of the mouse IL23R protein, e.g., amino acid region NWQPWSSPFVHQTSQETGKR (SEQ ID NO:239). In particular embodiments, the peptide inhibitors bind to a region of human IL-23R from about amino acid 230 to about amino acid residue 370.

In certain embodiments, peptide inhibitors show GI-restricted localization following oral administration. In particular embodiments, greater than 50%, greater than 60%, greater than 70%, greater than 80%, or greater than 90% of orally administered peptide inhibitor is localized to gastrointestinal organs and tissues. In particular embodiments, blood plasma levels of orally administered peptide inhibitor are less than 20%, less than 10%, less than 5%, less than 2%, less than 1% or less than 0.5% the levels of peptide inhibitor found in the small intestine mucosa, colon mucosa, or proximal colon.

The various peptide inhibitors of the invention may be constructed solely of natural amino acids. Alternatively, the peptide inhibitors may include non-natural amino acids including, but not limited to, modified amino acids. In certain embodiments, modified amino acids include natural amino acids that have been chemically modified to include a group, groups, or chemical moiety not naturally present on the amino acid. The peptide inhibitors of the invention may additionally include one or more D-amino acids. Still further, the peptide inhibitors of the invention may include amino acid analogs.

In certain embodiments, peptide inhibitors of the present invention include one or more modified or unnatural amino acids. In some embodiments of the present invention, a peptide inhibitor includes one or more non-natural amino acids shown in Table 1A. In certain embodiments, peptide inhibitors of the present invention include any of those described herein, including but not limited to any of those comprising an amino acid sequence or peptide inhibitor structure shown in any one of the tables herein.

The present invention also includes any of the peptide inhibitors described herein in either a free or a salt form. Thus, embodiments of any of the peptide inhibitors described herein (and related methods of use thereof) include a pharmaceutically acceptable salt of the peptide inhibitor.

The present invention also includes variants of any of the peptide inhibitors described herein, including but not limited to any of those comprising a sequence shown in any one of the tables herein, wherein one or more L-amino acid residue is substituted with the D isomeric form of the amino acid residue, e.g., an L-Ala is substituted with a D-Ala.

Peptide inhibitors described herein include isotopically-labeled peptide inhibitors. In particular embodiments, the present disclosure provides peptide inhibitors identical to any of those having or recited in the various formulas and structures presented herein, but for the fact that one or more atoms are replaced by an atom having an atomic mass or mass number different from the atomic mass or mass number usually found in nature. Examples of isotopes that can be incorporated into the present compounds include isotopes of hydrogen, carbon, nitrogen, oxygen, fluorine and chlorine, such as ²H, ³H, ¹³C, ¹⁴C, ¹⁵N, ¹⁸O, ¹⁷O, ³⁵S, ¹⁸F, ³⁶Cl, respectively. Certain isotopically-labeled compounds described herein, for example those into which radioactive isotopes such as ³H and ¹⁴C are incorporated, are useful in drug and/or substrate tissue distribution assays. Furthermore, substitution with isotopes such as deuterium, i.e., ²H, can afford certain therapeutic advantages resulting from greater metabolic stability, for example increased in vivo half-life or reduced dosage requirements.

The present invention also includes any of the peptide monomer inhibitors described herein linked to a linker moiety, including any of the specific linker moieties described herein. In particular embodiments, a linker is attached to an N-terminal or C-terminal amino acid, while in other embodiments, a linker is attached to an internal amino acid. In particular embodiments, a linker is attached to two internal amino acids, e.g., an internal amino acid in each of two monomer subunits that form a dimer. In some embodiments of the present invention, a peptide inhibitor is attached to one or more linker moieties shown.

The present invention also includes peptides and peptide dimers comprising a peptide having at least 90%, at least 95%, at least 98%, or at least 99% sequence identity to the peptide sequence of a peptide inhibitor described herein. In particular embodiments, peptide inhibitors of the present invention comprise a core peptide sequence and one or more N-terminal and/or C-terminal modification (e.g., Ac and NH₂) and/or one or more conjugated linker moiety and/or half-life extension moiety. As used herein, the core peptide sequence is the amino acid sequence of the peptide absent such modifications and conjugates. For example, for the peptide inhibitor: [Palm]-[isoGlu]-[PEG4]-[Pen]-NTWQ-[Pen]-[Phe[4-(2-aminoethoxy)]-[2-Nal]-[Aib]-[Lys(Ac)]-NN-NH₂ (SEQ ID NO:240), the core peptide sequence is: [Pen]-NTWQ-[Pen]-[Phe[4-(2-aminoethoxy)]-[2-Nal]-[Aib]-[Lys(Ac)]-NN (SEQ ID NO:240).

In certain embodiments, a peptide inhibitor or a monomer subunit of a peptide inhibitor of the present invention comprises, consists essentially of, or consists of 7 to 35 amino acid residues, 8 to 35 amino acid residues, 9 to 35 amino acid residues, 10 to 35 amino acid residues, 7 to 25 amino acid residues, 8 to 25 amino acid residues, 9 to 25 amino acid residues, 10 to 25 amino acid residues, 7 to 20 amino acid residues, 8 to 20 amino acid residues, 9 to 20 amino acid residues, 10 to 20 amino acid residues, 7 to 18 amino acid residues, 8 to 18 amino acid residues, 9 to 18 amino acid residues, or 10 to 18 amino acid residues, and, optionally, one or more additional non-amino acid moieties, such as a conjugated chemical moiety, e.g., a PEG or linker moiety. In particular embodiments, a peptide inhibitor of the present invention (or a monomer subunit thereof), including but not limited to those of any embodiments of Formula I, is greater than 10, greater than 12, greater than 15, greater than 20, greater than 25, greater than 30 or greater than 35 amino acids, e.g., 35 to 50 amino acids. In certain embodiments, a peptide inhibitor (or a monomer subunit thereof) is less than 50, less than 35, less than 30, less than 25, less than 20, less than 15, less than 12, or less than 10 amino acids. In particular embodiments, a monomer subunit of a peptide inhibitor (or a peptide monomer inhibitor) comprises or consists of 7, 8, 9, 10, 11, 12, 13, 14, 15, 16, 17, 18, 19, 20, 21, 22, 23, 24, 25, 26, 27, 28, 29, 30, 31, 32, 33, 34, or 35 amino acid residues. In particular embodiments, a monomer subunit of a peptide inhibitor of the present invention comprises or consists of 10 to 23 amino acid residues and, optionally, one or more additional non-amino acid moieties, such as a conjugated chemical moiety, e.g., a PEG or linker moiety. In various embodiments, the monomer subunit comprises or consists of 7 to 35 amino acid residues, 7 to 20 amino acid residues, 8 to 20 amino acid residues, 9 to 20 amino acid residues, 10 to 20 amino acid residues, 8 to 18 amino acid residues, 8 to 19 amino acid residues, 8 to 18 amino acid residues, 9 to 18 amino acid residues, or 10 to 18 amino acid residues. In particular embodiments of any of the various Formulas described herein, X comprises or consists of 7 to 35 amino acid residues, 8 to 35 amino acid residues, 9 to 35 amino acid residues, 10 to 35 amino acid residues, 7 to 25 amino acid residues, 8 to 25 amino acid residues, 9 to 25 amino acid residues, 10 to 25 amino acid residues, 7 to 18 amino acid residues, 8 to 18 amino acid residues, 9 to 18 amino acid residues, or 10 to 18 amino acid residues.

Certain illustrative peptide inhibitors described herein comprise 12 or more amino acid residues. However, the present invention also includes peptide inhibitors comprising a fragment of any of the peptide sequences described herein, including peptide inhibitors having 7, 8, 9, 10, or 11 amino acid residues. For example, peptide inhibitors of the present invention include peptides comprising or consisting of X4-X9, X4-X10, X4-X11, X4-X12, X4-X13, X4-X14, X4-X15, or X4-X16. In particular embodiments, the present invention includes peptide inhibitors having any of the sequences described herein, including but not limited to, those shown in any of the Formulas described herein or any of the tables provided herein, wherein one or more of X15, X16, X17, X18, X19, X20, X21, X22, or X23 is absent.

In particular embodiments of the present invention, the amino acid sequences of the peptide inhibitors are not present within an antibody, or are not present within a V_{H} or V_{L} region of an antibody.

### Peptide Inhibitors

Peptide inhibitors of the present invention include peptides having any of the amino acid sequences described herein, compounds having any of the structures described herein, including compounds comprising any of the peptide sequences described herein, and dimers of any of such peptides and compounds. Peptide inihibitors on the present invention include both peptides not having and those having a bond between X4 and X9, e.g., before and after a cross-link is introduced between X4 and X9. Illustrative peptides of the invention comprise an amino acid sequence or structure described in any of the accompanying tables.

In certain embodiments, the present invention includes a peptide inhibitor of an interleukin-23 receptor, or a pharmaceutically acceptable salt or solvate thereof, wherein the peptide inhibitor comprises an amino acid sequence of Formula (I): 
X0-X1-X2-X3-X4-X5-X6-X7-X8-X9-X10-X11-X12-X13-X14-X15-X16-X17-X18-X19-
X20-X21-X22-X23 (I) (SEQ ID NO:241)
 wherein
X0 is any amino acid or absent
X1 is any amino acid or absent:
X2 is any amino acid or absent;
X3 is any amino acid or absent;
X4 is Cys, (D)Cys), alpha-MeCys, Pen, (D)Pen, Abu, or (D)Abu;
X5 is any amino acid:
X6 is any amino acid:
X7 is any amino acid:
X8 is any amino acid;
X9 is Cys, (D)Cys), alpha-MeCys, Pen, (D)Pen, Abu, or (D)Abu;
X10 is Phe, Phe[4-(2-aminoethoxy)], Phe[4-(2-acetylaminoethoxy)], alpha-MeTyr, or Phe(4-CONH₂);
X11 is Trp, Trp(5-F), 1-Nal, Trp(7-Aza), Phe(2-Me), Phe(3-Me), Phe(4-Me), Phe(3,4-dimethoxy), or 2-Nal;
X12 is 4-amino-4-carboxy-tetrahydropyran (THP), alpha-MeLys, alpha-MeLeu, alpha-MeArg, alpha-MePhe, alpha-MeLeu, alpha-MeLys, alpha-MeAsn, alpha-MeTyr, or Aib;
X13 is Glu, Cit, Gln, alpha-MeArg, alpha-MeGlu, alpha-MeLeu, alpha-MeLys, alpha-Me-Asn, Lys(Ac), alpha-MeLys(Ac), Dab(Ac), Dap(Ac), homo-Lys(Ac),or Lys:
X14 is any amino acid;
X15 is any amino acid or absent,
X16 is any amino acid or absent;
X17 is any amino acid or absent;
X18 is any amino acid or absent;
X19 is any amino acid or absent; and
X20 is any amino acid or absent,
wherein X4 and X9 are capable of forming a bond with each other.

In related embodiments, the present invention includes a peptide inhibitor of an interleukin-23 receptor, or a pharmaceutically acceptable salt or solvate thereof, wherein the peptide inhibitor comprises an amino acid sequence of Formula (II): 
X0-X1-X2-X3-X4-X5-X6-X7-X8-X9-X10-X11-X12-X13-X14-X15-X16-X17-X18-X19-
X20-X21-X22-X23 (II) (SEQ ID NO:237)
 wherein
X0 is Gly, Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, or absent:
X1 is Gly, Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, or absent:
X2 is (D)Asp, Arg, (D)Arg, Phe, (D)Phe, 2-Nal, Thr, Leu, (D)Gln, (D)Asn, IsoGlu, Gly, Arg, Phe, Glu, Gln, Thr, (D)Glu, (D)Thr, (D)Leu, alpha-MeArg, alpha-MePhe, alpha-MeLeu, alpha-MeLys, alpha-MeAsn, alpha-MeTyr, or absent;
X3 is (D)Arg, (D)Tyr, Gly, alpha-MeArg, alpha-MePhe, alpha-MeLeu, alpha-MeLys, alpha-MeAsn, alpha-MeTyr, or absent;
X4 is Abu, Cys, (D)Cys), alpha-MeCys, (D)Abu, (D)Pen, or Pen;
X5 is Cit, Glu, Gly, Lys, Asn, Pro, alpha-MeGln, alpha-MeLys, alpha-MeLeu, alpha-MeAsn, Lys(Ac), alpha-MeLys(Ac), Dab(Ac), Dap(Ac), homo-Lys(Ac), or Gln;
X6 is Thr, Aib, Asp, Dab, Gly, Pro, Ser, alpha-MeGln, alpha-MeLys, alpha-MeLeu, alpha-MeAsn, alpha-MeThr, alpha-MeSer, or Val;
X7 is Trp, Trp(5-F), 1-Nal, 2-Nal, Phe(2-Me), Phe(3-Me), Phe(4-Me), Trp(7-Aza), or Phe(3,4-dimethoxy);
X8 is Gln, alpha-Me-Lys, alpha-MeLeu, alpha-MeLys(Ac), beta-homoGln, Cit, Glu, Phe, Asn, Thr, Val, Aib, alpha-MeGln, alpha-MeAsn, Lys(Ac), alpha-MeLys(Ac), Dab(Ac), Dap(Ac), homo-Lys(Ac), or Trp;
X9 is Cys, (D)Cys), alpha-MeCys, (D)Abu, (D)Pen, Pen or Abu;
X10 is Phe, Phe[4-(2-aminoethoxy)], Phe[4-(2-acetylaminoethoxy)], alpha-MeTyr, or Phe(4-CONH₂);
X11 is 2-Nal, Trp, Trp(5-F), Trp(7-Aza), Phe(2-Me), Phe(3-Me), Phe(4-Me), Phe(3,4-dimethoxy), or 1-Nal;
X12 is 4-amino-4-carboxy-tetrahydropyran (THP), alpha-MeLys, alpha-MeLeu, alpha-MeArg, alpha-MePhe, alpha-MeLeu, alpha-MeLys, alpha-MeAsn, alpha-MeTyr, or Aib;
X13 is Glu, Cit, Gln, alpha-MeArg, alpha-MeGlu, alpha-MeLeu, alpha-MeLys, alpha-Me-Asn, Lys(Ac), alpha-MeLys(Ac), Dab(Ac), Dap(Ac), homo-Lys(Ac), or Lys;
X14 is Asn, 2-Nap, Aib, Arg, Cit, Asp, Phe, Gly, Lys, Leu, Asn, n-Leu, Gln, Ser, Tic, Trp, alpha-MeGln, alpha-MeAsn, alpha-MeLys(Ac), Dab(Ac), Dap(Ac), homo-Lys(Ac),or Lys(Ac);
X15 is Asn, Aib, beta-Ala, Cit, Gln, Asp, alpha-MeGln, alpha-MeAsn, Lys(Ac), alpha-MeLys(Ac), Dab(Ac), Dap(Ac), homo-Lys(Ac),or absent;
X16 is Glu, Phe, Lys, Asn, Trp, Gly, Thr, Pro, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent;
X17 is Lys, Gly, Pro, The, Phe, Trp, Gln, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent:
X18 is Gly, Lys, Glu, Phe, Thr, Arg, Gln, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent:
X19 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent;
X20 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent;
X21 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent;
X22 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent; and
X23 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent,
wherein X4 and X9 are capable of forming a bond with each other.

**In** another aspect, the present invention provides a peptide inhibitor of an interleukin-23 receptor, or a pharmaceutically acceptable salt or solvate thereof, wherein the peptide inhibitor comprises an amino acid sequence of Formula (V): 
X0-X1-X2-X3-X4-X5-X6-X7-X8-X9-X10-X11-X12-X13-X14-X15-X16-X17-X18-X19-
X20-X21-X22-X23 (V) (SEQ ID NO:238)
 wherein
X0 is Gly, Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, or absent:
X1 is Gly, Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, or absent:
X2 is (D)Asp, Arg, (D)Arg, Phe, (D)Phe, 2-Nal, Thr, Leu, (D)Gln, (D)Asn, IsoGlu, Gly, Arg, Phe, Glu, Gln, Thr, (D)Glu, (D)Thr, (D)Leu, alpha-MeArg, alpha-MePhe, alpha-MeLeu, alpha-MeLys, alpha-MeAsn, alpha-MeTyr, or absent;
X3 is (D)Arg, (D)Tyr, Gly, alpha-MeArg, alpha-MePhe, alpha-MeLeu, alpha-MeLys, alpha-MeAsn, alpha-MeTyr, Lys(Ac), Lys(Y1-Ac), or absent, wherein Y1 is an amino acid;
X4 is Abu, Cys, (D)Cys), alpha-MeCys, (D)Abu, (D)Pen, Pen, or Pen(sulfoxide);
X5 is Cit, Glu, Gly, Lys, Asn, Pro, alpha-MeGln, alpha-MeLys, alpha-MeLeu, alpha-MeAsn, Lys(Ac), alpha-MeLys(Ac), Dab(Ac), Dap(Ac), homo-Lys(Ac), Gln, Asp, or Cys;
X6 is Thr, Aib, Asp, Dab, Gly, Pro, Ser, alpha-MeGln, alpha-MeLys, alpha-MeLeu, alpha-MeAsn, alpha-MeThr, alpha-MeSer, or Val;
X7 is Trp, Trp(5-F), 1-Nal, 2-Nal, Phe(2-Me), Phe(3-Me), Phe(4-Me), Trp(7-Aza), or Phe(3,4-dimethoxy);
X8 is Gln, alpha-Me-Lys, alpha-MeLeu, alpha-MeLys(Ac), beta-homoGln, Cit, Glu, Phe, Asn, Thr, Val, Aib, alpha-MeGln, alpha-MeAsn, Lys(Ac), alpha-MeLys(Ac), Dab(Ac), Dap(Ac), homo-Lys(Ac), 1-Nal, 2-Nal, or Trp;
X9 is Cys, (D)Cys), alpha-MeCys, (D)Abu, (D)Pen, Pen, or Abu;
X10 is Phe, Phe[4-(2-aminoethoxy)], Phe[4-(2-acetylaminoethoxy)], alpha-MeTyr, or Phe(4-CONH₂);
X11 is 2-Nal, Trp, Trp(5-F), Trp(7-Aza), Phe(2-Me), Phe(3-Me), Phe(4-Me), Phe(3,4-dimethoxy), or 1-Nal;
X12 is 4-amino-4-carboxy-tetrahydropyran (THP), alpha-MeLys, alpha-MeLeu, alpha-MeArg, alpha-MePhe, alpha-MeLeu, alpha-MeLys, alpha-MeAsn, alpha-MeTyr, Ala, cyclohexylAla, Lys, or Aib:
X13 is Glu, Cit, Gln, Lys(Ac), alpha-MeArg, alpha-MeGlu, alpha-MeLeu, alpha-MeLys, alpha-Me-Asn, alpha-MeLys(Ac), Dab(Ac), Dap(Ac), homo-Lys(Ac), Lys, pegylated Lys, b-homoGlu, or Lys(Y2-Ac), wherein Y2 is an amino acid;
X14 is Asn, 2-Nap, Aib, Arg, Cit, Asp, Phe, Gly, Lys, Leu, Asn, n-Leu, Gln, Ser, Tic, Trp, alpha-MeGln, alpha-MeAsn, alpha-MeLys(Ac), Dab(Ac), Dap(Ac), homo-Lys(Ac), or Lys(Ac);
X15 is Asn, Aib, beta-Ala, Cit, Gln, Asp, alpha-MeGln, alpha-MeAsn, Lys(Ac), alpha-MeLys(Ac), Dab(Ac), Dap(Ac), homo-Lys(Ac), or absent;
X16 is Glu, Phe, Lys, Asn, Trp, Gly, Thr, Pro, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, Ala, Asp, Tyr, Arg, Leu, Gln, Ser, Ile, 1-Nal, 2-Nal, (D)Ala, (D)Asp, (D)Tyr, (D)Arg, (D)Leu, (D)Ser, (D)Ile, or absent;
X17 is Lys, Gly, Pro, The, Phe, Trp, Gln, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent;
X18 is Gly, Lys, Glu, Phe, Thr, Arg, Gln, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent;
X19 is Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent;
X20 is Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent;
X21 is Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent;
X22 is Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent; and
X23 is Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent,
wherein the peptide inhibitor is cyclized via a bond between X4 and X9, and wherein the peptide inhibitor inhibits the binding of an interleukin-23 (IL-23) to an IL-23 receptor.

In certain embodiments of peptide inhibitors of Formula (V) or any other Formula disclosed herein, X3 is (D)Arg, (D)Tyr, Gly, alpha-MeArg, alpha-MePhe, alpha-MeLeu, alpha-MeLys, alpha-MeAsn, alpha-MeTyr, or absent. In certain embodiments, X3 is Lys(Ac) or Lys(Y1-Ac), wherein Y1 is an amino acid.

In certain embodiments of peptide inhibitors of Formula (V) or any other Formula disclosed herein, X4 is Abu, Cys, (D)Cys), alpha-MeCys, (D)Abu, (D)Pen, or Pen. In certain embodiments, X4 is Pen(sulfoxide).

In certain embodiments of peptide inhibitors of Formula (V) or any other Formula disclosed herein, X5 is Cit, Glu, Gly, Lys, Asn, Pro, alpha-MeGln, alpha-MeLys, alpha-MeLeu, alpha-MeAsn, Lys(Ac), alpha-MeLys(Ac), Dab(Ac), Dap(Ac), homo-Lys(Ac), or Gln. In certain embodiments, X5 is Asp or Cys.

In certain embodiments of peptide inhibitors of Formula (V) or any other Formula disclosed herein, X8 is Gln, alpha-Me-Lys, alpha-MeLeu, alpha-MeLys(Ac), beta-homoGln, Cit, Glu, Phe, Asn, Thr, Val, Aib, alpha-MeGln, alpha-MeAsn, Lys(Ac), alpha-MeLys(Ac), Dab(Ac), Dap(Ac), homo-Lys(Ac), or Trp. In certain embodiments, X8 is 1-Nal or 2-Nal.

In certain embodiments of peptide inhibitors of Formula (V) or any other Formula disclosed herein, X12 is 4-amino-4-carboxy-tetrahydropyran (THP), alpha-MeLys, alpha-MeLeu, alpha-MeArg, alpha-MePhe, alpha-MeLeu, alpha-MeLys, alpha-MeAsn, alpha-MeTyr, Ala, cyclohexylAla, Lys, or Aib. In certain embodiments, X12 is Ala, cyclohexylAla, or Lys.

In certain embodiments of peptide inhibitors of Formula (V) or any other Formula disclosed herein, X13 is Glu, Cit, Gln, Lys(Ac), alpha-MeArg, alpha-MeGlu, alpha-MeLeu, alpha-MeLys, alpha-Me-Asn, alpha-MeLys(Ac), Dab(Ac), Dap(Ac), homo-Lys(Ac), or Lys. In certain embodiments, X13 is Lys, pegylated Lys, b-homoGlu, or Lys(Y2-Ac), wherein Y2 is an amino acid;

In certain embodiments of peptide inhibitors of Formula (V) or any other Formula disclosed herein, X16 is Glu, Phe, Lys, Asn, Trp, Gly, Thr, Pro, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent. In certain embodiments, X16 is Ala, Asp, Tyr, Arg, Leu, Gln, Ser, Ile, 1-Nal, 2-Nal, (D)Ala, (D)Asp, (D)Tyr, (D)Arg, (D)Leu, (D)Ser, or (D)Ile.

In particular embodiments of peptides of Formula (I), Formula (II), or Formula (V), or any other peptide inhibitor disclosed herein or one or both monomer subunits thereof of a peptide dimer disclosed herein, the peptide inhibitor is cyclized via the bond between X4 and X9. In certain embodiments, the bond is a disulfide bond or a thioether bond. In certain embodiments, the peptide inhibitor inhibits the binding of an interleukin-23 (IL-23) to an IL-23 receptor.

In some embodiments, X4 and X9 of the peptides of Formulas (I), (II) and (V) (or any other peptide inhibitor disclosed herein or one or both monomer subunits thereof of a peptide dimer disclosed herein) are Cys, alpha-Me-Cys, Pen, or D-Pen, and the intramolecular bond is a disulfide bond. In certain embodiments, both X4 and X9 are Cys, or both X4 and X9 are Pen, and the intramolecular bond is a disulfide bond.

In one embodiment of peptides of Formulas (I), (II) and (V), or any other peptide inhibitor disclosed herein or one or both monomer subunits thereof of a peptide dimer disclosed herein, X4 is Abu, Cys, Pen, D-Pen, or D-Abu; X9 is Abu, Cys, Pen, D-Pen, or D-Abu; and the intramolecular bond is a thioether bond. In certain embodiments, X4 is Abu and X9 is Cys, and the intramolecular bond is a thioether bond. In certain embodiment, X4 is Abu or D-Abu; X9 is Cys: the intramolecular bond is a thioether bond; and wherein S of Cys is attached to γ-C of Abu. In certain embodiment, X4 is Abu or D-Abu; X9 is Cys; the intramolecular bond is a thioether bond; and the intermolecular bond form -X3-N(H)-C(H)(CH₂CH₂-S*-)C(O)-X5-; wherein "S*" is S of Cys.

In particular embodiments of peptides of Formulas (I), (II), (V), (IIIa) or (IVa), or any other peptide inhibitor disclosed herein or one or both monomer subunits thereof of a peptide dimer disclosed herein, X7 is (Trp(5-F)).

In certain embodiments of any of the peptide inhibitors or monomer subunits described herein, including those having peptides of Formulas (I), (II), (V), (IIIa), or (IVa), or any other peptide inhibitor disclosed herein or one or both monomer subunits thereof of a peptide dimer disclosed herein, X7 and X11 are both W. In certain embodiments of any of the peptide inhibitors or monomer subunits, X7 and X11 are not both Trp. In particular embodiments, X7 is Trp and X11 is not Trp. In certain embodiments, X7 is Trp; and X11 is 2-Nal or Trp(5-F). In certain embodiments, X7 and X11 are both W; and X10 is Phe[4-(2-aminoethoxy)], Phe[4-(2-acetylaminoethoxy)], Phe(4-OMe), or alpha-MeTyr. In certain embodiments, X7 and X11 are both W; X10 is Phe[4-(2-aminoethoxy)], Phe[4-(2-acetylaminoethoxy)], Phe(4-OMe) or alpha-Me-Tyr; and X11 is 2-Nal or Trp(5-F). In certain embodiments, X7 is Trp; X11 is 2-Nal or Trp (5-F); and X10 is Phe[4-(2-aminoethoxy)], Phe[4-(2-acetylaminoethoxy)], Phe(4-OMe) or alpha-Me-Tyr. In certain embodiments, X7 is Trp; X10 is Phe[4-(2-aminoethoxy)], Phe[4-(2-acetylaminoethoxy)] or Phe(4-OMe) or alpha-Me-Tyr; and X11 is 2-Nal or Trp(5-F).

In certain embodiments, X7 and X11 are both W, or X7 is Trp and X11 is 2-Nal or Trp(5-F); X10 is Phe[4-(2-acetylaminoethoxy)], Phe[4-(2-aminoethoxy)], Phe(CONH₂) or alpha-Me-Tyr; and X4 and X9 are amino acid residues capable of forming a thioether bond or a disulfide bond. In certain embodiments, both X4 and X9 are Pen and the intramolecule bond is a disulfide bond. In certain embodiments, X4 is Abu, X9 is Cys, and the intramolecular bond is a thioether bond.

In certain embodiments of peptides of Formula (I), Formula (II), Formula (V), Formula (IIIa), or Formula (IVa), or any other peptide inhibitor disclosed herein or one or both monomer subunits thereof of a peptide dimer disclosed herein, X5-X8 are selected from any of the following tetrapeptide sequences: QTWQ (SEQ ID NO:242), NDWQ (SEQ ID NO:243), N(Dab)WQ (SEQ ID NO:244), NT(1-Nal)Q (SEQ ID NO:245), NT(2-Nal)Q (SEQ ID NO:246), NTWE (SEQ ID NO:247), NTWF (SEQ ID NO:248), NTWQ (SEQ ID NO:249), NT[Trp(5-F)]Q (SEQ ID NO:250). In certain embodiments of peptides of Formula (I), Formula (II), Formula (V), Formula (IIIa), or Formula (IVa), X5-X8 are selected from any of the following tetrapeptide sequences: QTWQ (SEQ ID NO:242), QTWE (SEQ ID NO:251), ETWQ (SEQ ID NO:252), ETWE (SEQ ID NO:253), QTW-(alpha-MeLeu) (SEQ ID NO:254), QTW-(alpha-MeLys) (SEQ ID NO:255), QTW-(alpha-MeLys(Ac)) (SEQ ID NO:256), QTW-((D)Gln) (SEQ ID NO:257), QTW-(B-homoGln) (SEQ ID NO:258), QTWF (SEQ ID NO:259), QTWW (SEQ ID NO:260), QTW-[Aib] (SEQ ID NO:261), QTWT (SEQ ID NO:262), QTWV (SEQ ID NO:263), or QT-[Trp(5-F)]-Q (SEQ ID NO:264).

In certain embodiments, peptides of Formula (I), (II), (V), (IIIa) or (IVa), or any other peptide inhibitor disclosed herein or one or both monomer subunits thereof of a peptide dimer disclosed herein, comprise Asn residues at both X14 and X15.

In certain embodiments, peptides of Formula (I), (II), (V), (IIIa) or (IVa, or any other peptide inhibitor disclosed herein or one or both monomer subunits thereof of a peptide dimer disclosed herein, comprise at least one, at least two, at least three, or at least four amino acid residues N-terminal to X4. In certain embodiments, at least one, at least two, at least three, or at least four of the amino acid residues N-terminal to X4 are the same amino acid residue as each other. In certain embodiments, they are all the same residue as each other. In certain embodiments, at least one, at least two, at least three, or at least four of the amino acid residues N-terminal to X4 are selected from G, R, F, E, Q, T, and (D)-Arg). In certain embodiments, X0-X3 are the same as depicted in any of the corresponding residues shown in any of the peptides in Tables 2-5.

In certain embodiments, peptides of Formula (I), (II), (V), (IIIa) or (IV a), or any other peptide inhibitor disclosed herein or one or both monomer subunits thereof of a peptide dimer disclosed herein, comprise at least two, at least three, at least four, at least five, or at least six amino acid residues carboxy to X14. In certain embodiments, at least two, at least three, at least four, at least five, or at least six of the amino acid residues carboxy to X14 are the same amino acid residue as each other. In certain embodiments, they are all the same residue as each other. In certain embodiments, X14 and X15 are both N. In certain embodiments, at least two, at least three, or at least four of the amino acid residues carboxy to X14 are selected from N, E, F, K, W, G, T, P, K, F, or Q. In certain embodiments, X14-X23 are the same as depicted in any of the corresponding residues shown in any of the peptides in Tables 2-5.

In certain embodiments, peptides of Formula (I), (II), (V), (IIIa) or (IVa), or any other peptide inhibitor disclosed herein or one or both monomer subunits thereof of a peptide dimer disclosed herein, comprise an N-terminal Ac group. In certain embodiments, peptides of Formula (I), (II), (V), (IIIa) or (IVa), and any peptide inhibitor disclosed herein, comprise a C-terminal NH₂ group.

In certain embodiments, a peptide inhibitor of the present invention comprises or consists of an amino acid sequence shown herein, e.g., in Tables 2-6. In certain embodiments, a peptide inhibitor of the present invention has a structure shown herein, e.g., in Tables 2-6. In certain embodiments, any of the Phe[4-(2-aminoethoxy)] residues present in a peptide inhibitor described herein may be substituted by Phe[4-(2-acetylaminoethoxy)].

In additional embodiments, the present invention includes peptide inhibitors that comprise a peptide comprising a variant of any of the sequences of Formulas (I), (II), (V), (IIIa), or (IVa), or shown in Tables 2-6, which comprises an isostere replacement of one or more amino acid residues of X0-X23. In particular embodiments, the isostere replacement is a conservative amino acid substitution, and in certain embodiments, the isostere replacement is a substitution with an analog of an amino acid.

In additional embodiments, the present invention includes peptide inhibitors that comprise a peptide comprising a variant of any of the sequences of Formulas (I), (II), (V), (IIIa), or (IVa), or shown in Tables 2-6, which comprises different amino acid residues (or chemical entities) at one or both of amino acid residues X4 and X9, but wherein the amino acid residues at X4 and X9 are capable of binding each other, e.g., to form an intramolecule bond or triazole ring within the peptide. In particular embodiments, the bond is a disulfide bond, a thioether bond, a lactam bond, a triazole ring, a selenoether bond, a diselenide bond, or an olefin bond.

For example, in certain embodiments, X4 is Abu, 2-chloromethylbenzoic acid, mercapto-propanoic acid, mercapto-butyric acid, 2-chloro-acetic acid, 3-choro-propanoic acid, 4-chloro-butyric acid, or 3-chloro-isobutyric acid; X9 is Abu, Cys, Pen, hCys, D-Pen, D-Cys or D-hCys; and the intramolecular bond is a thioether bond. In certain embodiments, X4 is Abu and X9 is Pen, and the intramolecular bond is a thioether bond. In particular embodiments, X4 is a 2-methylbenzoyl moiety capable of forming a thioether bond with X9, and X9 is selected from Cys: N-Me-Cys, D-Cys, hCys, Pen, and D-Pen. In particular embodiments, X4 is Abu and X9 is Cys: and the intramolecular bond is a thioether bond. In particular instances, a peptide monomer, dimer, or subunit thereof of any of the Formulas and peptides described herein, X4 is selected from the group consisting of modified Ser, modified hSer (e.g., Homo-Ser-Cl), a suitable isostere, and corresponding D-amino acids. In other instances, X4 is an aliphatic acid having from one to four carbons and forming a thioether bond with X9. In some instances, X4 is a five- or six-membered alicyclic acid having a modified 2-methyl group that forms a thioether bond with X9. In some embodiments, X4 is a 2-methylbenzoyl moiety. In certain embodiments, X4 is selected from Cys, hCys, Pen, and a 2-methylbenzoyl moiety. In certain embodiments, X4 is selected from the group consisting of a modified Ser, a modified hSer, a suitable isostere, and corresponding D-amino acids. In one embodiment, X4 is a hSerCl (before the thioether bond is formed with X9 whereby the Cl is removed) or a hSer precursor (e.g., homoSer(O-TBDMS). In other instances, X4 is an aliphatic acid having from one to four carbons and forming a thioether bond with X9. In some instances, X4 is a five- or six-membered alicyclic acid having a modified 2-methyl group that forms a thioether bond with X9. In some instances, X4 is a 2-methylbenzoyl moiety. In certain embodiments wherein X4 is not an amino acid but is a chemical moiety that binds to X9, X1, X2, and X3 are absent, and X4 is conjugated to or bound to X5. In some embodiments, the amino acid directly carboxyl to X9 is an aromatic amino acid. In certain embodiments, X4 is an amino acid, while in other embodiments, X4 is another chemical moiety capable of binding to X9, e.g., to form a thioether bond. In particular embodiments, X4 is another chemical moiety selected from any of the non-amino acid moieties described herein for X4. In particular embodiments wherein X4 is another chemical moiety, X1, X2 and X3 are absent, and the another chemical moiety is bound to or conjugated to X5. In certain embodiments, X4 is defined as a chemical moiety including a group such as a chloride, e.g., in 2-chloromethylbenzoic acid, 2-chloro-acetic acid, 3-choropropanoic acid, 4-chlorobutyric acid, 3-chloroisobutyric acid. However, the skilled artisan will appreciate that once the peptide has undergone ring closing cyclization to form a thioether bond between X4 and X9, the chloride group is no longer present. The description of chemical moieties at X4 that include a reactant group such as chloride thus means both the group with the chloride and also the group without the chloride, i.e., after formation of the bond with X9.The present invention also includes peptides comprising the same structure as shown in any of the other Formulas or tables described herein, but where the thioether bond is in the reverse orientation. In such embodiments of the invention, it may generally be considered that the amino acid residues or other chemical moieties shown at X4 are instead present at X9, and the amino acid residues shown at X9 are instead present at X4, i.e., the amino acid residue comprising the sulfur of the resulting thioether bond is located at X4 instead of X9, and the amino acid residue or other moiety having a carbon side chain capable of forming a thioether bond with X4 is located at X9. In this reverse orientation, however, the amino acid or chemical moiety at position X9 is one that comprises a free amine. For example, in particular embodiments, the amino acid at X9 is a protected homoserine, such as, e.g., homoserine (OTBDMS). Thus, in particular reverse orientation embodiments of peptide inhibitors of any of the Formulas described herein, X9 is an amino acid residue having a side chain with one or two carbons, and forming a thioether bond with X4, and X4 is selected from the group consisting of Cys, N-Me-Cys, D-Cys, HCys, Pen, and D-Pen. Specific examples of amino acid residues and other chemical moieties present at corresponding positions of other Formulas and tables are described herein.

In certain peptides that form a thioether bond between X4 and X9, X4 is an amino acid, aliphatic acid, alicyclic acid or modified 2- methyl aromatic acid having a carbon side chain capable of forming a thioether bind with X9, and X9 is a sulfur-containing amino acid capable of forming a thioether bond with X4. In certain embodiments, X4 is Cys, Pen, hCys, D-Pen, D-Cys, D-hCys, Met, Glu, Asp, Lys, Orn, Dap, Dab, D-Dap, D-Dab, D-Asp, D-Glu, D-Lys, Sec, 2-chloromethylbenzoic acid, mercapto-propanoic acid, mercapto-butyric acid, 2-chloro-acetic acid, 3-choro-propanoic acid, 4-chloro-butyric acid, 3-chloro-isobutyric acid, Abu, β-azido-Ala-OH, propargylglycine, 2-(3'-butenyl)glycine, 2-allylglycine, 2-(3'-butenyl)glycine, 2-(4'-pentenyl)glycine, 2-(5'-hexenyl)glycine; and X9 is X9 is Cys, Pen, hCys, D-Pen, D-Cys, D-hCys, Glu, Lys, Orn, Dap, Dab, D-Dap, D-Dab, D-Asp, D-Glu, D-Lys, Asp, Leu, Val, Phe, Ser, Sec, Abu, β-azido-Ala-OH, propargylglycine, 2-2-allylglycine, 2-(3'-butenyl)glycine, 2-(4'-pentenyl)glycine, or 2-(5'-hexenyl)glycine. In certain embodiments, X4 is Abu, 2-chloromethylbenzoic acid, mercapto-propanoic acid, mercapto-butyric acid, 2-chloro-acetic acid, 3-chloro-propanoic acid, 4-chloro-butyric acid, 3-chloro-isobutyric acid; and X9 is Abu, Cys, Pen, hCys, D-Pen, D-Cys, or D-hCys.

In one embodiment, X4 and X9 are each Glu, Asp, Lys, Orn, Dap, Dab, D-Dap, D-Dab, D-Asp, D-Glu or D-Lys, and the intramolecular bond is a lactam bond.

In certain embodiments, X4 and X9 are each β-azido-Ala-OH or propargylglycine, and the peptide inhibitor (or monomer subunit) is cyclized through a triazole ring.

In certain embodiments, X4 and X9 are each 2-allylglycine, 2-(3'-butenyl)glycine, 2-(4'-pentenyl)glycine, or 2-(5'-hexenyl)glycinem and the peptide inhibitor (or monomer subunit) is cyclized via ring closing methasis to give the corresponding olefin / "stapled peptide."

In certain embodiments, X4 is 2-chloromethylbenzoic acid, mercapto-propanoic acid, mercapto-butyric acid, 2-chloro-acetic acid, 3-choro-propanoic acid, 4-chloro-butyric acid, 3-chloro-isobutyric acid, or hSer(Cl); X9 is hSer(Cl), Cys, Pen, hCys, D-Pen, D-Cys or D-hCys; and the intramolecular bond is a thioether bond. In certain embodiments, X4 is 2-chloromethylbenzoic acid or hSer(Cl); X9 is Cys or Pen, and the intramolecular bond is a thioether bond. In certain embodiments, X4 is Abu, and X9 is Cys or Pen.

**In** certain embodiments, X4 is 2-chloromethylbenzoic acid, 2-chloro-acetic acid, 3-choro-propanoic acid, 4-chloro-butyric acid, 3-chloro-isobutyric acid, Abu or Sec; X9 is Abu or Sec; and the intramolecular bond is a selenoether bond.

In certain embodiments, the intramolecular bond between X4 and X9 is a diselenide bond.

In some embodiments, with respect to any of the formulas described herein, X3 is (D)Arg, (D)Tyr, Gly, alpha-MeArg, alpha-MePhe, alpha-MeLeu, alpha-MeLys, alpha-MeAsn, alpha-MeTyr, or absent. In other embodiments, X3 is Lys(Ac) or Lys(Y1-Ac); wherein Y1 is an amino acid. In one embodiment, Y1 is a natural amino acid. In another embodiment, Y 1 is a (D) amino acid. In certain embodiments, Y1 is Glu, Phe, Tyr, Ser, Arg, Leu, or Pro.

In some embodiments, with respect to any of the formulas described herein, X5 is Cit, Glu, Gly, Lys, Asn, Pro, alpha-MeGln, alpha-MeLys, alpha-MeLeu, alpha-MeAsn, Lys(Ac), alpha-MeLys(Ac), Dab(Ac), Dap(Ac), homo-Lys(Ac), or Gln. In other embodiments, X5 is Asp, or Cys.

In some embodiments, with respect to any of the formulas described herein, X8 is Gln, alpha-Me-Lys, alpha-MeLeu, alpha-MeLys(Ac), beta-homoGln, Cit, Glu, Phe, Asn, Thr, Val, Aib, alpha-MeGln, alpha-MeAsn, Lys(Ac), alpha-MeLys(Ac), Dab(Ac), Dap(Ac), homo-Lys(Ac), or Trp. In other embodiments, X8 is 1-Nal, or 2-Nal.

In some embodiments, with respect to any of the formulas described herein, X12 is 4-amino-4-carboxy-tetrahydropyran (THP), alpha-MeLys, alpha-MeLeu, alpha-MeArg, alpha-MePhe, alpha-MeLeu, alpha-MeLys, alpha-MeAsn, alpha-MeTyr, or Aib. In other embodiments, X12 is Ala, or cyclohexylAla, or Lys. In a particular embodiment, X12 is cyclohexylAla. In certain embodiments, X12 is conjugated, e.g., to a chemical substituent. In certain embodiment, X12 is Lys, and Lys is conjugated, e.g., to a chemical substituent.

In some embodiments, with respect to any of the formulas described herein, X13 is Glu, Cit, Gln, Lys(Ac), alpha-MeArg, alpha-MeGlu, alpha-MeLeu, alpha-MeLys, alpha-Me-Asn, alpha-MeLys(Ac), Dab(Ac), Dap(Ac), homo-Lys(Ac), or Lys. In other embodiments, X13 is Lys, pegylated Lys, b-homoGlu, or Lys(Y2-Ac); Y2 is an amino acid. In one embodiment, Y2 is an natural amino acid. In another embodiment, Y2 is an (D) amino acid. In certain embodiments, Y2 is Glu, Phe, Asn, Thr, Asp, Tyr, Ser, Arg, Leu, or Pro. In certain embodiments, Y2 is (D)Glu, (D)Phe, (D)Asn, (D)Thr, (D)Asp, (D)Tyr, (D)Ser, (D)Arg, or (D)Leu.

In some embodiments, with respect to any of the formulas described herein, X16 is Glu, Phe, Lys, Asn, Trp, Gly, Thr, Pro, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent. In other embodiments, X¹⁶ is Ala, Asp, Tyr, Arg, Leu, Gln, Ser, Ile, 1-Nal, 2-Nal, (D)Ala, (D)Asp, (D)Tyr, (D)Arg, (D)Leu, (D)Ser, or (D)Ile.

In certain embodiments, the present invention includes a peptide inhibitor of an interleukin-23 receptor, wherein the peptide inhibitor has the structure of Formula XI:

R¹-X-R² (XI)

or a pharmaceutically acceptable salt or solvate thereof,
wherein R¹ is a bond, hydrogen, a C1-C6 alkyl, a C6-C12 aryl, a C6-C12 aryl, a C1-C6 alkyl, a C1-C20 alkanoyl, an alkylsulphonate, an acid, γ-Glu or pGlu, appended to the N-terminus, and including PEGylated versions (e.g., 200 Da to 60,000 Da), alone or as a spacer of any of the foregoing;
R² is a bond, OH or NH₂; and
X is an amino acid sequence of 8 to 20 amino acids or 8 to 35 amino acids.

In one embodiment, R¹ is a bond, hydrogen, or a C1-C20 alkanoyl. In another embodiment, R¹ is a bond, hydrogen, or Ac. In a particular embodiment, R¹ is Ac. In another particular embodiment, R¹ is PEGylated.

In one embodiment, R² is OH. In a particular embodiment, R² is NH₂.

In particular embodiments of peptide inhibitor of Formula XI, X comprises or consists of the sequence of Formula XII:
X2-X3-X4-X5-T-X7-X8-X9-X10-X11-X12-X13-X14-X15-X16 (XII) (SEQ ID NO:275) wherein
X2 is Arg, (D)Arg, Gln, or absent;
X3 is (D)Arg, Phe, (D)Phe, Lys, (D)Lys, Lys(Y1-Ac), (D)Lys(Y1-Ac), or absent, wherein Y1 is an amino acid or Y1 is absent;
X4 is Cys, (D)Cys), alpha-MeCys, Abu, (D)Pen, Pen, (D)Pensulfoxide, or Pensulfoxide;
X5 is Cit, Lys, Asn, Asp, Glu, Lys(Ac), or Gln;
X7 is Trp, substituted Trp, or 1-Nal;
X8 is Gln, Lys, Lys(Ac), a-MeLeu, Cit, Glu, 1-Nal, 2-Nal, Trp, substituted Trp, or Lys(Peg12);
X9 is Cys, Abu, or Pen;
X10 is Phe, Phe[4-(2-aminoethoxy)], Phe(Cmd), or Phe[4-(2-acetylaminoethoxy)];
X11 is 2-Nal, Phe(2-Me), Phe(3-Me), Phe(4-Me), Phe(3,4-dimethoxy), or 1-Nal;
X12 is alpha-MeLeu, Aib, Lys, cyclohexylAla, tetrahydropyranAla, or Lys(Peg12)
X13 is Glu, b-homoGlu, Lys, (D)Lys, Lys(Y2-Ac), or (D)Lys(Y2-Ac), wherein Y2 is an amino acid or Y2 is absent:
X14 is Asn, Asp, Cit, or Lys(Ac);
X15 is Asn, Lys, Lys(Ac), Cit, Asp, Gly, Ala, b-Ala, or Sarc;
X16 is an amino acid, or absent: substituted Trp is Trp substituted with halo, or azaTrp:
wherein X4 and X9 are capable of forming a disulfide bond or a thioether bond or are linked via a disulfide bond or a thioether bond.

In one embodiment, substituted Trp is fluoro substituted Trp. In another embodiment, substituted Trp is azaTrp. In a particular embodiment, substituted Trp is (5-F)Trp. In another particular embodiment, substituted Trp is (7-aza)Trp.

In one embodiment, X2 is Arg, (D)Arg, or absent. In a particular embodiment, X2 is absent.

In one embodiment, X7 is Trp or Trp(5-F). In a particular embodiment, X7 is Trp.

In one embodiment, X10 is Phe[4-(2-aminoethoxy)].

In one embodiment, X11 is 2-Nal.

In one embodiment, X15 is Asn.

In particular embodiments, with respect to Formula XI, X comprises or consists of the sequence of Formula XIII:
X3-X4-X5-T-Trp-X8-X9-Phe[4-(2-aminoethoxy)]-(2-Nal)-X12-X13-X14-Asn-X16 (XIII) (SEQ ID NO:276) wherein X3, X4, X5, X8, X9, X12, X13, X14, or X16 are as described for Formula XII.

In one embodiment, X4 and X9 are joined together to form a disulfide bond. In one embodiment, X4 and X9 are joined together to form a thioether bond.

In one embodiment, X4 is Abu or Pen. In one embodiment, X4 is Abu; and X4 and X9 are joined together to form a thioether bond. In another embodiment, X4 is Pen, and X4 and X9 are joined together to form a disulfide bond.

In a particular embodiment, with respect to Formula XI, X comprises or consists of the sequence of Formula XIVa or XIVb:
X3-Abu-X5-T-Trp-X8-X9-Phe[4-(2-aminoethoxy)]-(2-Nal)-X12-X13-X14-Asn-X16 (XIVa) (SEQ ID NO:277); or
X3-Pen-X5-T-Trp-X8-X9-Phe[4-(2-aminoethoxy)]-(2-Nal)-X12-X13-X14-Asn-X16 (XIVb) (SEQ ID NO:278);
wherein X3, X5, X8, X9, X12, X13, X14, or X16 are as described for Formula XII.

In one embodiment, X5 is Asn or Gln.

In a particular embodiment, with respect to Formula XI, X comprises or consists of the sequence of Formula XVa, XVb, XVc or XVd:
X3-Abu-Asn-T-Trp-X8-X9-Phe[4-(2-aminoethoxy)]-(2-Nal)-X12-X13-X14-Asn-X16 (XVa) (SEQ ID NO:279);
X3-Pen-Asn-T-Trp-X8-X9-Phe[4-(2-aminoethoxy)]-(2-Nal)-X12-X13-X14-Asn-X16 (XVb) (SEQ ID NO:280);
X3-Abu-Gln-T-Trp-X8-X9-Phe[4-(2-aminoethoxy)]-(2-Nal)-X12-X13-X14-Asn-X16 (XVc) (SEQ ID NO:281); or
X3-Pen-Gln-T-Trp-X8-X9-Phe[4-(2-aminoethoxy)]-(2-Nal)-X12-X13-X14-Asn-X16 (XVd) (SEQ ID NO:282);
wherein X3, X8, X9, X12, X13, X14, or X16 are as described for Formula XII.

In one embodiment, X4 and X9 are joined together to form a disulfide bond or a thioether bond.

In one embodiment, X9 is Cys or Pen.

In a particular embodiment, with respect to Formula XI, X comprises or consists of the sequence of Formula XVIa, XVIb, XVIc, XVId, XVIe, XVIf, XVIg, or XVIh:,
X3-Abu-Asn-T-Trp-X8-Cys-Phe[4-(2-aminoethoxy)]-(2-Nal)-X12-X13-X14-Asn-X16 (XVIa) (SEQ ID NO:283);
X3-Pen-Asn-T-Trp-X8-Cys-Phe[4-(2-aminoethoxy)]-(2-Nal)-X12-X13-X14-Asn-X16 (XVIb) (SEQ ID NO:284);
X3-Abu-Gln-T-Trp-X8-Cys-Phe[4-(2-aminoethoxy)]-(2-Nal)-X12-X13-X14-Asn-X16 (XVIc) (SEQ ID NO:285);
X3-Pen-Gln-T-Trp-X8-Cys-Phe[4-(2-aminoethoxy)]-(2-Nal)-X12-X13-X14-Asn-X16 (XVId) (SEQ ID NO:286);
X3-Abu-Asn-T-Trp-X8-Pen-Phe[4-(2-aminoethoxy)]-(2-Nal)-X12-X13-X14-Asn-X16 (XVIe) (SEQ ID NO:287):
X3-Pen-Asn-T-Trp-X8-Pen-Phe[4-(2-aminoethoxy)]-(2-Nal)-X12-X13-X14-Asn-X16 (XVIf) (SEQ ID NO:288):
X3-Abu-Gln-T-Trp-X8-Pen-Phe[4-(2-aminoethoxy)]-(2-Nal)-X12-X13-X14-Asn-X16 (XVIg) (SEQ ID NO:289); or
X3-Pen-Gln-T-Trp-X8-Pen-Phe[4-(2-aminoethoxy)]-(2-Nal)-X12-X13-X14-Asn-X16 (XVIh) (SEQ ID NO:290);
wherein X3, X8, X12, X13, X14, or X16 are as described for Formula XII.

In one embodiment, with respect to Formula XVIa-XVIh, Abu and Cys, Pen and Cys, Abu and Pen, or Pen and Pen joined together to form a disulfide bond or a thioether bond.

In one embodiment, with respect to Formula XI-XVIh, X12 is tetrahydropyran-Ala (THP-Ala) or a-MeLeu.

In one embodiment, with respect to Formula XI-XVIh, X14 is Lys(Ac) or Asn. In a particular embodiment, X14 is Asn.

In a particular embodiment, with respect to Formula XI, X comprises or consists of the sequence of Formula XVIIa, XVIIb, XVIIc, XVIId, XVIIe, XVIIf, XVIIg, or XVIIh:
X3-Abu-Asn-T-Trp-X8-Cys-Phe[4-(2-aminoethoxy)]-(2-Nal)-[THP-Ala]-X13-Asn-Asn-X16 (XVIIa) (SEQ ID NO:291);
X3-Pen-Asn-T-Trp-X8-Cys-Phe[4-(2-aminoethoxy)]-(2-Nal)-[THP-Ala]-X13-Asn-Asn-X16 (XVIIb) (SEQ ID NO:292);
X3-Abu-Gln-T-Trp-X8-Cys-Phe[4-(2-aminoethoxy)]-(2-Nal)-[THP-Ala]-X13-Asn-Asn-X16 (XVIIc) (SEQ ID NO:293);
X3-Pen-Gln-T-Trp-X8-Cys-Phe[4-(2-aminoethoxy)]-(2-Nal)-[THP-Ala]-X13-Asn-Asn-X16 (XVIId) (SEQ ID NO:294);
X3-Abu-Asn-T-Trp-X8-Pen-Phe[4-(2-aminoethoxy)]-(2-Nal)-[THP-Ala]-X13-Asn-Asn-X16 (XVIIe) (SEQ ID NO:295);
X3-Pen-Asn-T-Trp-X8-Pen-Phe[4-(2-aminoethoxy)]-(2-Nal)-[THP-Ala]-X13-Asn-Asn-X16 (XVIIf) (SEQ ID NO:296);
X3-Abu-Gln-T-Trp-X8-Pen-Phe[4-(2-aminoethoxy)]-(2-Nal)-[THP-Ala]-X13-Asn-Asn-X16 (XVIIg) (SEQ ID NO:297); or
X3-Pen-Gln-T-Trp-X8-Pen-Phe[4-(2-aminoethoxy)]-(2-Nal)-[THP-Ala]-X13-Asn-Asn-X16 (XVIIh) (SEQ ID NO:298):
wherein X3, X8, X12, X13, or X16 are as described for Formula XII.

In a particular embodiment, with respect to Formula XI, X comprises or consists of the sequence of Formula XVIIIa, XVIIIb, XVIIIc, XVIIId, XVIIIe, XVIIIf, XVIIIg, or XVIIIh:,
X3-Abu-Asn-T-Trp-X8-Cys-Phe[4-(2-aminoethoxy)]-(2-Nal)-[a-MeLeu]-X13-Asn-Asn-X16 (XVIIIa) (SEQ ID NO:299);
X3-Pen-Asn-T-Trp-X8-Cys-Phe[4-(2-aminoethoxy)]-(2-Nal)-[a-MeLeu]-X13-Asn-Asn-X16 (XVIIIb) (SEQ ID NO:300);
X3-Abu-Gln-T-Trp-X8-Cys-Phe[4-(2-aminoethoxy)]-(2-Nal)-[a-MeLeu]-X13-Asn-Asn-X16 (XVIIIc) (SEQ ID NO:301);
X3-Pen-Gln-T-Trp-X8-Cys-Phe[4-(2-aminoethoxy)]-(2-Nal)-[a-MeLeu]-X13-Asn-Asn-X16 (XVIIId) (SEQ ID NO:302);
X3-Abu-Asn-T-Trp-X8-Pen-Phe[4-(2-aminoethoxy)]-(2-Nal)-[a-MeLeu]-X13-Asn-Asn-X16 (XVIIIe) (SEQ ID NO:303);
X3-Pen-Asn-T-Trp-X8-Pen-Phe[4-(2-aminoethoxy)]-(2-Nal)-[a-MeLeu]-X13-Asn-Asn-X16 (XVIIIf) (SEQ ID NO:304);
X3-Abu-Gln-T-Trp-X8-Pen-Phe[4-(2-aminoethoxy)]-(2-Nal)-[a-MeLeu]-X13-Asn-Asn-X16 (XVIIIg) (SEQ ID NO:305); or
X3-Pen-Gln-T-Trp-X8-Pen-Phe[4-(2-aminoethoxy)]-(2-Nal)-[a-MeLeu]-X13-Asn-Asn-X16 (XVIIIh) (SEQ ID NO:306);
wherein X3, X8, X13, or X16 are as described for Formula XII.

In certain embodiments, with respect to Formula XI-XVIIIh, X3 is (D)Arg, Phe, (D)Phe, Lys, (D)Lys, Lys(Y1-Ac), (D)Lys(Y1-Ac), or absent; and Y1 is an amino acid, or absent. In one embodiment, Y1 is a natural amino acid. In another embodiment, Y1 is an (D) amino acid. In certain embodiments, Y1 is Glu, Phe, Tyr, Ser, Arg, Leu, or Pro. In a particular embodiment, X3 is absent. In another particular embodiment, X3 is (D)Arg. In a more particular embodiment, X3 is Lys(Y1-Ac) or (D)Lys(Y1-Ac). In one embodiment, Y1 is Leu. In another embodiment, Y1 is Glu. In another embodiment, Y1 is Phe. In another embodiment, Y1 is Tyr. In another embodiment, Y1 is Pro. In another embodiment, Y1 is Ser. In another embodiment, Y1 is Arg.

In certain embodiments, with respect to Formula XI-XVIIIh, X8 is Gln, Lys, Lys(Ac), a-MeLeu, Cit, or Glu. In a particular embodiment, X8 is Gln, Glu, Lys(Ac) or a-MeLeu. In another particular embodiment, X8 is Gln or Lys(Ac). In a more particular embodiment, X8 is Gln.

In certain embodiments, with respect to Formula XI-XVIIIh, X13 is Glu, b-homoGlu, Lys, (D)Lys, Lys(Y2-Ac), or (D)Lys(Y2-Ac); and Y2 is an amino acid, or Y2 is absent. In a particular embodiment, X13 is Glu, Cit, Lys, or Lys(Ac). In a more particular embodiment, X13 is Glu or Lys(Ac).

In certain embodiments, with respect to Formula XI-XVIIIh, X16 is absent or is an amino acid. In a particular embodiment, X16 is absent. In a more particular embodiment, X16 is an amino acid. In one embodiment, the amino acid is Sar, Lys, (D)Lys, Ahx, b-Ala, Gly, Arg, (D)Arg, Ile, Gln, (D)Gln, Tyr, Ser, (D)Ser, (D)Tyr, Ala, Trp, Asp, or (D)Asp.

In particular embodiments of peptide inhibitors comprising a variant of any of Fromulas (I), (II), (V), (IIIa), or (IVa) wherein X4 is not an amino acid, then X1, X2, and X3 are absent. In certain embodiments, X1 is a D-amino acid or absent. In certain embodiments, X2 is a D-amino acid or absent. In certain embodiments, X3 is a D-amino acid or absent. In certain embodiments, X16 is a D-amino acid or absent. In certain embodiments, X17 is a D-amino acid or absent. In certain embodiments, X18 is a D-amino acid or absent. In certain embodiments, X19 is a D-amino acid or absent. In certain embodiments, X20 is a D-amino acid or absent.

In particular embodiments, peptides of Formula (I), (II), (V), (IIIa) or (IVa) are conjugated to one or more chemical substituents, such as lipophilic substituents and polymeric moieties, which may be referred to herein as half-life extension moieties. In particular embodiments, peptides of Formula (I), (II), (V), (IIIa) or (IVa) are conjugated to one or more detectable marker or dye.

In some embodiments, wherein a peptide of the invention is conjugated to an acidic compound such as, e.g., isovaleric acid, isobutyric acid, valeric acid, and the like, the presence of such a conjugation is referenced in the acid form. So, for example, but not to be limited in any way, instead of indicating a conjugation of isovaleric acid to a peptide by referencing isovaleroyl (e.g., isovaleroyl-[Pen]-QTWQ[Pen]-[Phe(4-OMe)]-[2-Nal]-[a-MeLys]-[Lys(Ac)]-NG-NH₂ (SEQ ID NO:307) in some embodiments, the present application references such a conjugation as isovaleric acid-[Pen]-QTWQ[Pen]-[Phe(4-OMe)]-[2-Nal]-[a-MeLys]-[Lys(Ac)]-NG-NH₂ (SEQ ID NO:307).

In certain embodiments, peptide inhibitors do not include compounds, disclosed in any or all of PCT Application No. PCT/US2014/030352, PCT Application No. PCT/US2015/038370 Pct Application No. PCT/US2015/040658, or PCT Application No. PCT/US2016/042680.

### Illustrative Peptide Inhibitors Comprising Pen-Pen Disulfide Bonds

In certain embodiments, the present invention includes a peptide inhibitor of an interleukin-23 receptor, wherein the peptide inhibitor has the structure of Formula III:

R¹-X-R² (III)

or a pharmaceutically acceptable salt or solvate thereof,
wherein R¹ is a bond, hydrogen, a C1-C6 alkyl, a C6-C12 aryl, a C6-C12 aryl, a C1-C6 alkyl, a C1-C20 alkanoyl, an alkylsulphonate, an acid, γ-Glu or pGlu, appended to the N-terminus, and including PEGylated versions (e.g., 200 Da to 60,000 Da), alone or as a spacer of any of the foregoing;
R² is a bond, OH or NH₂; and
X is an amino acid sequence of 8 to 20 amino acids or 8 to 35 amino acids.

In particular embodiments of peptide inhibitor of Formula III, X comprises or consists of the sequence of Formula IIIa: 
X0-X1-X2-X3-X4-X5-X6-X7-X8-X9-X10-X11-X12-X13-X14-X15-X16-X17-X18-X19-
X20-X21-X22-X23 (IIIa) (SEQ ID NO:265)
 wherein
X0 is Gly, Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, or absent;
X1 is Gly, Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, or absent;
X2 is (D)Asp, Arg, (D)Arg, Phe, (D)Phe, 2-Nal, Thr, Leu, (D)Gln, (D)Asn, IsoGlu, Gly, Arg, Phe, Glu, Gln, Thr, (D)Glu, (D)Thr, (D)Leu, alpha-MeArg, alpha-MePhe, alpha-MeLeu, alpha-MeLys, alpha-MeAsn, alpha-MeTyr, or absent;
X3 is (D)Arg, (D)Tyr, Gly, alpha-MeArg, alpha-MePhe, alpha-MeLeu, alpha-MeLys, alpha-MeAsn, alpha-MeTyr, or absent;
X4 is Cys, (D)Cys), alpha-MeCys, (D)Pen, or Pen;
X5 is Cit, Glu, Gly, Lys, Asn, Pro, alpha-MeGln, alpha-MeLys, alpha-MeLeu, alpha-MeAsn, Lys(Ac), alpha-MeLys(Ac), Dab(Ac), Dap(Ac), homo-Lys(Ac), or Gln;
X6 is Thr, Aib, Asp, Dab, Gly, Pro, Ser, alpha-MeGln, alpha-MeLys, alpha-MeLeu, alpha-MeAsn, alpha-MeThr, alpha-MeSer, or Val;
X7 is Trp, Trp(5-F), 1-Nal, 2-Nal, Phe(2-Me), Phe(3-Me), Phe(4-Me), Trp(7-Aza), or Phe(3,4-dimethoxy);
X8 is Gln, alpha-Me-Lys, alpha-MeLeu, alpha-MeLys(Ac), beta-homoGln, Cit, Glu, Phe, Asn, Thr, Val, Aib, alpha-MeGln, alpha-MeAsn, Lys(Ac), alpha-MeLys(Ac), Dab(Ac), Dap(Ac), homo-Lys(Ac),or Trp;
X9 is Cys, (D)Cys), alpha-MeCys, (D)Pen, or Pen;
X10 is Phe, Phe[4-(2-aminoethoxy)], Phe[4-(2-acetylaminoethoxy)], alpha-MeTyr, or Phe(4-CONH₂);
X11 is 2-Nal, Trp, Trp(5-F), Trp(7-Aza), Phe(2-Me), Phe(3-Me), Phe(4-Me), Phe(3,4-dimethoxy), or 1-Nal:
X12 is 4-amino-4-carboxy-tetrahydropyran (THP), alpha-MeLys, alpha-MeLeu, alpha-MeArg, alpha-MePhe, alpha-MeLeu, alpha-MeLys, alpha-MeAsn, alpha-MeTyr, or Aib:
X13 is Glu, Cit, Gln, alpha-MeArg, alpha-MeGlu, alpha-MeLeu, alpha-MeLys, alpha-MeAsn, Lys(Ac), alpha-MeLys(Ac), Dab(Ac), Dap(Ac), homo-Lys(Ac), or Lys;
X14 is Asn, 2-Nap, Aib, Arg, Cit, Asp, Phe, Gly, Lys, Leu, Asn, n-Leu, Gln, Ser, Tic, Trp, alpha-MeGln, alpha-MeAsn, alpha-MeLys(Ac), Dab(Ac), Dap(Ac), homo-Lys(Ac), or Lys(Ac);
X15 is Asn, Aib, beta-Ala, Cit, Gln, Asp, alpha-MeGln, alpha-MeAsn, Lys(Ac), alpha-MeLys(Ac), Dab(Ac), Dap(Ac), homo-Lys(Ac),or absent;
X16 is Glu, Phe, Lys, Asn, Trp, Gly, Thr, Pro, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent;
X17 is Lys, Gly, Pro, The, Phe, Trp, Gln, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent;
X18 is Gly, Lys, Glu, Phe, Thr, Arg, Gln, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent;
X19 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent;
X20 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent;
X21 is Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent:
X22 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent: and
X23 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent,
wherein X4 and X9 are capable of forming a disulfide bond.

In particular embodiments of peptides of Formula (IIIa), the peptide inhibitor is cyclized via a dissulfide bond between X4 and X9. In certain embodiments, both X4 and X9 are Cys, or both X4 and X9 are Pen, and the intramolecular bond is a disulfide bond.

In certain embodiments, the peptide inhibitor inhibits the binding of an interleukin-23 (IL-23) to an IL-23 receptor.

In certain embodiments, X7 and X11 are both W, or X7 is Trp and X11 is 2-Nal or Trp(5-F); X10 is Phe[4-(2-acetylaminoethoxy)], Phe[4-(2-aminoethoxy)], Phe(CONH₂) or alpha-MeTyr, and X4 and X9 are amino acid residues capable of forming a disulfide bond. In certain embodiments, both X4 and X9 are Pen and the intramolecule bond is a disulfide bond.

In certain embodiments of peptides of Formula (IIIa), X5-X8 are selected from any of the following tetrapeptide sequences: QTWQ (SEQ ID NO:242), NDWQ (SEQ ID NO:243), N(Dab)WQ (SEQ ID NO:244), NT(1-Nal)Q (SEQ ID NO:245), NT(2-Nal)Q (SEQ ID NO:246), NTWE (SEQ ID NO:247), NTWF (SEQ ID NO:248), NTWQ (SEQ ID NO:249), and NT[Trp(5-F)]Q (SEQ ID NO:250).

In certain embodiments, peptides of Formula (IIIa) comprise Asn residues at both X14 and X15. In related embodiments, these peptides further comprise at least two, three or four amino acid residues carboxy to X15. In certain embodiments, the carboxy amino acid residues are the same amino acid residue as each other.

In particular embodiments of a peptide inhibitor of Formula (III), one or more, two or more, three or more, or all four of X0, X1, X2, and X3 are absent. In certain embodiments, X0 is absent and/or X1 is absent. In certain embodiments, X0, X1 and X2 are absent. In certain embodiments, X0, X1, X2 and X3 are absent. In certain embodiments of a peptide inhibitor of Formula III, one or more, two or more, three or more, or all four of X0, X1, X2 and X3 are present, i.e., are not absent. In certain embodiments, X3 is present; in certain embodiments, X3 and X2 are present: in certain embodiments, X3, X2 and X1 are present; and in certain embodiments, X3, X2, X1 and X0 are present, i.e., there is an amino acid present at each position.

In particular embodiments of a peptide inhibitor of Formula III, one or more, two or more, three or more, four or more, five or more, six or more, seven or more, eight or more, or all nine of X15, X16, X17, X18, X19, X20, X21, X22 and X23 are absent. In particular embodiments of a peptide inhibitor of Formula III, one or more, two or more, three or more, or all of X17, X18, X19 and X20 are absent. In certain embodiments, one or more, two or more, or all three of X17, X19 and X20 are absent. In particular embodiments of a peptide inhibitor of Formula III, one or more, two or more, three or more, four or more, five or more, six or more, seven or more, eight or more, or all nine of X15, X16, X17, X18, X19, X20, X21, X22 and X23 are absent. In particular embodiments of a peptide inhibitor of Formula III, one or more, two or more, three or more, four or more, five or more, six or more or all seven of X17, X18, X19, X20, X21, X22 and X23 are present. In certain embodiments, one or more, two or more, or all three of X18, X19 and X20 are present.

In certain embodiments of any of the peptide inhibitors described herein, any of the amino acids of the peptide inhibitor are connected by a linker moiety, e.g., a PEG.

In certain embodiments, the N-terminus of the peptide inhibitor comprises an Ac group.

In certain embodiments, the C-terminus of the peptide inhibitor comprises an NH₂ group.

In particular embodiments of a peptide inhibitor of Formula III, X10 is not Tyr.

In particular embodiments, peptides of Formula (IIIa) comprise any of the following sequences: 
[Pen]-X5-X6-X7-X8-[Pen]-[Phe[4-(2-aminoethoxy)]-[2-Nal]-[4-amino-4-carboxy-
tetrahydropyran] (SEQ ID NO:266);
[Pen]-X5-X6-X7-X8-[Pen]-[Phe[4-(2-aminoethoxy)]-W-[α-MeLeu]-[Lys(Ac)] (SEQ ID NO:267): or
[Pen]-X5-X6-X7-X8-[Pen]-[Phe[4-(2-aminoethoxy)]-[2-Nal]-[α-MeLeu]-[Lys(Ac)] (SEQ ID NO:268),

wherein X5-X8 are defined as described above; and further comprise: (i) at least one, at least two, or at least three amino acid residues N-terminal of the most N-terminal Pen: or (ii) at least three at least four, at least five, at least six, or at least seven amino acid residues C-terminal of the Citermial amino acid residue shown above. In particular embodiments, X5-X8 are selected from: QTWQ (SEQ ID NO:242), NDWQ (SEQ ID NO:243), N(Dab)WQ (SEQ ID NO:244), NT(1-Nal)Q (SEQ ID NO:245), NT(2-Nal)Q (SEQ ID NO:246), NTWE (SEQ ID NO:247), NTWF (SEQ ID NO:248), NTWQ (SEQ ID NO:249), and NT[Trp(5-F)]Q (SEQ ID NO:250).

In particular embodiments of peptides of Formulas (I), (II), IIIa) or (IVa), X7 is (Trp(5-F).

In particular embodiments of a peptide inhibitor of Formula III, the peptide inhibitor has a structure shown in Table 2 or Table 3 or comprises an amino acid sequence set forth in Table 2 or Table 3 (or a pharmaceutically acceptable salt thereof), wherein the two Pen residues may be linked via a disulfide bond.

**Table 2. Illustrative Peptides Containing the Ac-[Pen]-XXWX-[Pen]-XXXX Motif (SEQ ID NO:269) and Analogues**

| **SEQ ID NO.** | **Sequence** |
|---|---|
| 1 | Ac-[(D)Arg]-[Pen]-QTWQ-[Pen]-[Phe[4-(2-aminoethoxy)]-[2-Nal]-[ 4-amino-4- carboxy-tetrahydropyran]-ENN-NH2 |
| 2 | Ac-[Pen]-N-DWQ-[Pen]-[Phe[4-(2-aminoethoxy)]-[2-Nal]-[4-amino-4-carboxy- tetrahydropyran]-ENN-NH2 |
| 3 | Ac-[Pen]-N-[Dab]-WQ-[Pen]-[Phe[4-(2-aminoethoxy)]-[2-Nal]-[ 4-amino-4- carboxy-tetrahydropyran]-ENN-NH2 |
| 4 | Ac-[Pen]-NT-[1-Nal]-Q-[Pen]-[Phe[4-(2-aminoethoxy)]-[2-Nal]-[4-amino-4- carboxy-tetrahydropyran]-[Lys(Ac)]-NN-NH2 |
| 5 | Ac-[Pen]-NT-[2-Nal]-Q-[Pen]-[Phe[4-(2-aminoethoxy)]-[2-Nal]- [4-amino-4- carboxy-tetrahydropyran]-[Lys(Ac)]-NN-NH2 |
| 6 | Ac-[Pen]-NTWE-[Pen]-[Phe[4-(2-aminoethoxy)]-[2-Nal]-[ 4-amino-4-carboxy- tetrahydropyran]-[Lys(Ac)]-NN-NH2 |
| 7 | Ac-[Pen]-NTWF-[Pen]-[Phe[4-(2-aminoethoxy)]-[2-Nal]-[ 4-amino-4-carboxy- tetrahydropyran]-[Lys(Ac)]-NN-NH2 |
| 8 | Ac-[Pen]-NTWQ-[Pen]-[Phe[4-(2-aminoethoxy)]-W-[4-amino-4-carboxy- tetrahydropyran]- [Lys(Ac)]-NN-NH2 |
| 9 | Ac-[Pen]-NT-[Trp(5-F)]-Q-[Pen]-[Phe[4-(2-aminoethoxy)]-W-[α-MeLeu]- [Lys(Ac)]-NN-NH2 |

**Table 3. Illustrative Peptides Containing the Ac-[Pen]-XXWX-[Pen]-XXXX Motif (SEQ ID NO:269) and Analogues**

| **SEQ ID NO.** | **Sequence** |
|---|---|
| 10 | Ac-[Pen]-NTWQ-[Pen]-[Phe[4-(2-aminoethoxy)]-[2-Nal]-[α-MeLeu]-[Lys(Ac)]- NNE-NH2 |
| 11 | Ac-[Pen]-NTWQ-[Pen]-[Phe[4-(2-aminoethoxy)]-[2-Nal]-[α-MeLeu]-[Lys(Ac)]- NNF-NH2 |
| 12 | Ac-[Pen]-NTWQ-[Pen]-[Phe[4-(2-aminoethoxy)]-[2-Nal]-[α-MeLeu]-[Lys(Ac)]- NNK-NH2 |
| 13 | Ac-[Pen]-NTWQ-[Pen]-[Phe[4-(2-aminoethoxy)]-[2-Nal]-[α-MeLeu]-[Lys(Ac)]- NNN-NH2 |
| 14 | Ac-[Pen]-NTWQ-[Pen]-[Phe[4-(2-aminoethoxy)]-[2-Nal]-[α-MeLeu]-[Lys(Ac)]- NNW-NH2 |
| 15 | Ac-[Pen]-NTWQ-[Pen]-[Phe[4-(2-aminoethoxy)]-[2-Nal]-[α-MeLeu]-[Lys(Ac)]- NNG-NH2 |
| 16 | Ac-[Pen]-NTWQ-[Pen]-[Phe[4-(2-aminoethoxy)]-[2-Nal]-[α-MeLeu]-[Lys(Ac)]- NNT-NH2 |
| 17 | Ac-[Pen]-NTWQ-[Pen]-[Phe[4-(2-aminoethoxy)]-[2-Nal]-[α-MeLeu]-[Lys(Ac)]- NNPK-NH2 |
| 18 | Ac-[Pen]-NTWQ-[Pen]-[Phe[4-(2-aminoethoxy)]-[2-Nal]-[α-MeLeu]-[Lys(Ac)]- NNPG-NH2 |
| 19 | Ac-[Pen]-NTWQ-[Pen]-[Phe[4-(2-aminoethoxy)]-[2-Nal]-[α-MeLeu]-[Lys(Ac)]- NNEP-NH2 |
| 20 | Ac-[Pen]-NTWQ-[Pen]-[Phe[4-(2-aminoethoxy)]-[2-Nal]-[α-MeLeu]-[Lys(Ac)]- NNGK-NH2 |
| 21 | Ac-[Pen]-NTWQ-[Pen]-[Phe[4-(2-aminoethoxy)]-[2-Nal]-[α-MeLeu]-[Lys(Ac)]- NNPT-NH2 |
| 22 | Ac-[Pen]-NTWQ-[Pen]-[Phe[4-(2-aminoethoxy)]-[2-Nal]-[α-MeLeu]-[Lys(Ac)]- NNKGF-NH₂ |
| 23 | Ac-[Pen]-NTWQ-[Pen]-[Phe[4-(2-aminoethoxy)]-[2-Nal]-[α-MeLeu]-[Lys(Ac)]- NNGW-NH2 |
| 24 | Ac-[Pen]-NTWQ-[Pen]-[Phe[4-(2-aminoethoxy)]-[2-Nal]-[α-MeLeu]-[Lys(Ac)]- NNGQ-NH₂ |
| 25 | Ac-[Pen]-NTWQ-[Pen]-[Phe[4-(2-aminoethoxy)]-[2-Nal]-[α-MeLeu]-[Lys(Ac)]- NNGGG-NH2 |
| 26 | Ac-[Pen]-NTWQ-[Pen]-[Phe[4-(2-aminoethoxy)]-[2-Nal]-[α-MeLeu]-[Lys(Ac)]- NNKKK-NH2 |
| 27 | Ac-[Pen]-NTWQ-[Pen]-[Phe[4-(2-aminoethoxy)]-[2-Nal]-[α-MeLeu]-[Lys(Ac)]- NNEEE-NH2 |
| 28 | Ac-[Pen]-NTWQ-[Pen]-[Phe[4-(2-aminoethoxy)]-[2-Nal]-[α-MeLeu]-[Lys(Ac)]- NNFFF-NH2 |
| 29 | Ac-[Pen]-NTWQ-[Pen]-[Phe[4-(2-aminoethoxy)]-[2-Nal]-[α-MeLeu]-[Lys(Ac)]- NNTTT-NH2 |
| 30 | Ac-[Pen]-NTWQ-[Pen]-[Phe[4-(2-aminoethoxy)]-[2-Nal]-[α-MeLeu]-[Lys(Ac)]- NNGGGR-NH2 |
| 31 | Ac-[Pen]-NTWQ-[Pen]-[Phe[4-(2-aminoethoxy)]-[2-Nal]-[α-MeLeu]-[Lys(Ac)]- NNGGGF-NH2 |
| 32 | Ac-[Pen]-NTWQ-[Pen]-[Phe[4-(2-aminoethoxy)]-[2-Nal]-[α-MeLeu]-[Lys(Ac)]- NNGGGE-NH2 |
| 33 | Ac-[Pen]-NTWQ-[Pen]-[Phe[4-(2-aminoethoxy)]-[2-Nal]-[α-MeLeu]-[Lys(Ac)]- NNGGGQ-NH2 |
| 34 | Ac-[Pen]-NTWQ-[Pen]-[Phe[4-(2-aminoethoxy)]-[2-Nal]-[α-MeLeu]-[Lys(Ac)]- NNGGGT-NH2 |
| 35 | Ac-[Pen]-NTWQ-[Pen]-[Phe[4-(2-aminoethoxy)]-[2-Nal]-[α-MeLeu]- [Lys(Ac)]- NNGGGGR-NH2 |
| 36 | Ac-[Pen]-NTWQ-[Pen]-[Phe[4-(2-aminoethoxy)]-[2-Nal]-[α-MeLeu]-[Lys(Ac)]- NNGGGGF-NH₂ |
| 37 | Ac-[Pen]-NTWQ-[Pen]-[Phe[4-(2-aminoethoxy)]-[2-Nal]-[α-MeLeu]-[Lys(Ac)]- NNGGGGE-NH2 |
| 38 | Ac-[Pen]-NTWQ-[Pen]-[Phe[4-(2-aminoethoxy)]-[2-Nal]-[α-MeLeu]-[Lys(Ac)]- NNGGGGQ-NH2 |
| 39 | Ac-[Pen]-NTWQ-[Pen]-[Phe[4-(2-aminoethoxy)]-[2-Nal]-[α-MeLeu]-[Lys(Ac)]- NNGGGGT-NH2 |
| 40 | Ac-[Pen]-NTWQ-[Pen]-[Phe[4-(2-aminoethoxy)]-[2-Nal]-[α-MeLeu]-[Lys(Ac)]- NNRRRRR-NH2 |
| 41 | Ac-[Pen]-NTWQ-[Pen]-[Phe[4-(2-aminoethoxy)]-[2-Nal]-[α-MeLeu]-[Lys(Ac)]- NNFFFFF-NH2 |
| 42 | Ac-[Pen]-NTWQ-[Pen]-[Phe[4-(2-aminoethoxy)]-[2-Nal]-[α-MeLeu]-[Lys(Ac)]- NNEEEEE-NH2 |
| 43 | Ac-[Pen]-NTWQ-[Pen]-[Phe[4-(2-aminoethoxy)]-[2-Nal]-[α-MeLeu]-[Lys(Ac)]- NNQQQQ-NH2 |
| 44 | Ac-[Pen]-NTWQ-[Pen]-[Phe[4-(2-aminoethoxy)]-[2-Nal]-[α-MeLeu]-[Lys(Ac)]- NNTTTTT-NH₂ |
| 45 | Ac-GGG-[Pen]-NTWQ-[Pen]-[Phe[4-(2-aminoethoxy)]-[2-Nal]-[α-MeLeu]- [Lys(Ac)]-NN-NH₂ |
| 46 | Ac-RRR-[Pen]-NTWQ-[Pen]-[Phe[4-(2-aminoethoxy)]-[2-Nal]-[α-MeLeu]- [Lys(Ac)]-NN-NH₂ |
| 47 | Ac-FFF-[Pen]-NTWQ-[Pen]-[Phe[4-(2-aminoethoxy)]-[2-Nal]-[α-MeLeu]- [Lys(Ac)]-NN-NH₂ |
| 48 | Ac-EEE-[Pcn]-NTWQ-[Pcn]-[Phc[4-(2-aminocthoxy)]-[2-Nal]-[α-McLcu]- [Lys(Ac)]-NN-NH2 |
| 49 | Ac-QQQ-[Pen]-NTWQ-[Pen]-[Phe[4-(2-aminoethoxy)]-[2-Nal]-[α-MeLeu]- [Lys(Ac)]-NN-NH₂ |
| 50 | Ac-TTT-[Pen]-NTWQ-[Pen]-[Phe[4-(2-aminoethoxy)]-[2-Nal]-[α-MeLeu]- [Lys(Ac)]-NN-NH2 |
| 51 | Ac-RG-[Pen]-NTWQ-[Pen]-[Phe[4-(2-aminoethoxy)]-[2-Nal]-[α-MeLeu]- [Lys(Ac)]-NN-NH2 |
| 52 | Ac-FG-[Pen]-NTWQ-[Pen]-[Phe[4-(2-aminoethoxy)]-[2-Nal]-[α-MeLeu]- [Lys(Ac)]-NN-NH2 |
| 53 | Ac-EG-[Pen]-NTWQ-[Pen]-[Phe[4-(2-aminoethoxy)]-[2-Nal]-[α-MeLeu]- [Lys(Ac)]-NN-NH2 |
| 54 | Ac-QG-[Pen]-NTWQ-[Pen]-[Phe[4-(2-aminoethoxy)]-[2-Nal]-[α-MeLeu]- [Lys(Ac)]-NN-NH2 |
| 55 | Ac-TG-[Pen]-NTWQ-[Pen]-[Phe[4-(2-aminoethoxy)]-[2-Nal]-[α-MeLeu]- [Lys(Ac)]-NN-NH2 |
| 56 | Ac-[Pen]-NTWQ-[Pen]-[Phe[4-(2-aminoethoxy)]-[2-Nal]-[α-MeLeu]- [Lys(Palm)]-NN-NH₂ |
| 57 | Ac-[Pen]-NTWQ-[Pen]-[Phe[4-(2-aminoethoxy)]-[2-Nal]-[α-MeLeu]- [Lys(isoGlu-Palm)]-NN-NH2 |
| 58 | Ac-[Pen]-NTWQ-[Pen]-[Phe[4-(2-aminoethoxy)]-[2-Nal]-[α-MeLeu]- [Lys(PEG11-Palm)]-NN-NH2 |
| 59 | Ac-[Pen]-NTWQ-[Pen]-[Phe[4-(2-aminoethoxy)]-[2-Nal]-[α-MeLeu]-[Lys(Ahx- Palm)]-NN-NH₂ |
| 60 | Ac-[Pen]-NTWQ-[Pen]-[Phe[4-(2-aminoethoxy)]-[2-Nal]-[α-MeLeu]- [Lys(isoGlu-Ahx-Palm)]-NN-NH₂ |
| 61 | [Palm]-[Pen]-NTWQ-[Pen]-[Phe[4-(2-aminoethoxy)]-[2-Nal]-[α-MeLeu]- [Lys(Ac)]-NN-NH₂ |
| 62 | [Palm-isoGlu]-[Pen]-NTWQ-[Pen]-[Phe[4-(2-aminoethoxy)]-[2-Nal]-[α-MeLeu]- [Lys(Ac)]-NN-NH₂ |
| 63 | [Palm-PEG11]-[Pen]-NTWQ-[Pen]-[Phe[4-(2-aminoethoxy)]-[2-Nal]-[α- MeLeu]-[Lys(Ac)]-NN-NH2 |
| 64 | [Palm-Ahx]-[Pen]-NTWQ-[Pen]-[Phe[4-(2-aminoethoxy)]-[2-Nal]-[α-MeLeu]- [Lys(Ac)]-NN-NH2 |
| 65 | [Palm-Ahx-isoGlu]-[Pen]-NTWQ-[Pen]-[Phe[4-(2-aminoethoxy)]-[2-Nal]-[α- MeLeu]-[Lys(Ac)]-NN-NH2 |
| 66 | Ac-[Pen]-NTWQ-[Pen]-[Phe[4-(2-aminoethoxy)]-[2-Nal]-[α-MeLeu]-[Lys(Ac)]- NN-Lys[Palm]-NH2 |
| 67 | Ac-[Pen]-NTWQ-[Pen]-[Phe[4-(2-aminoethoxy)]-[2-Nal]-[α-MeLeu]-[Lys(Ac)]- NN-Lys[isoGlu-Palm]-NH₂ |
| 68 | Ac-[Pen]-NTWQ-[Pen]-[Phe[4-(2-aminoethoxy)]-[2-Nal]-[α-MeLeu]-[Lys(Ac)]- NN-Lys[PEG11-Palm]-NH2 |
| 69 | Ac-[Pen]-NTWQ-[Pen]-[Phe[4-(2-aminoethoxy)]-[2-Nal]-[α-MeLeu]-[Lys(Ac)]- NN-Lys[Ahx-Palm]-NH2 |
| 70 | Ac-[Pen]-NTWQ-[Pen]-[Phe[4-(2-aminoethoxy)]-[2-Nal]-[α-MeLeu]-[Lys(Ac)]- NN-Lys[isoGlu-Ahx-Palm]-NH2 |

### Illustrative Peptide Inhibitors Comprising Thioether Bonds

In certain embodiments, the present invention includes a peptide inhibitor of an interleukin-23 receptor, wherein the peptide inhibitor has the structure of Formula IV:

R¹-X-R² (IV)

or a pharmaceutically acceptable salt or solvate thereof,
wherein R¹ is a bond, hydrogen, a C1-C6 alkyl, a C6-C12 aryl, a C6-C12 aryl, a C1-C6 alkyl, a C 1-C20 alkanoyl, an alkylsulphonate, an acid, γ-Glu or pGlu, appended to the N-terminus, and including PEGylated versions (e.g., 200 Da to 60,000 Da), alone or as a spacer of any of the foregoing;
R² is a bond, OH or NH₂; and
X is an amino acid sequence of 8 to 20 amino acids or 8 to 35 amino acids,

In particular embodiments of peptide inhibitors of Formula IV, X comprises or consists of the sequence of Formula IVa: 
X0-X1-X2-X3-X4-X5-X6-X7-X8-X9-X10-X11-X12-X13-X14-X15-X16-X17-X18-X19-
X20-X21-X22-X23 (IVa) (SEQ ID NO:270)
 wherein
X0 is Gly, Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, or absent;
X1 is Gly, Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, or absent:
X2 is (D)Asp, Arg, (D)Arg, Phe, (D)Phe, 2-Nal, Thr, Leu, (D)Gln, (D)Asn, IsoGlu, Gly, Arg, Phe, Glu, Gln, Thr, (D)Glu, (D)Thr, (D)Leu, alpha-MeArg, alpha-MePhe, alpha-MeLeu, alpha-MeLys, alpha-MeAsn, alpha-MeTyr, or absent;
X3 is (D)Arg, (D)Tyr, Gly, alpha-MeArg, alpha-MePhe, alpha-MeLeu, alpha-MeLys, alpha-MeAsn, alpha-MeTyr, or absent;
X4 is Abu, Cys, (D)Cys), alpha-MeCys, (D)Abu, (D)Pen, or Pen;
X5 is Cit, Glu, Gly, Lys, Asn, Pro, alpha-MeGln, alpha-MeLys, alpha-MeLeu, alpha-MeAsn, Lys(Ac), alpha-MeLys(Ac), Dab(Ac), Dap(Ac), homo-Lys(Ac), or Gln;
X6 is Thr, Aib, Asp, Dab, Gly, Pro, Ser, alpha-MeGln, alpha-MeLys, alpha-MeLeu, alpha-MeAsn, alpha-MeThr, alpha-MeSer, or Val;
X7 is Trp, Trp(5-F), 1-Nal, 2-Nal, Phe(2-Me), Phe(3-Me), Phe(4-Me), Trp(7-Aza), or Phe(3,4-dimethoxy):
X8 is Gln, alpha-Me-Lys, alpha-MeLeu, alpha-MeLys(Ac), beta-homoGln, Cit, Glu, Phe, Asn, Thr, Val, Aib, alpha-MeGln, alpha-MeAsn, Lys(Ac), alpha-MeLys(Ac), Dab(Ac), Dap(Ac), homo-Lys(Ac), or Trp;
X9 is Cys, (D)Cys), alpha-MeCys, (D)Abu, (D)Pen, Pen or Abu;
X10 is Phe, Phe[4-(2-aminoethoxy)], Phe[4-(2-acetylaminoethoxy)], alpha-MeTyr, or Phe(4-CONH₂);
X11 is 2-Nal, Trp, Trp(5-F), Trp(7-Aza), Phe(2-Me), Phe(3-Me), Phe(4-Me), Phe(3,4-dimethoxy), or 1-Nal;
X12 is 4-amino-4-carboxy-tetrahydropyran (THP), alpha-MeLys, alpha-MeLeu, alpha-MeArg, alpha-MePhe, alpha-MeLeu, alpha-MeLys, alpha-MeAsn, alpha-MeTyr, or Aib;
X13 is Glu, Cit, Gln, alpha-MeArg, alpha-MeGlu, alpha-MeLeu, alpha-MeLys, alpha-MeAsn, Lys(Ac), alpha-MeLys(Ac), Dab(Ac), Dap(Ac), homo-Lys(Ac), or Lys:
X14 is Asn, 2-Nap, Aib, Arg, Cit, Asp, Phe, Gly, Lys, Leu, Asn, n-Leu, Gln, Ser, Tic, Trp, alpha-MeGln, alpha-MeAsn, alpha-MeLys(Ac), Dab(Ac), Dap(Ac), homo-Lys(Ac), or Lys(Ac):
X15 is Asn, Aib, beta-Ala, Cit, Gln, Asp, alpha-MeGln, alpha-MeAsn, Lys(Ac), alpha-MeLys(Ac), Dab(Ac), Dap(Ac), homo-Lys(Ac), or absent;
X16 is Glu, Phe, Lys, Asn, Trp, Gly, Thr, Pro, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent:
X17 is Lys, Gly, Pro, The, Phe, Trp, Gln, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent:
X18 is Gly, Lys, Glu, Phe, Thr, Arg, Gln, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent:
X19 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent:
X20 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent:
X21 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent:
X22 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent; and
X23 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent,
wherein X4 and X9 are apable of forming a thioether bond.

In particular embodiments of peptides of Formula (IVa), the peptide inhibitor is cyclized via a thioether bond between X4 and X9. In one embodiment of peptides of Formulas (IVa), X4 is Abu and X9 is Cys, and the intramolecular bond is a thioether bond. In certain embodiments, the peptide inhibitor inhibits the binding of an interleukin-23 (IL-23) to an IL-23 receptor.

In certain embodiments, X7 and X11 are both W, or X7 is Trp and X11 is 2-Nal or Trp(5-F); X10 is Phe[4-(2-acetylaminoethoxy)], Phe[4-(2-aminoethoxy)], Phe(CONH₂) or alpha-MeTyr, and X4 and X9 are amino acid residues capable of forming a thioether bond. In certain embodiments, X4 is Abu, X9 is Cys, and the intramolecular bond is a thioether bond.

In certain embodiments of peptides of Formula (IVa), X5-X8 are selected from any of the following tetrapeptide sequences: QTWQ (SEQ ID NO:242), NDWQ (SEQ ID NO:243), N(Dab)WQ (SEQ ID NO:244), NT(1-Nal)Q (SEQ ID NO:245), NT(2-Nal)Q (SEQ ID NO:246), NTWE (SEQ ID NO:247), NTWF (SEQ ID NO:248), NTWQ (SEQ ID NO:249), and NT[Trp(5-F)]Q (SEQ ID NO:250).

In certain embodiments, peptides of Formula (IVa) comprise Asn residues at both X14 and X15. In related embodiments, these peptides further comprise at least two, three or four amino acid residues carboxy to X15. In certain embodiments, the carboxy amino acid residues are the same amino acid residue as each other.

In particular embodiments, the peptide inhibitor of Formula IV is cyclized. In certain embodiments, the peptide inhibitor is cyclized via a thioether bond between X4 and X9. In certain embodiments, the peptide inhibitor of Formula IV is linear or not cyclized.

In particular embodiments of a peptide inhibitor of Formula IV, one or more, two or more, three or more, or all four of X0, X1, X2, and X3 are absent. In certain embodiments, X0 is absent and/or X1 is absent. In certain embodiments, Xo, X1 and X2 are absent. In certain embodiments, X0, X1, X2 and X3 are absent. In certain embodiments of a peptide inhibitor of Formula IV, one or more, two or more, three or more, or all four of X0, X1, X2 and X3 are present, i.e., are not absent. In certain embodiments, X3 is present; in certain embodiments, X3 and X2 are present; in certain embodiments, X3, X2 and X1 are present; and in certain embodiments, X3, X2, X1 and X0 are present, i.e., there is an amino acid present at each position.

In particular embodiments of a peptide inhibitor of Formula IV, one or more, two or more, three or more, four or more, five or more, six or more, seven or more, eight or more, or all nine of X15, X16, X17, X18, X19, X20, X21, X22 and X23 are absent. In particular embodiments of a peptide inhibitor of Formula IV, one or more, two or more, three or more, or all of X17, X18, X19 and X20 are absent. In certain embodiments, one or more, two or more, or all three of X17, X19 and X20 are absent. In particular embodiments of a peptide inhibitor of Formula IV, one or more, two or more, three or more, four or more, five or more, six or more, seven or more, eight or more, or all nine of X15, X16, X17, X18, X19, X20, X21, X22 and X23 are absent. In particular embodiments of a peptide inhibitor of Formula IV, one or more, two or more, three or more, four or more, five or more, six or more or all seven of X17, X18, X19, X20, X21, X22 and X23 are present. In certain embodiments, one or more, two or more, or all three of X18, X19 and X20 are present.

In particular embodiments of a peptide inhibitor of Formula IV, one of X4 or X9 is Abu, and the other of X4 or X9 is not Abu. In certain embodiments, X4 is Abu and X9 is Cys.

In certain embodiments of any of the peptide inhibitors described herein, any of the amino acids of the peptide inhibitor are connected by a linker moiety, e.g., a PEG.

In certain embodiments, the N-terminus of the peptide inhibitor comprises an Ac group.

In certain embodiments, the C-terminus of the peptide inhibitor comprises an NH₂ group.

In particular embodiments of a peptide inhibitor of Formula IV, X10 is not Tyr.

In particular embodiments, peptides of Formula (IVa) comprise any of the following sequences: 
[Abu]-X5-X6-X7-X8-[Cys]-[Phe[4-(2-aminoethoxy)]-[2-Nal]-[4-amino-4-carboxy-
tetrahydropyran] (SEQ ID NO:271);
[Abu]-X5-X6-X7-X8-[Cys]-[Phe[4-(2-aminoethoxy)]-W-[α-MeLeu]-[Lys(Ac)] (SEQ ID NO:272); or
[Abu]-X5-X6-X7-X8-[Cys]-[Phe[4-(2-aminoethoxy)]-[2-Nal]-[α-MeLeu]-[Lys(Ac)] (SEQ ID NO:273),
wherein X5-X8 are defined as described above; and further comprise: (i) at least one, at least two, or at least three amino acid residues N-terminal of the Abu; or (ii) at least three at least four, at least five, at least six, or at least seven amino acid residues C-terminal of the C-terminal amino acid residue shown above. In particular embodiments, X5-X8 are selected from: QTWQ (SEQ ID NO:242), QTWE (SEQ ID NO:251), ETWQ (SEQ ID NO:252), ETWE (SEQ ID NO:253), QTW-(alpha-MeLeu) (SEQ ID NO:254), QTW-(alpha-MeLys) (SEQ ID NO:255), QTW-(alpha-MeLys(Ac)) (SEQ ID NO:256), QTW-((D)Gln) (SEQ ID NO:257), QTW-(B-homoGln) (SEQ ID NO:258), QTWF (SEQ ID NO:259), QTWW (SEQ ID NO:260), QTWAib (SEQ ID NO:261), QTWT (SEQ ID NO:262), QTWV (SEQ ID NO:263), or QT-(Trp(5-F))-Q (SEQ ID NO:264).

In particular embodiments of peptides of Formulas (I), (II), IIIa) or (IVa), X7 is (Trp(5-F).

In certain embodiments, the present invention includes a peptide comprising or consisting of an amino acid sequence shown in any of the Tables 4 or 5 or a peptide inhibitor comprising or consisting of a structure shown in any of the Tables 4 or 5 (or a pharmaceutically acceptable salt thereof). In particular embodiments, the peptide does not include the conjugated moieties but does include the Abu residue. In particular embodiments, the peptide or inhibitor comprises a thioether bond between the two Abu and Cys residues, or between the two outermost amino acids within the brackets folloing the term "cyclo", which indicated the presence of a cyclic structure. In particular embodiments, the inhibitor is an acetate salt. The peptide sequence of illustrative inhibitors is shown in Tables 4 and 5 from N-term to C-term, with conjugated moieties, and N-terminal Ac and/or C-terminal NH₂ groups indicated. The cyclic structure is indicated by "cyclo" as illustrated in Table 5, indicating the presence of a thioether bond between the bracketed Abu at X4 and Cys at X9. An illustrative example of the structure of a peptide inhibitor is shown below in Table 4 and Table 5.

**Table 4. Illustrative Peptide Inhibitors (Thioethers)**

| 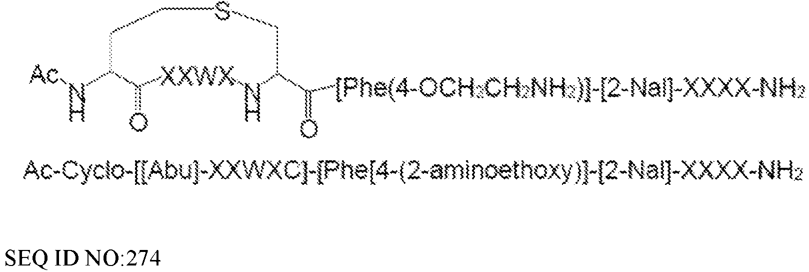 | |
|---|---|
| **SEQ ID No.** | **Sequence** |
| 71 | Alexa488-[PEG4]-[(D)Arg]-cyclo[[Abu]-QTWQC]-[Phe[4-(2-aminoethoxy)]-[2-Nal]-[4- amino-4-carboxy-tetrahydropyran]-ENN-NH₂ |
| 72 | Alexa647-[PEG4]-[(D)Arg]-cyclo[[Abu]-QTWQC]-[Phe[4-(2-aminoethoxy)]-[2-Nal]-[4- amino-4-carboxy-tetrahydropyran]-ENN-NH2 |
| 73 | Ac-[(D)Arg]-cyclo[[Abu]-QTWQC]-[Phe[4-(2-aminoethoxy)]-[2-Nal]-[4-amino-4-carboxy- tetrahydropyran]-END-NH2 |
| 74 | Ac-[(D)Arg]-cyclo[[Abu]-QTWQC]-[Phe[4-(2-aminoethoxy)]-[2-Nal]-[4-amino-4-carboxy- tetrahydropyran]-EDN-NH2 |
| 75 | Ac-[(D)Arg]-cyclo[[Abu]-QTWEC]-[Phe[4-(2-aminoethoxy)]-[2-Nal]-[4-amino-4-carboxy- tetrahydropyran]-ENN-NH2 |
| 76 | Ac-[(D)Arg]-cyclo[[Abu]-ETWQC]-[Phe[4-(2-aminoethoxy)]-[2-Nal]-[4-amino-4-carboxy- tetrahydropyran]-ENN-NH2 |
| 77 | Ac-[(D)Arg]-cyclo[[Abu]-QTWQC]-[Phe[4-(2-aminoethoxy)]-[2-Nal]-[4-amino-4-carboxy- tetrahydropyran]-EDD-NH2 |
| 78 | Ac-[(D)Arg]-cyclo[[Abu]-QTWEC]-[Phe[4-(2-aminoethoxy)]-[2-Nal]-[4-amino-4-carboxy- tetrahydropyran]-END-NH2 |
| 79 | Ac-[(D)Arg]-cyclo[[Abu]-ETWQC]-[Phe[4-(2-aminoethoxy)]-[2-Nal]-[4-amino-4-carboxy- tetrahydropyran]-END-NH2 |
| 80 | Ac-[(D)Arg]-cyclo[[Abu]-QTWEC]-[Phe[4-(aminoethoxy)]-[2-Nal]-[4-amino-4-carboxy- tetrahydropyran]-EDN-NH2 |
| 81 | Ac-[(D)Arg]-cyclo[[Abu]-ETWQC]-[Phe[4-(2-aminoethoxy)]-[2-Nal]-[4-amino-4-carboxy- tetrahydropyran]-EDN-NH₂ |
| 82 | Ac-[(D)Arg]-cyclo[[Abu]-ETWEC]-[Phe[4-(2-aminoethoxy)]-[2-Nal]-[4-amino-4-carboxy- tetrahydropyran]-ENN-NH2 |
| 83 | Ac-[(D)Arg]-cyclo[[Abu]-QTWQC]-[Phe[4-(2-aminoethoxy)]-[2-Nal]-[4-amino-4-carboxy- tetrahydropyran]-ENN-OH |
| 84 | Ac-[(D)Arg]-cyclo[[Abu]-QTWQC]-[Phe[4-(2-aminoethoxy)]-[2-Nal]-[4-amino-4-carboxy- tetrahydropyran]-END-OH |
| 85 | Ac-[(D)Arg]-cyclo[[Abu]-QTWQC]-[Phe[4-(2-aminoethoxy)]-[2-Nal]-[4-amino-4-carboxy- tetrahydropyran]-EDN-OH |
| 86 | Ac-[(D)Arg]-cyclo[[Abu]-QTWEC]-[Phe[4-(2-aminoethoxy)]-[2-Nal]-[4-amino-4-carboxy- tetrahydropyran]-ENN-OH |
| 87 | [Ac-[(D)Arg]-cyclo[[Abu]-ETWQC]-[Phe[4-(2-aminoethoxy)]-[2-Nal]-[4-amino-4-carboxy- tetrahydropyran]-ENN-OH |
| 88 | [NH₂-PEG4]-[(D)Arg]-cyclo[[Abu]-QTWQC]-[Phe[4-(2-aminoethoxy)]-[2-Nal]-[4-amino-4- carboxy-tetrahydropyran]-ENN-NH₂ |
| 89 | [Biotin]-[PEG4]-[(D)Arg]-cyclo[[Abu]-QTWQC]-[Phe[4-(2-aminoethoxy)]-[2-Nal]-[4- amino-4-carboxy-tetrahydropyran]-ENN-NH2 |
| 90 | [NH₂-PEG4]-[(D)Arg]-cyclo[[Abu]-QTWQC]-[Phe[4-(2-aminoethoxy)-(PEG4-NH₂)]-[2- Nal]-[4-amino-4-carboxy-tetrahydropyran]-ENN-NH₂ |
| 91 | [NH₂-PEG4]-[(D)Arg]-cyclo[[Abu]-QTWQC]-[Phe[4-(2-aminoethoxy)-(PEG4)-(Biotin)]-[2- Nal]-[4-amino-4-carboxy-tetrahydropyran]-ENN-NH₂ |
| 92 | Ac-[(D)Arg]-cyclo[[Abu]-QTWQ-[Pen]]-[Phe[4-(2-aminoethoxy)]-[2-Nal]-[4-amino-4- carboxy-tetrahydropyran]-ENN-NH₂ |
| 93 | Ac-[(D)Arg]-cyclo[[Abu]-QTW-[α-MeLeu]-C]-[Phe[4-(2-aminoethoxy)]-[2-Nal]-[4-amino- 4-carboxy-tetrahydropyran]-ENN-NH₂ |
| 94 | Ac-[(D)Arg]-cyclo[[Abu]-QTW-[α-MeLys]-C]-[Phe[4-(2-aminoethoxy)]-[2-Nal]-[4-amino- 4-carboxy-tetrahydropyran]-ENN-NH₂ |
| 95 | Ac-[(D)Arg]-cyclo[[Abu]-QTW-[α-MeLys(Ac)]-C]-[Phe[4-(2-aminoethoxy)]-[2-Nal]-[4- amino-4-carboxy-tetrahydropyran]-ENN-NH₂ |
| 96 | Ac-[(D)Arg]-cyclo[[Abu]-QTW-[(D)Gln]-C]-[Phe[4-(2-aminoethoxy)]-[2-Nal]-[4-amino-4- carboxy-tetrahydropyran]-ENN-NH2 |
| 97 | Ac-[(D)Arg]-cyclo[[Abu]-QTW-[β–homoGln]-C]-[Phe[4-(2-aminoethoxy)]-[2-Nal]-[4- amino-4-carboxy-tetrahydropyran]-ENN-NH₂ |
| 98 | Ac-[(D)Arg]-cyclo[[Abu]-QTW-[α-MeLys(Ac)]-C]-[Phe[4-(2-acetylaminoethoxy]-[2-Nal]- [4-amino-4-carboxy-tetrahydropyran]-ENN-NH₂ |
| 99 | Ac-[(D)Arg]-cyclo[[Abu]-QTWFC]-[Phe[4-(2-aminoethoxy)]-[2-Nal]-[4-amino-4-carboxy- tetrahydropyran]-ENN-NH2 |
| 100 | Ac-[(D)Arg]-cyclo[[Abu]-QTWWC]-[Phe[4-(2-aminoethoxy)]-[2-Nal]-[4-amino-4-carboxy- tetrahydropyran]-ENN-NH2 |
| 101 | Ac-[(D)Arg]-cyclo[[Abu]-QTW-[Aib]-C]-[Phe[4-(2-aminoethoxy)]-[2-Nal]-[4-amino-4- carboxy-tetrahydropyran]-ENN-NH2 |
| 102 | Ac-[(D)Arg]-cyclo[[Abu]-QTWTC]-[Phe[4-(2-aminoethoxy)]-[2-Nal]-[4-amino-4-carboxy- tetrahydropyran]-ENN-NH₂ |
| 103 | Ac-[(D)Arg]-cyclo[[Abu]-QTWVC]-[Phe[4-(2-aminoethoxy)]-[2-Nal]-[4-amino-4-carboxy- tetrahydropyran]-ENN-NH2 |
| 104 | Ac-[(D)Arg]-cyclo[[Abu]-QT-[Trp(5-F)]-QC]-[Phe[4-(2-aminoethoxy)]-[2-Nal]-[4-amino-4- carboxy-tetrahydropyran]-ENN-NH2 |
| 105 | Ac-[(D)Arg]-cyclo[[Abu]-QT-[Trp(5-F)]-QC]-[Phe[4-(2-aminoethoxy)]-[2-Nal]-[α-MeLeu]- ENN-NH2 |
| 106 | Ac-cyclo[[Abu]-QT-[Trp(5-F)]-QC]-[Phe[4-(2-aminoethoxy)]-[2-Nal]-[α-MeLeu]-ENN-NH₂ |
| 107 | Ac-[(D)Arg]-cyclo[[Abu]-QTWQC]-[Phe[4-(2-aminoethoxy)]-[2-Nal]-[α-MeLeu]-ENN-NH₂ |
| 108 | Ac-[(D)Arg]-cyclo[[Abu]-QTW-[2-Nal]-C]-[Phe[4-(2-aminoethoxy)]-[2-Nal]-[ 4-amino-4- carboxy-tetrahydropyran]-ENN-NH2 |
| 109 | Ac-[(D)Arg]-cyclo[[Abu]-QTW-[1-Nal]-C]-[Phe[4-(2-aminoethoxy)]-[2-Nal]-[ 4-amino-4- carboxy-tetrahydropyran]-ENN-NH₂ |
| 110 | Ac-[(D)Arg]-cyclo[[Abu]-QTW-[Trp(5-F)]-C]-[Phe[4-(2-aminoethoxy)]-[2-Nal]-[ 4-amino- 4-carboxy-tetrahydropyran]-ENN-NH₂ |
| 308 | Ac-[(D)Arg]-cyclo[[Abu]-QT-[2-Nal]-QC]-[Phe[4-(2-aminoethoxy)]-[2-Nal]-[ 4-amino-4- carboxy-tetrahydropyran]-ENN-NH2 |
| 309 | Ac-[(D)Arg]-cyclo[[Abu]-QT-[1-Nal]-QC]-[Phe[4-(2-aminoethoxy)]-[2-Nal]-[ 4-amino-4- carboxy-tetrahydropyran]-ENN-NH2 |
| 310 | Ac-[(D)Arg]-cyclo[[Abu]-QT-[Trp(5-F)]-QC]-[Phe[4-(2-aminoethoxy)]-[2-Nal]-[ 4-amino- 4-carboxy-tetrahydropyran]-ENN-NH2 |

**Table 5. Illustrative Peptide Inhibitors (Thioethers)**

| 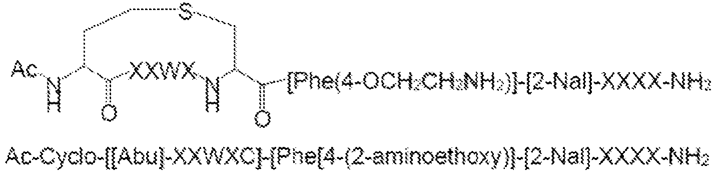 | |
|---|---|
| **SEQ. ID NO.** | **Sequence** |
| 111 | Ac-cyclo[[Abu]-QTWQC]-[Phe[4-(2-aminoethoxy)]-[2-Nal]-[4-amino-4-carboxy- tetrahydropyran]-[Lys(Ac)]-NNE-NH2 |
| 112 | Ac-cyclo[[Abu]-QTWQC]-[Phe[4-(2-aminoethoxy)]-[2-Nal]-[4-amino-4-carboxy- tetrahydropyran]-[Lys(Ac)]-NNF-NH2 |
| 113 | Ac-cyclo[[Abu]-QTWQC]-[Phe[4-(2-aminoethoxy)]-[2-Nal]-[4-amino-4-carboxy- tetrahydropyran]-[Lys(Ac)]-NNK-NH2 |
| 114 | Ac-cyclo[[Abu]-QTWQC]-[Phe[4-(2-aminoethoxy)]-[2-Nal]-[4-amino-4-carboxy- tetrahydropyran]-[Lys(Ac)]-NNN-NH2 |
| 115 | Ac-cyclo[[Abu]-QTWQC]-[Phe[4-(2-aminoethoxy)]-[2-Nal]-[4-amino-4-carboxy- tetrahydropyran]-[Lys(Ac)]-NNW-NH2 |
| 116 | Ac-cyclo[[Abu]-QTWQC]-[Phe[4-(2-aminoethoxy)]-[2-Nal]-[4-amino-4-carboxy- tetrahydropyran]-[Lys(Ac)]-NNT-NH2 |
| 117 | Ac-cyclo[[Abu]-QTWQC]-[Phe[4-(2-aminoethoxy)]-[2-Nal]-[4-amino-4-carboxy- tetrahydropyran]-[Lys(Ac)]-NNG-NH2 |
| 118 | Ac-cyclo[[Abu]-QTWQC]-[Phe[4-(2-aminoethoxy)]-[2-Nal]-[4-amino-4-carboxy- tetrahydropyran]-[Lys(Ac)]-NNPK-NH2 |
| 119 | Ac-cyclo[[Abu]-QTWQC]-[Phe[4-(2-aminoethoxy)]-[2-Nal]-[4-amino-4-carboxy- tetrahydropyran]-[Lys(Ac)]-NNPG-NH2 |
| 120 | Ac-cyclo[[Abu]-QTWQC]-[Phe[4-(2-aminoethoxy)]-[2-Nal]-[4-amino-4-carboxy- tetrahydropyran]-[Lys(Ac)]-NNEP-NH2 |
| 121 | Ac-cyclo[[Abu]-QTWQC]-[Phe[4-(2-aminoethoxy)]-[2-Nal]-[4-amino-4-carboxy- tetrahydropyran]-[Lys(Ac)]-NNGK-NH2 |
| 122 | Ac-cyclo[[Abu]-QTWQC]-[Phe[4-(2-aminoethoxy)]-[2-Nal]-[4-amino-4-carboxy- tetrahydropyran]-[Lys(Ac)]-NNPT-NH2 |
| 123 | Ac-cyclo[[Abu]-QTWQC]-[Phe[4-(2-aminoethoxy)]-[2-Nal]-[4-amino-4-carboxy- tetrahydropyran]-[Lys(Ac)]-NNGF-NH2 |
| 124 | Ac-cyclo[[Abu]-QTWQC]-[Phe[4-(2-aminoethoxy)]-[2-Nal]-[4-amino-4-carboxy- tetrahydropyran]-[Lys(Ac)]-NNGW-NH2 |
| 125 | Ac-cyclo[[Abu]-QTWQC]-[Phe[4-(2-aminoethoxy)]-[2-Nal]-[4-amino-4-carboxy- tetrahydropyran]-[Lys(Ac)]-NNGQ-NH₂ |
| 126 | Ac-cyclo[[Abu]-QTWQC]-[Phe[4-(2-aminoethoxy)]-[2-Nal]-[4-amino-4-carboxy- tetrahydropyran]-[Lys(Ac)]-NNGGG-NH2 |
| 127 | Ac-cyclo[[Abu]-QTWQC]-[Phe[4-(2-aminoethoxy)]-[2-Nal]-[4-amino-4-carboxy- tetrahydropyran]-[Lys(Ac)]-NNKKK-NH2 |
| 128 | Ac-cyclo[[Abu]-QTWQC]-[Phe[4-(2-aminoethoxy)]-[2-Nal]-[4-amino-4-carboxy- tetrahydropyran]-[Lys(Ac)]-NNEEE-NH2 |
| 129 | Ac-cyclo[[Abu]-QTWQC]-[Phe[4-(2-aminoethoxy)]-[2-Nal]-[4-amino-4-carboxy- tetrahydropyran]-[Lys(Ac)]-NNFFF-NH2 |
| 130 | Ac-cyclo[[Abu]-QTWQC]-[Phe[4-(2-aminoethoxy)]-[2-Nal]-[4-amino-4-carboxy- tetrahydropyran]-[Lys(Ac)]-NNTTT-NH2 |
| 131 | Ac-[(D)Arg]-cyclo[[Abu]-QT-[AzaTrp]-QC]-[Phe[4-(2-aminoethoxy)]-[2-Nal]-[α- MeLeu]-ENN-NH2 |
| 132 | Ac-[(D)Arg]-cyclo[[Abu]-QT-[Trp(5-CN)]-QC]-[Phe[4-(2-aminoethoxy)]-[2-Nal]-[α- MeLeu]-ENN-NH2 |
| 133 | Ac-[(D)Arg]-cyclo[[Abu]-QT-[Trp(5-Cl)]-QC]-[Phe[4-(2-aminoethoxy)]-[2-Nal]-[α- MeLeu]-ENN-NH2 |
| 134 | Ac-[(D)Arg]-cyclo[[Abu]-QT-[Trp(6-OH)]-QC]-[Phe[4-(2-aminoethoxy)]-[2-Nal]-[α- MeLeu]-ENN-NH₂ |

In certain embodiments, the peptide or the peptide dimer is selected from the peptides listed in Table 6.

**Table 6. Additional Illustrative Peptide Inhibitors**

| **SEQ. ID No.** | **Sequence** |
|---|---|
| 135 | Ac-cyclo[[(D)Abu]-QTWQC]-[Phe[4-(2-aminoethoxy)]-[2-Nal]-[α-MeLeu]-ENN-NH2 |
| 136 | Ac-cyclo[[(D)Abu]-NTWQ-[Pen]]-[Phe[4-(2-aminoethoxy)]-[2-Nal]-[α-MeLeu]-[Lys(Ac)]- NN-NH2 |
| 137 | Ac-cyclo[Pen]-NTWQ-[(D)Abu]]-[Phe[4-(2-aminoethoxy)]-[2-Nal]-[α-MeLeu]-[Lys(Ac)]- NN-NH2 |
| 138 | Ac-[(D)Arg]-cyclo[[Abu]-QTWQC]-[Phe[4-(2-aminoethoxy)]-[2-Nal]-[α-MeLeu]-ENN- NH2 |
| 139 | Ac-[(D)Arg]-cyclo[[Abu]-QTW-[2-Nal]-C]-[Phe[4-(2-aminoethoxy)]-[2-Nal]-[4-amino-4- carboxy-tetrahydropyran]-ENN-NH2 |
| 140 | Ac-[(D)Arg]-cyclo[[Abu]-QTW-[1-Nal]-C]-[Phe[4-(2-aminoethoxy)]-[2-Nal]-[4-amino-4- carboxy-tetrahydropyran]-ENN-NH2 |
| 141 | Ac-[(D)Arg]-cyclo[[Abu]-QTW-[Trp(5-F)]-C]-[Phe[4-(2-aminoethoxy)]-[2-Nal]-[4-amino- 4-carboxy-tetrahydropyran]-ENN-NH2 |
| 142 | Ac-[Pen]-NTWQ-[Pen]-[Phe[4-(2-aminoethoxy)]-[2-Nal]-[α-MeLeu]-[Lys(Ac)]-NN-OH |
| 143 | Ac-[Pen]-NTWQ-[Pen]-[Phe[4-(2-aminoethoxy)]-[2-Nal]-[α-MeLeu]-[Lys(Ac)]-ND-NH₂ |
| 144 | Ac-[Pen]-NTWQ-[Pen]-[Phe[4-(2-aminoethoxy)]-[2-Nal]-[α-MeLeu]-[Lys(Ac)]-DN-NH₂ |
| 145 | Ac-[Pen]-NTWE-[Pen]-[Phe[4-(2-aminoethoxy)]-[2-Nal]-[α-MeLeu]-[Lys(Ac)]-NN-NH₂ |
| 146 | Ac-[Pen]-DTWQ-[Pen]-[Phe[4-(2-aminoethoxy)]-[2-Nal]-[α-MeLeu]-[Lys(Ac)]-NN-NH₂ |
| 147 | Ac-[(D)Arg]-cyclo[[Abu]-QTWQC]-[Phe[4-(2-aminoethoxy)]-[2-Nal]-[3-cyclohexyl-Ala]- ENN-NH2 |
| 148 | Ac-[(D)Arg]-cyclo[[Abu]-QTWQC]-[Phe[4-(2-aminoethoxy)]-[2-Nal]-[Aib]-ENN-NH₂ |
| 149 | [(D)Arg]-cyclo[[Abu]-QTWQC]-[Phe[4-(2-aminoethoxy)]-[2-Nal]-[4-amino-4-carboxy- tetrahydropyran]-ENN-NH2 |
| 150 | Ac-[(D)Arg]-cyclo[[(D)Pen(sulfoxide)]-QTWQ-[Abu]]-[Phe[4-(2-aminoethoxy)]-[2-Nal]-[4- amino-4-carboxy-tetrahydropyran]-ENN-NH2 |
| 151 | Ac-cyclo[[(D)Pen]-QTWQ-[Abu]]-[Phe[4-(2-aminoethoxy)]-[2-Nal]-[4-amino-4-carboxy- tetrahydropyran]-ENN-NH2 |
| 152 | Ac-cyclo[[(D)Pen(sulfoxide )]-QTWQ-[Abu]]-[Phe[4-(2-aminoethoxy)]-[2-Nal]-[4-amino-4- carboxy-tetrahydropyran]-ENN-NH2 |
| 153 | Ac-[(D)Arg]-cyclo[[Abu]-QTW-Lys(isoGlu-Palm)-C]-[Phe[4-(2-aminoethoxy)]-[2-Nal]-[4- amino-4-carboxy-tetrahydropyran]-ENN-NH2 |
| 154 | Ac-[(D)Arg]-cyclo[[Abu]-QTW-[Lys(PEG12)]-C]-[Phe[4-(2-aminoethoxy)]-[2-Nal]-[4- amino-4-carboxy-tetrahydropyran]-ENN-NH2 |
| 155 | Ac-[(D)Arg]-cyclo[[Abu]-QTWQC]-[Phe[4-(2-aminoethoxy)]-[2-Nal]-[4-amino-4-carboxy- tetrahydropyran]-Lys(isoGlu-Palm)-NN-NH2 |
| 156 | Ac-[(D)Arg]-cyclo[[Abu]-QTWQC]-[Phe[4-(2-aminoethoxy)]-[2-Nal]-[4-amino-4-carboxy- tetrahydropyran]-[Lys(PEG12)]-NN-NH2 |
| 157 | Ac-[(D)Arg]-cyclo[[Abu]-QTWQC]-[Phe[4-(2-aminoethoxy)]-[2-Nal]-[Lys(PEG12)]-ENN- NH2 |
| 158 | Ac-[Pen]-NTWQ-[Pen]-[Phe[4-(2-aminoethoxy)]-[2-Nal]-[α-MeLcu]-[Lys(Ac)]-NND-NH₂ |
| 159 | Ac-[Pen]-NTWQ-[Pen]-[Phe[4-(2-aminoethoxy)]-[2-Nal]-[α-MeLeu]-[Lys(Ac)]-NNY-NH₂ |
| 160 | Ac-[Pen]-NTWQ-[Pen]-[Phe[4-(2-aminoethoxy)]-[2-Nal]-[α-MeLeu]-[Lys(Ac)]-NNW-NH₂ |
| 161 | Ac-[Pen]-NTWQ-[Pen]-[Phe[4-(2-aminoethoxy)]-[2-Nal]-[α-MeLeu]-[Lys(Ac)]-NNR-NH₂ |
| 162 | Ac-[Pen]-NTWQ-[Pen]-[Phe[4-(2-aminoethoxy)]-[2-Nal]-[α-MeLeu]-[Lys(Ac)]-NNL-NH₂ |
| 163 | Ac-[Pen]-NTWQ-[Pen]-[Phe[4-(2-aminoethoxy)]-[2-Nal]-[α-MeLeu]-[Lys(Ac)]-NNG-NH₂ |
| 164 | Ac-[Pen]-NTWQ-[Pen]-[Phe[4-(2-aminoethoxy)]-[2-Nal]-[α-MeLeu]-[Lys(Ac)]-NNQ-NH₂ |
| 165 | Ac-[Pen]-NTWQ-[Pen]-[Phe[4-(2-aminoethoxy)]-[2-Nal]-[α-MeLeu]-[Lys(Ac)]-NNS-NH₂ |
| 166 | Ac-[Pen]-NTWQ-[Pen]-[Phe[4-(2-aminoethoxy)]-[2-Nal]-[α-MeLeu]-[Lys(Ac)]-N- [Lys(Ac)]-NH2 |
| 167 | Ac-[Pen]-NTWQ-[Pen]-[Phe[4-(2-aminoethoxy)]-[2-Nal]-[α-MeLeu]-E-[Lys(Ac)]- [Lys(Ac)]-NH2 |
| 168 | Ac-[Pen]-NTWQ-[Pen]-[Phe[4-(2-aminoethoxy)]-[2-Nal]-[α-MeLeu]-ENN-NH₂ |
| 169 | Ac-[Pen]-[Lys(Ac)]-TWQ-[Pen]-[Phe[4-(2-aminoethoxy)]-[2-Nal]-[α-MeLeu]-[Lys(Ac)]- NN-NH2 |
| 170 | Ac-[Pen]-NTW-[Lys(Ac)]-[Pen]-[Phe[4-(2-aminoethoxy)]-[2-Nal]-[α-MeLeu]-[Lys(Ac)]- NN-NH2 |
| 171 | Ac-[Pen]-NTWQ-[Pen]-[Phe[4-(2-aminoethoxy)]-[2-Nal]-[α-MeLeu]-[Lys(Ac)]-NN- [(D)Asp]-NH2 |
| 172 | Ac-[Pen]-NTWQ-[Pen]-[Phe[4-(2-aminoethoxy)]-[2-Nal]-[α-MeLeu]-[Lys(Ac)]-NN- [(D)Tyr]-NH₂ |
| 173 | Ac-[Pen]-NTWQ-[Pen]-[Phe[4-(2-aminoethoxy)]-[2-Nal]-[α-MeLeu]-[Lys(Ac)]-NN-[2- Nal]-NH2 |
| 174 | Ac-[Pen]-NTWQ-[Pen]-[Phe[4-(2-aminoethoxy)]-[2-Nal]-[α-MeLeu]-[Lys(Ac)]-NN- [(D)Arg]-NH2 |
| 175 | Ac-[Pen]-NTWQ-[Pen]-[Phe[4-(2-aminoethoxy)]-[2-Nal]-[α-MeLeu]-[Lys(Ac)]-NN- [(D)Ile]-NH₂ |
| 176 | Ac-[Pen]-NTWQ-[Pen]-[Phe[4-(2-aminoethoxy)]-[2-Nal]-[α-MeLeu]-[Lys(Ac)]-NN- [(D)Ala]-NH2 |
| 177 | Ac-[Pen]-NTWQ-[Pen]-[Phe[4-(2-aminoethoxy)]-[2-Nal]-[α-MeLeu]-[Lys(Ac)]-NN- [(D)Gln]-NH2 |
| 178 | Ac-[Pen]-NTWQ-[Pen]-[Phe[4-(2-aminoethoxy)]-[2-Nal]-[α-MeLeu]-[Lys(Ac)]-NN- [(D)Ser]-NH2 |
| 179 | Ac-[Lys(Ac)]-[Pen]-NTWQ-[Pen]-[Phe[4-(2-aminoethoxy)]-[2-Nal]-[α-MeLeu]-[Lys(Ac)]- NN-NH2 |
| 180 | Ac-[Lys(Ac)]-[Pen]-NTWQ-[Pen]-[Phe(4-CONH₂)]-[2-Nal]-[α-MeLeu]-[Lys(Ac)]-NN-NH₂ |
| 181 | Ac-[Pen]-NTWQ-[Pen]-[Phe[4-(2-aminoethoxy)]-[2-Nal]-[α-MeLeu]-[Lys(Ac)]-NN- [Lys(Ac)]-NH2 |
| 182 | Ac-[Pen]-NTWQ-[Pen]-[Phe(4-CONH₂)]-[2-Nal]-[α-MeLeu]-[Lys(Ac)]-NN-[Lys(Ac)]-NH₂ |
| 183 | Ac-[Pen]-NTWQ-[Pen]-[Phe(4-CONH₂)]-[2-Nal]-[α-MeLeu]-[Lys(Ac)]-NN-[(D)Lys]-NH₂ |
| 184 | (Ac-[Pen]-NTWQ-[Pen]-[Phe(4-CONH2)]-[2-Nal]-[α-MeLeu]-[Lys(Ac)]-NN-[(D)Lys]- NH2)2 -DIG |
| 185 | Ac-[Pen]-NT-[Trp(5-F)]-Q-[Pen]-[Phe[4-(2-aminoethoxy)]-[2-Nal]-[α-MeLeu]-[Lys(Ac)]- NN-NH2 |
| 186 | Ac-[Pen]-NTWQ-[Pen]-[Phe[4-(2-aminoethoxy)]-[2-Nal]-[α-MeLeu]-[β-homo-Glu]-NN- NH2 |
| 187 | Ac-[Pen]-NTWQ-[Pen]-[Phe[4-(2-aminoethoxy)]-[2-Nal]-[α-MeLeu]-DNN-NH₂ |
| 188 | Ac-[Pen]-NTWQ-[Pen]-[Phe[4-(2-aminoethoxy)]-[2-Nal]-[α-MeLeu]-[Lys(Ac)]-[Cit]-N- NH2 |
| 189 | Ac-[Pen]-NTWQ-[Pen]-[Phe[4-(2-aminoethoxy)]-[2-Nal]-[α-MeLeu]-[Lys(Ac)]-N-[Cit]- NH2 |
| 190 | Ac-[Pen]-NTWQ-[Pen]-[Phe[4-(2-aminoethoxy)]-[2-Nal]-[α-MeLeu]-[Lys(Ac)]-NG-NH₂ |
| 191 | Ac-[Pen]-NTW-[Cit]-[Pen]-[Phe[4-(2-aminoethoxy)]-[2-Nal]-[α-MeLeu]-[Lys(Ac)]-NN- NH2 |
| 192 | Ac-[Pen]-NTWQ-[Pen]-[Phe[4-(2-aminoethoxy)]-[2-Nal]-[α-MeLeu]-[Lys(Ac)]-N-[Sarc]- NH2 |
| 193 | Ac-[Pen]-[Cit]-TW-[Cit]-[Pen]-[Phe[4-(2-aminoethoxy)]-[2-Nal]-[α-MeLeu]-[Lys(Ac)]-NN- NH2 |
| 194 | Ac-[Pen]-NTWQ[Pen]-[Phe[4-(2-aminoethoxy)]-[2-Nal]-[Deg]-[Lys(Ac)]-NN-NH₂ |
| 195 | Ac-[Pen]-NTW-[Lys(Ac)]-[Pen]-[Phe[4-(2-aminoethoxy)]-[2-Nal]-[α-MeLeu]-[Lys(Ac)]- NNG-NH₂ |
| 196 | Ac-[Pen]-NTW-[Lys(Ac)]-[Pen]-[Phe[4-(2-aminoethoxy)]-[2-Nal]-[α-MeLeu]-[Lys(Ac)]- NN-[β-Ala]-NH₂ |
| 197 | Ac-[Pen]-NTW-[Lys(Ac)]-[Pen]-[Phe[4-(2-aminoethoxy)]-[2-Nal]-[α-MeLeu]-[Lys(Ac)]- NN-[Ahx ]-NH₂ |
| 198 | Ac-[Pen]-NTW-[Lys(Ac)]-[Pen]-[Phe[4-(2-aminoethoxy)]-[2-Nal]-[α-MeLeu]-[Lys(Ac)]- NN-[Sar]-NH₂ |
| 199 | Ac-[Pen]-NTW-[Lys(Ac)]-[Pen]-[Phe[4-(2-aminoethoxy)]-[2-Nal]-[α-MeLeu]-[Lys(Ac)]- NN-[(D)Arg]-NH2 |
| 200 | Ac-[Pen]-NTW-[Lys(Ac)]-[Pen]-[Phe[4-(2-aminoethoxy)]-[2-Nal]-[α-MeLeu]-[Lys(Ac)]- NN-[(D)Ile]-NH2 |
| 201 | Ac-[Pen]-NTWQ-[Pen]-[Phe[4-(2-aminoethoxy)]-[2-Nal]-[α-MeLeu]-DNN-NH₂ |
| 202 | Ac-[Pen]-NT-[Trp(5-F)]-[Lys(Ac)]-[Pen]-[Phe[4-(2-aminoethoxy)]-[2-Nal]-[α-MeLeu]- [Lys(Ac)]-NN-NH₂ |
| 203 | Ac-[Pen]-NT-[Trp(5-F)]-[Lys(Ac)]-[Pen]-[Phe[4-(2-aminoethoxy)]-[2-Nal]-[α-MeLeu]- [Lys(Ac)]-NNG-NH2 |
| 204 | (Ac-[Pen]-NTW-[Lys(Ac)]-[Pen]-[Phe[4-(2-aminoethoxy)]-[2-Nal]-[α-MeLeu]-[Lys(Ac)]- NN-NH2)2-[-C(O)CH2OCH2C(O)-]* |
| 205 | (Ac-[Pen]-NTW-[Lys(Ac)]-[Pen]-[Phe[4-(2-aminoethoxy)]-[2-Nal]-[α-MeLeu]-[Lys(Ac)]- NN-NH2)2-[-C(O)CH2N(H)CH2C(O)-]** |
| 206 | (Ac-[Pen]-NTW-[Lys(Ac)]-[Pen]-[Phe[4-(2-aminoethoxy)]-[2-Nal]-[α-MeLeu]-[Lys(Ac)]- NN-NH2)2-[-C(O)-(CH2CH2O)2CH2CH2C(O)-]*** |
| 207 | (Ac-[Pen]-NTW-[Lys(Ac)]-[Pen]-[Phe[4-(2-aminoethoxy)]-[2-Nal]-[α-MeLeu]-[Lys(Ac)]- NN-NH2)2-[-C(O)-(CH2CH2O)4CH2CH2C(O)-]*** |
| 208 | (Ac-[Pen]-NTW-[Lys(Ac)]-[Pen]-[Phe[4-(2-aminoethoxy)]-[2-Nal]-[α-MeLeu]-[Lys(Ac)]- NN-NH2)2-[-C(O)-(CH2CH2O)13CH2CH2C(O)-]*** |
| 209 | Ac-[Pen]-NTW-[Lys(Ac)]-[Pen]-[Phe[4-(2-aminoethoxy)]-[2-Nal]-[α-MeLeu]-[Lys(Ac- Arg)]-NN-[(D)Ile]-NH2 |
| 210 | Ac-[Pen]-NTW[-Lys(Ac)]-[Pen]-[Phe[4-(2-aminoethoxy)]-[2-Nal]-[α-MeLeu]-[Lys(Ac- Tyr)]-NN-[(D)Ile]-NH2 |
| 211 | Ac-[Pen]-NTW[-Lys(Ac)]-[Pen]-[Phe[4-(2-aminoethoxy)]-[2-Nal]-[α-MeLeu]-[Lys(Ac- Asn)]-NN-[(D)Ile]-NH2 |
| 212 | Ac-[Pen]-NTW-[Lys(Ac)]-[Pen]-[Phe[4-(2-aminoethoxy)]-[2-Nal]-[α-MeLeu]-[Lys(Ac- Thr)]-NN-[(D)Ile]-NH₂ |
| 213 | Ac-[Pen]-NTW-[Lys(Ac)]-[Pen]-[Phe[4-(2-aminoethoxy)]-[2-Nal]-[α-MeLeu]-[Lys(Ac- Asp)]-NN-[(D)Ile]-NH2 |
| 214 | Ac-[Pen]-NTW-[Lys(Ac)]-[Pen]-[Phe[4-(2-aminoethoxy)]-[2-Nal]-[α-MeLeu]-[Lys(Ac- Leu)]-NN-[(D)Ile]-NH2 |
| 215 | Ac-[Pen]-NTW-[Lys(Ac)]-[Pen]-[Phe[4-(2-aminoethoxy)]-[2-Nal]-[α-MeLeu]-[Lys(Ac- Phe)]-NN-[(D)Ile]-NH2 |
| 216 | Ac-[Pen]-NTW-[Lys(Ac)]-[Pen]-[Phe[4-(2-aminoethoxy)]-[2-Nal]-[α-MeLeu]-[Lys(Ac- (D)Arg)]-NN-[(D)Ile]-NH2 |
| 217 | Ac-[Pen]-NTW-[Lys(Ac)]-[Pen]-[Phe[4-(2-aminoethoxy)]-[2-Nal]-[α-MeLeu]-[Lys(Ac- (D)Tyr)]-NN-[(D)Ile]-NH2 |
| 218 | Ac-[Pen]-NTW-[Lys(Ac)]-[Pen]-[Phe[4-(2-aminoethoxy)]-[2-Nal]-[α-MeLeu]-[Lys(Ac- (D)Asn)]-NN-[(D)Ile]-NH2 |
| 219 | Ac-[Pen]-NTW-[Lys(Ac)]-[Pen]-[Phe[4-(2-aminoethoxy)]-[2-Nal]-[α-MeLeu]-[Lys(Ac- (D)Thr)]-NN-[(D)Ile]-NH2 |
| 220 | Ac-[Pen]-NTW-[Lys(Ac)]-[Pen]-[Phe[4-(2-aminoethoxy)]-[2-Nal]-[α-MeLeu]-[Lys(Ac- (D)Asp)]-NN-[(D)Ile]-NH2 |
| 221 | Ac-[Pen]-NTW-[Lys(Ac)]-[Pen]-[Phe[4-(2-aminoethoxy)]-[2-Nal]-[α-MeLeu]-[Lys(Ac- ((D)Leu)]-NN-[(D)Ile]-NH2 |
| 222 | Ac-[Pen]-NTW-[Lys(Ac)]-[Pen]-[Phe[4-(2-aminoethoxy)]-[2-Nal]-[α-MeLeu]-[Lys(Ac- (D)Phe)]-NN-[(D)Ile]-NH2 |
| 223 | Ac-[Lys(Ac-Glu)]-[Pen]-NTW-[Lys(Ac)]-[Pen]-[Phe[4-(2-aminoethoxy)]-[2-Nal]-[α- MeLeu]-[Lys(Ac)]-NN-NH2 |
| 224 | Ac-[Lys(Ac-Phe)]-[Pen]-NTW-[Lys(Ac)]-[Pen]-[Phe[4-(2-aminoethoxy)]-[2-Nal]-[α- MeLeu]-[Lys(Ac)]-NN-NH2 |
| 225 | Ac-[Lys(Ac-Tyr)]-[Pen]-NTW-[Lys(Ac)]-[Pen]-[Phe[4-(2-aminoethoxy)]-[2-Nal]-[α- MeLeu]-[Lys(Ac)]-NN-NH₂ |
| 226 | Ac-[Lys(Ac-Ser)]-[Pen]-NTW-[Lys(Ac)]-[Pen]-[Phe[4-(2-aminoethoxy)]-[2-Nal]-[α- MeLeu]-[Lys(Ac)]-NN-NH₂ |
| 227 | Ac-[Lys(Ac-Arg)]-[Pen]-NTW-[Lys(Ac)]-[Pen]-[Phe[4-(2-aminoethoxy)]-[2-Nal]-[α- MeLeu]-[Lys(Ac)]-NN-NH2 |
| 228 | Ac-[Lys(Ac-Leu)]-[Pen]-NTW-[Lys(Ac)]-[Pen]-[Phe[4-(2-aminoethoxy)]-[2-Nal]-[α- MeLeu]-[Lys(Ac)]-NN-NH2 |
| 229 | Ac-[Lys(Ac-Pro)]-[Pen]-NTW-[Lys(Ac)]-[Pen]-[Phe[4-(2-aminoethoxy)]-[2-Nal]-[α- MeLeu]-[Lys(Ac)]-NN-NH2 |
| 230 | Ac-[(D)Lys(Ac-Glu)]-[Pen]-NTW-[Lys(Ac)]-[Pen]-[Phe[4-(2-aminoethoxy)]-[2-Nal]-[α- MeLeu]-[Lys(Ac)]-NN-NH₂ |
| 231 | Ac-[(D)Lys(Ac-Phe)]-[Pen]-NTW-[Lys(Ac)]-[Pen]-[Phe[4-(2-aminoethoxy)]-[2-Nal]-[α- MeLeu]-[Lys(Ac)]-NN-NH₂ |
| 232 | Ac-[(D)Lys(Ac-Tyr)]-[Pen]-NTW-[Lys(Ac)]-[Pen]-[Phe[4-(2-aminoethoxy)]-[2-Nal]-[α- MeLeu]-[Lys(Ac)]-NN-NH2 |
| 233 | Ac-[(D)Lys(Ac-Ser)]-[Pen]-NTW-[Lys(Ac)]-[Pen]-[Phe[4-(2-aminoethoxy)]-[2-Nal]-[α- MeLeu]-[Lys(Ac)]-NN-NH2 |
| 234 | Ac-[(D)Lys(Ac-Arg)]-[Pen]-NTW-[Lys(Ac)]-[Pen]-[Phe[4-(2-aminoethoxy)]-[2-Nal]-[α- MeLeu]-[Lys(Ac)]-NN-NH2 |
| 235 | Ac-[(D)Lys(Ac-Leu)]-[Pen]-NTW-[Lys(Ac)]-[Pen]-[Phe[4-(2-aminoethoxy)]-[2-Nal]-[α- MeLeu]-[Lys(Ac)]-NN-NH₂ |
| 236 | Ac-[(D)Lys(Ac-Pro)]-[Pen]-NTW-[Lys(Ac)]-[Pen]-[Phe[4-(2-aminoethoxy)]-[2-Nal]-[α- MeLeu]-[Lys(Ac)]-NN-NH2 |

| | |
|---|---|
| *wherein NH₂ of 2-aminoethoxy and C(O) of DIG are joined to form a single bond: **wherein NH₂ of 2-aminoethoxy and C(O) of IDA are joined to form a single bond: ***wherein NH₂ of 2-aminoethoxy and C(O) of PEG are joined to form a single bond. | |

In a particular embodiment, the peptide comprises or consists of the sequence of : 
Alexa488-[PEG4]-[(D)Arg]-cyclo[[Abu]-QTWQC]-[Phe[4-(2-aminoethoxy)]-[2-Nal]-[4-
amino-4-carboxy-tetrahydropyran]-ENN-NH2 (SEQ ID NO:71);
 
[Biotin]-[PEG4]-[(D)Arg]-cyclo[[Abu]-QTWQC]-[Phe[4-(2-aminoethoxy)]-[2-Nal]-[4-
amino-4-carboxy-tetrahydropyran]-ENN-NH2 (SEQ ID NO:89);
 
Ac-[(D)Arg]-cyclo[[Abu]-QT-[Trp(5-F)]-QC]-[Phe[4-(2-aminoethoxy)]-[2-Nal]-[α-MeLeu]-
ENN-NH2 (SEQ ID NO:105);
 
Ac-cyclo[[Abu]-QT-[Trp(5-F)]-QC]-[Phe[4-(2-aminoethoxy)]-[2-Nal]-[α-MeLeu]-ENN-
NH2 (SEQ ID NO:106);
 
Ac-[(D)Arg]-cyclo[[Abu]-QTWQC]-[Phe[4-(2-aminoethoxy)]-[2-Nal]-[α-MeLeu]-ENN-
NH2 (SEQ ID NO:107);
Ac-[(D)Arg]-cyclo[[Abu]-QTWQC]-[Phe[4-(2-aminoethoxy)]-[2-Nal]-[α-MeLeu]-ENN-NH₂ (SEQ ID NO:138); 
Ac-[(D)Arg]-cyclo[[Abu]-QTWQC]-[Phe[4-(2-aminoethoxy)]-[2-Nal]-[3-cyclohexyl-Ala]-
ENN-NH2 (SEQ ID NO:147);
Ac-[(D)Arg]-cyclo[[Abu]-QTWQC]-[Phe[4-(2-aminoethoxy)]-[2-Nal]-[Aib]-ENN-NH₂ (SEQ ID NO:148);
Ac-[Pen]-NTWQ-[Pen]-[Phe[4-(2-aminoethoxy)]-[2-Nal]-[α-MeLeu]-[Lys(Ac)]-NNY-NH₂ (SEQ ID NO:159);
Ac-[Pen]-NTWQ-[Pen]-[Phe[4-(2-aminoethoxy)]-[2-Nal]-[α-MeLeu]-[Lys(Ac)]-NNR-NH₂ (SEQ ID NO:161);
Ac-[Pen]-NTWQ-[Pen]-[Phe[4-(2-aminoethoxy)]-[2-Nal]-[α-MeLeu]-[Lys(Ac)]-NNQ-NH₂ (SEQ ID NO:164);
Ac-[Pen]-NTWQ-[Pen]-[Phe[4-(2-aminoethoxy)]-[2-Nal]-[α-MeLeu]-[Lys(Ac)]-NNS-NH₂ (SEQ ID NO:165);

Ac-[Pen]-NTW-[Lys(Ac)]-[Pen]-[Phe[4-(2-aminoethoxy)]-[2-Nal]-[α-MeLeu]-[Lys(Ac)]-
NN-NH2 (SEQ ID NO:170);

Ac-[Pen]-NTWQ-[Pen]-[Phe[4-(2-aminoethoxy)]-[2-Nal]-[α-MeLeu]-[Lys(Ac)]-NN-
[(D)Arg]-NH2 (SEQ ID NO:174);

Ac-[Pen]-NTWQ-[Pen]-[Phe[4-(2-aminoethoxy)]-[2-Nal]-[α-MeLeu]-[Lys(Ac)]-NN-
[(D)Ile]-NH2 (SEQ ID NO:175);

Ac-[Pen]-NTWQ-[Pen]-[Phe[4-(2-aminoethoxy)]-[2-Nal]-[α-MeLeu]-[Lys(Ac)]-NN-
[(D)Gln]-NH2 (SEQ ID NO:177);

(Ac-[Pen]-NTWQ-[Pen]-[Phe(4-CONH2)]-[2-Nal]-[α-MeLeu]-[Lys(Ac)]-NN-[(D)Lys]-
NH2)2 –DIG (SEQ ID NO:184);

Ac-[Pen]-NTW-[Lys(Ac)]-[Pen]-[Phe[4-(2-aminoethoxy)]-[2-Nal]-[α-MeLeu]-[Lys(Ac)]-
NNG-NH2 (SEQ ID NO:195);

Ac-[Pen]-NTW-[Lys(Ac)]-[Pen]-[Phe[4-(2-aminoethoxy)]-[2-Nal]-[α-MeLeu]-[Lys(Ac)]-
NN-[β-Ala]-NH2 (SEQ ID NO:196);

Ac-[Pen]-NTW-[Lys(Ac)]-[Pen]-[Phe[4-(2-aminoethoxy)]-[2-Nal]-[α-MeLeu]-[Lys(Ac)]-
NN-[Ahx]-NH2 (SEQ ID NO:197);

Ac-[Pen]-NTW-[Lys(Ac)]-[Pen]-[Phe[4-(2-aminoethoxy)]-[2-Nal]-[α-MeLeu]-[Lys(Ac)]-
NN-[Sar]-NH2 (SEQ ID NO:198);

Ac-[Pen]-NTW-[Lys(Ac)]-[Pen]-[Phe[4-(2-aminoethoxy)]-[2-Nal]-[α-MeLeu]-[Lys(Ac-
(D)Asn)]-NN-[(D)Ile]-NH2 (SEQ ID NO:218);

Ac-[Lys(Ac-Glu)]-[Pen]-NTW-[Lys(Ac)]-[Pen]-[Phe[4-(2-aminoethoxy)]-[2-Nal]-[α-
MeLeu]-[Lys(Ac)]-NN-NH2 (SEQ ID NO:223);

Ac-[Lys(Ac-Phe)]-[Pen]-NTW-[Lys(Ac)]-[Pen]-[Phe[4-(2-aminoethoxy)]-[2-Nal]-[α-
MeLeu]-[Lys(Ac)]-NN-NH2 (SEQ ID NO:224);

Ac-[Lys(Ac-Tyr)]-[Pen]-NTW-[Lys(Ac)]-[Pen]-[Phe[4-(2-aminoethoxy)]-[2-Nal]-[α-
MeLeu]-[Lys(Ac)]-NN-NH2 (SEQ ID NO:225);

Ac-[Lys(Ac-Ser)]-[Pen]-NTW-[Lys(Ac)]-[Pen]-[Phe[4-(2-aminoethoxy)]-[2-Nal]-[α-
MeLeu]-[Lys(Ac)]-NN-NH2 (SEQ ID NO:226);

Ac-[Lys(Ac-Arg)]-[Pen]-NTW-[Lys(Ac)]-[Pen]-[Phe[4-(2-aminoethoxy)]-[2-Nal]-[α-
MeLeu]-[Lys(Ac)]-NN-NH2 (SEQ ID NO:227);

Ac-[Lys(Ac-Leu)]-[Pen]-NTW-[Lys(Ac)]-[Pen]-[Phe[4-(2-aminoethoxy)]-[2-Nal]-[α-
MeLeu]-[Lys(Ac)]-NN-NH2 (SEQ ID NO:228);

Ac-[Lys(Ac-Pro)]-[Pen]-NTW-[Lys(Ac)]-[Pen]-[Phe[4-(2-aminoethoxy)]-[2-Nal]-[α-
MeLeu]-[Lys(Ac)]-NN-NH2 (SEQ ID NO:229);

Ac-[(D)Lys(Ac-Glu)]-[Pen]-NTW-[Lys(Ac)]-[Pen]-[Phe[4-(2-aminoethoxy)]-[2-Nal]-[α-
MeLeu]-[Lys(Ac)]-NN-NH2 (SEQ ID NO:230);

Ac-[(D)Lys(Ac-Phe)]-[Pen]-NTW-[Lys(Ac)]-[Pen]-[Phe[4-(2-aminoethoxy)]-[2-Nal]-[α-
MeLeu]-[Lys(Ac)]-NN-NH2 (SEQ ID NO:231);

Ac-[(D)Lys(Ac-Tyr)]-[Pen]-NTW-[Lys(Ac)]-[Pen]-[Phe[4-(2-aminoethoxy)]-[2-Nal]-[α-
MeLeu]-[Lys(Ac)]-NN-NH2 (SEQ ID NO:232);

Ac-[(D)Lys(Ac-Ser)]-[Pen]-NTW-[Lys(Ac)]-[Pen]-[Phe[4-(2-aminoethoxy)]-[2-Nal]-[α-
MeLeu]-[Lys(Ac)]-NN-NH2 (SEQ ID NO:233);

Ac-[(D)Lys(Ac-Arg)]-[Pen]-NTW-[Lys(Ac)]-[Pen]-[Phe[4-(2-aminoethoxy)]-[2-Nal]-[α-
MeLeu]-[Lys(Ac)]-NN-NH2 (SEQ ID NO:234);

Ac-[(D)Lys(Ac-Leu)]-[Pen]-NTW-[Lys(Ac)]-[Pen]-[Phe[4-(2-aminoethoxy)]-[2-Nal]-[α-
MeLeu]-[Lys(Ac)]-NN-NH2 (SEQ ID NO:235); or

Ac-[(D)Lys(Ac-Pro)]-[Pen]-NTW-[Lys(Ac)]-[Pen]-[Phe[4-(2-aminoethoxy)]-[2-Nal]-[α-
MeLeu]-[Lys(Ac)]-NN-NH2 (SEQ ID NO:236).

In another particular embodiments, the peptide comprises or consists of the sequence of: 
Ac-[(D)Arg]-[Pen]-QTWQ-[Pen]-[Phe[4-(2-aminoethoxy)]-[2-Nal]-[ 4-amino-4-carboxy-
tetrahydropyran]-ENN-NH2 (SEQ ID NO:1);
 
Ac-[Pen]-N-[Dab]-WQ-[Pen]-[Phe[4-(2-aminoethoxy)]-[2-Nal]-[ 4-amino-4-carboxy-
tetrahydropyran]-ENN-NH2 (SEQ ID NO:3);
 
Ac-[Pen]-NT-[2-Nal]-Q-[Pen]-[Phe[4-(2-aminoethoxy)]-[2-Nal]- [4-amino-4-carboxy-
tetrahydropyran]-[Lys(Ac)]-NN-NH2 (SEQ ID NO:5);
 
Ac-[Pen]-NTWE-[Pen]-[Phe[4-(2-aminoethoxy)]-[2-Nal]-[ 4-amino-4-carboxy-tetrahydropyran]-
[Lys(Ac)]-NN-NH2 (SEQ ID NO:6);
 
Ac-[Pen]-NTWQ-[Pen]-[Phe[4-(2-aminoethoxy)]-W-[4-amino-4-carboxy-tetrahydropyran]-
[Lys(Ac)]-NN-NH2 (SEQ ID NO:8);
Ac-[Pen]-NT-[Trp(5-F)]-Q-[Pen]-[Phe[4-(2-aminoethoxy)]-W-[α-MeLeu]-[Lys(Ac)]-NN-NH₂ (SEQ ID NO:9); 
Ac-[(D)Arg]-cyclo[[Abu]-QTWQC]-[Phe[4-(2-aminoethoxy)]-[2-Nal]-[4-amino-4-carboxy-
tetrahydropyran]-END-NH2 (SEQ ID NO:73)
 
Ac-[(D)Arg]-cyclo[[Abu]-QTWEC]-[Phe[4-(2-aminoethoxy)]-[2-Nal]-[4-amino-4-carboxy-
tetrahydropyran]-ENN-NH2 (SEQ ID NO:75);
 
Ac-[(D)Arg]-cyclo[[Abu]-ETWQC]-[Phe[4-(2-aminoethoxy)]-[2-Nal]-[4-amino-4-carboxy-
tetrahydropyran]-ENN-NH2 (SEQ ID NO:76);
 
Ac-[(D)Arg]-cyclo[[Abu]-QTWEC]-[Phe[4-(2-aminoethoxy)]-[2-Nal]-[4-amino-4-carboxy-
tetrahydropyran]-END-NH2 (SEQ ID NO:78);
 
Ac-[(D)Arg]-cyclo[[Abu]-ETWQC]-[Phe[4-(2-aminoethoxy)]-[2-Nal]-[4-amino-4-carboxy-
tetrahydropyran]-END-NH2 (SEQ ID NO:79);
 
Ac-[(D)Arg]-cyclo[[Abu]-ETWEC]-[Phe[4-(2-aminoethoxy)]-[2-Nal]-[4-amino-4-carboxy-
tetrahydropyran]-ENN-NH2 (SEQ ID NO:82);
 
Ac-[(D)Arg]-cyclo[[Abu]-QTWQC]-[Phe[4-(2-aminoethoxy)]-[2-Nal]-[4-amino-4-carboxy-
tetrahydropyran]-ENN-OH (SEQ ID NO:83);
 
Ac-[(D)Arg]-cyclo[[Abu]-QTWEC]-[Phe[4-(2-aminoethoxy)]-[2-Nal]-[4-amino-4-carboxy-
tetrahydropyran]-ENN-OH (SEQ ID NO:86);
 
[Ac-[(D)Arg]-cyclo[[Abu]-ETWQC]-[Phe[4-(2-aminoethoxy)]-[2-Nal]-[4-amino-4-carboxy-
tetrahydropyran]-ENN-OH (SEQ ID NO:87);
 
[NH2-PEG4]-[(D)Arg]-cyclo[[Abu]-QTWQC]-[Phe[4-(2-aminoethoxy)]-[2-Nal]-[4-amino-4-carboxy-
tetrahydropyran]-ENN-NH2 (SEQ ID NO:88);
 
[NH2-PEG4]-[(D)Arg]-cyclo[[Abu]-QTWQC]-[Phe[4-(2-aminoethoxy)-(PEG4-NH2)]-[2-Nal]-[4-
amino-4-carboxy-tetrahydropyran]-ENN-NH2 (SEQ ID NO:90);
 
[NH₂-PEG4]-[(D)Arg]-cyclo[[Abu]-QTWQC]-[Phe[4-(2-aminoethoxy)-(PEG4)-(Biotin)]-[2-Nal]-[4-
amino-4-carboxy-tetrahydropyran]-ENN-NH₂ (SEQ ID NO:91);
 
Ac-[(D)Arg]-cyclo[[Abu]-QTW-[a-MeLeu]-C]-[Phe[4-(2-aminoethoxy)]-[2-Nal]-[4-amino-4-
carboxy-tetrahydropyran]-ENN-NH2 (SEQ ID NO:93);
 
Ac-[(D)Arg]-cyclo[[Abu]-QTW-[a-MeLys(Ac)]-C]-[Phe[4-(2-aminoethoxy)]-[2-Nal]-[4-amino-4-
carboxy-tetrahydropyran]-ENN-NH2 (SEQ ID NO:95);
 
Ac-[(D)Arg]-cyclo[[Abu]-QTWWC]-[Phe[4-(2-aminoethoxy)]-[2-Nal]-[4-amino-4-carboxy-
tetrahydropyran]-ENN-NH₂ (SEQ ID NO:100);
 
Ac-[(D)Arg]-cyclo[[Abu]-QT-[Trp(5-F)]-QC]-[Phe[4-(2-aminoethoxy)]-[2-Nal]-[4-amino-4-carboxy-
tetrahydropyran]-ENN-NH2 (SEQ ID NO:104);
Ac-cyclo[[(D)Abu]-NTWQ-[Pen]]-[Phe[4-(2-aminoethoxy)]-[2-Nal]-[α-MeLeu]-[Lys(Ac)]-NN-NH₂ (SEQ ID NO:136); 
Ac-[(D)Arg]-cyclo[[Abu]-QTW-[2-Nal]-C]-[Phe[4-(2-aminoethoxy)]-[2-Nal]-[4-amino-4-carboxy-
tetrahydropyran]-ENN-NH₂ (SEQ ID NO:139);
 
Ac-[(D)Arg]-cyclo[[Abu]-QTW-[1-Nal]-C]-[Phe[4-(2-aminoethoxy)]-[2-Nal]-[4-amino-4-carboxy-
tetrahydropyran]-ENN-NH2 (SEQ ID NO:140);
 
Ac-[(D)Arg]-cyclo[[Abu]-QTW-[5-fluor-Trp]-C]-[Phe[4-(2-aminoethoxy)]-[2-Nal]-[4-amino-4-
carboxy-tetrahydropyran]-ENN-NH2 (SEQ ID NO:141);
Ac-[Pen]-NTWQ-[Pen]-[Phe[4-(2-aminoethoxy)]-[2-Nal]-[α-MeLcu]-[Lys(Ac)]-NN-OH(SEQ ID NO:142);
Ac-[Pen]-NTWQ-[Pen]-[Phe[4-(2-aminoethoxy)]-[2-Nal]-[α-MeLeu]-[Lys(Ac)]-ND-NH₂ (SEQ ID NO:143);
Ac-[Pen]-NTWE-[Pen]-[Phe[4-(2-aminoethoxy)]-[2-Nal]-[α-MeLeu]-[Lys(Ac)]-NN-NH₂ (SEQ ID NO:145);
Ac-[Pen]-DTWQ-[Pen]-[Phe[4-(2-aminoethoxy)]-[2-Nal]-[α-MeLeu]-[Lys(Ac)]-NN-NH₂ (SEQ ID NO: 146); 
Ac-[(D)Arg]-cyclo[[Abu]-QTW-[Lys(PEG12)]-C]-[Phe[4-(2-aminoethoxy)]-[2-Nal]-[4-amino-4-
carboxy-tetrahydropyran]-ENN-NH2 (SEQ ID NO:154);
 
Ac-[(D)Arg]-cyclo[[Abu]-QTWQC]-[Phe[4-(2-aminoethoxy)]-[2-Nal]-[4-amino-4-carboxy-
tetrahydropyran]-[Lys(PEG12)]-NN-NH2 (SEQ ID NO:156);
Ac-[Pen]-NTWQ-[Pen]-[Phe[4-(2-aminoethoxy)]-[2-Nal]-[α-MeLeu]-[Lys(Ac)]-NND-NH₂ (SEQ ID NO: 158);
Ac-[Pen]-NTWQ-[Pen]-[Phe[4-(2-aminoethoxy)]-[2-Nal]-[α-MeLeu]-[Lys(Ac)]-NNW-NH₂ (SEQ ID NO: 160);
Ac-[Pen]-NTWQ-[Pen]-[Phe[4-(2-aminoethoxy)]-[2-Nal]-[α-MeLeu]-[Lys(Ac)]-NNL-NH₂ (SEQ ID NO: 162);
Ac-[Pen]-NTWQ-[Pen]-[Phe[4-(2-aminoethoxy)]-[2-Nal]-[α-MeLeu]-[Lys(Ac)]-NNG-NH₂ (SEQ ID NO: 163);
Ac-[Pen]-NTWQ-[Pen]-[Phe[4-(2-aminoethoxy)]-[2-Nal]-[α-MeLeu]-[Lys(Ac)]-N-[Lys(Ac)]-NH₂ (SEQ ID NO:166);
Ac-[Pen]-NTWQ-[Pen]-[Phe[4-(2-aminoethoxy)]-[2-Nal]-[α-MeLeu]-ENN-NH₂ (SEQ ID NO:168);
Ac-[Pen]-[Lys(Ac)]-TWQ-[Pen]-[Phe[4-(2-aminoethoxy)]-[2-Nal]-[α-MeLeu]-[Lys(Ac)]-NN-NH₂ (SEQ ID NO:169);
Ac-[Pen]-NTWQ-[Pen]-[Phe[4-(2-aminoethoxy)]-[2-Nal]-[α-MeLeu]-[Lys(Ac)]-NN-[(D)Asp]-NH₂ (SEQ ID NO:171);
Ac-[Pen]-NTWQ-[Pen]-[Phe[4-(2-aminoethoxy)]-[2-Nal]-[α-MeLeu]-[Lys(Ac)]-NN-[(D)Tyr]-NH₂ (SEQ ID NO:172);
Ac-[Pen]-NTWQ-[Pen]-[Phe[4-(2-aminoethoxy)]-[2-Nal]-[α-MeLcu]-[Lys(Ac)]-NN-[2-Nal]-NH₂ (SEQ ID NO:173);
Ac-[Pen]-NTWQ-[Pen]-[Phe[4-(2-aminoethoxy)]-[2-Nal]-[α-MeLcu]-[Lys(Ac)]-NN-[(D)Ala]-NH₂ (SEQ ID NO:176);
Ac-[Pen]-NTWQ-[Pen]-[Phe[4-(2-aminoethoxy)]-[2-Nal]-[α-MeLcu]-[Lys(Ac)]-NN-[(D)Ser]-NH₂ (SEQ ID NO:178);
Ac-[Lys(Ac)]-[Pen]-NTWQ-[Pen]-[Phe[4-(2-aminoethoxy)]-[2-Nal]-[α-MeLeu]-[Lys(Ac)]-NN-NH₂ (SEQ ID NO:179);
Ac-[Lys(Ac)]-[Pen]-NTWQ-[Pen]-[Phe(4-CONH₂)]-[2-Nal]-[α-MeLeu]-[Lys(Ac)]-NN-NH₂ (SEQ ID NO: 180);
Ac-[Pen]-NTWQ-[Pen]-[Phe[4-(2-aminoethoxy)]-[2-Nal]-[α-MeLeu]-[Lys(Ac)]-NN-[Lys(Ac)]-NH₂ (SEQ ID NO:181);
Ac-[Pen]-NTWQ-[Pen]-[Phe(4-CONH₂)]-[2-Nal]-[α-MeLeu]-[Lys(Ac)]-NN-[Lys(Ac)]-NH₂ (SEQ ID NO: 182);
Ac-[Pen]-NTWQ-[Pen]-[Phe(4-CONH₂)]-[2-Nal]-[α-MeLeu]-[Lys(Ac)]-NN-[(D)Lys]-NH₂ (SEQ ID NO: 183); 
Ac-[Pen]-NT-[5-Fluro-Trp]-Q-[Pen]-[Phe[4-(2-aminoethoxy)]-[2-Nal]-[α-MeLeu]-[Lys(Ac)]-NN-
NH2 (SEQ ID NO:185);
Ac-[Pen]-NTWQ-[Pen]-[Phe[4-(2-aminoethoxy)]-[2-Nal]-[α-MeLcu]-[β-homo-Glu]-NN-NH₂ (SEQ ID NO:186);
Ac-[Pen]-NTWQ-[Pen]-[Phe[4-(2-aminoethoxy)]-[2-Nal]-[α-MeLcu]-[Lys(Ac)]-[Cit]-N-NH₂ (SEQ ID NO:188);
Ac-[Pen]-NTWQ-[Pen]-[Phe[4-(2-aminoethoxy)]-[2-Nal]-[α-MeLeu]-[Lys(Ac)]-N-[Cit]-NH₂ (SEQ ID NO:189);
Ac-[Pen]-NTWQ-[Pen]-[Phe[4-(2-aminoethoxy)]-[2-Nal]-[α-MeLeu]-[Lys(Ac)]-NG-NH₂ (SEQ ID NO:190);
Ac-[Pen]-NTW-[Cit]-[Pen]-[Phe[4-(2-aminoethoxy)]-[2-Nal]-[α-MeLeu]-[Lys(Ac)]-NN-NH₂ (SEQ ID NO:191);
Ac-[Pen]-NTWQ-[Pen]-[Phe[4-(2-aminoethoxy)]-[2-Nal]-[α-MeLeu]-[Lys(Ac)]-N-[Sarc]-NH₂ (SEQ ID NO:192);
Ac-[Pen]-[Cit]-TW-[Cit]-[Pen]-[Phe[4-(2-aminoethoxy)]-[2-Nal]-[α-MeLeu]-[Lys(Ac)]-NN-NH₂ (SEQ ID NO:193);
Ac-[Pen]-NTWQ[Pen]-[Phe[4-(2-aminoethoxy)]-[2-Nal]-[Deg ]-[Lys(Ac)]-NN-NH₂ (SEQ ID NO:194); 
Ac-[Pen]-NTW-[Lys(Ac)]-[Pen]-[Phe[4-(2-aminoethoxy)]-[2-Nal]-[α-MeLeu]-[Lys(Ac)]-NN-
[(D)Arg]-NH2 (SEQ ID NO:199);
 
Ac-[Pen]-NTW-[Lys(Ac)]-[Pen]-[Phe[4-(2-aminoethoxy)]-[2-Nal]-[α-MeLeu]-[Lys(Ac)]-NN-
[(D)Ile]-NH2 (SEQ ID NO:200);
 
Ac-[Pen]-NT-[Trp(5-F)]-[Lys(Ac)]-[Pen]-[Phe[4-(2-aminoethoxy)]-[2-Nal]-[α-MeLeu]-[Lys(Ac)]-
NN-NH2 (SEQ ID NO:202);
 
Ac-[Pen]-NT-[Trp(5-F)]-[Lys(Ac)]-[Pen]-[Phe[4-(2-aminoethoxy)]-[2-Nal]-[α-MeLeu]-[Lys(Ac)]-
NNG-NH2 (SEQ ID NO:203);
 
(Ac-[Pen]-NTW-[Lys(Ac)]-[Pen]-[Phe[4-(2-aminoethoxy)]-[2-Nal]-[α-MeLeu]-[Lys(Ac)]-NN-
NH₂)₂-DIG (SEQ ID NO:204);
 
(Ac-[Pen]-NTW-[Lys(Ac)]-[Pen]-[Phe[4-(2-aminoethoxy)]-[2-Nal]-[α-MeLeu]-[Lys(Ac)]-NN-
NH₂)₂-PEG4 (SEQ ID NO:207);
 
(Ac-[Pen]-NTW-[Lys(Ac)]-[Pen]-[Phe[4-(2-aminoethoxy)]-[2-Nal]-[α-MeLeu]-[Lys(Ac)]-NN-
NH₂)₂ -PEG13 (SEQ ID NO:208);
 
Ac-[Pen]-NTW-[Lys(Ac)]-[Pen]-[Phe[4-(2-aminoethoxy)]-[2-Nal]-[α-MeLeu]-[Lys(Ac-Arg)]- NN-
[(D)Ile]-NH2 (SEQ ID NO:209);
 
Ac-[Pen]-NTW[-Lys(Ac)]-[Pen]-[Phe[4-(2-aminoethoxy)]-[2-Nal]-[α-MeLeu]-[Lys(Ac-Tyr)]-NN-
[(D)Ile]-NH2 (SEQ ID NO:210);
 
Ac-[Pen]-NTW[-Lys(Ac)]-[Pen]-[Phe[4-(2-aminoethoxy)]-[2-Nal]-[α-MeLeu]-[Lys(Ac-Asn)]-NN-
[(D)Ile]-NH2 (SEQ ID NO:211);
 
Ac-[Pen]-NTW-[Lys(Ac)]-[Pen]-[Phe[4-(2-aminoethoxy)]-[2-Nal]-[α-MeLeu]-[Lys(Ac-Thr)]-NN-
[(D)Ile]-NH2 (SEQ ID NO:212);
 
Ac-[Pen]-NTW-[Lys(Ac)]-[Pen]-[Phe[4-(2-aminoethoxy)]-[2-Nal]-[α-MeLeu]-[Lys(Ac-Asp)]-NN-
[(D)Ile]-NH2 (SEQ ID NO:213);
 
Ac-[Pen]-NTW-[Lys(Ac)]-[Pen]-[Phe[4-(2-aminoethoxy)]-[2-Nal]-[α-MeLeu]-[Lys(Ac-Leu)]-NN-
[(D)Ile]-NH₂ (SEQ ID NO:214);
 
Ac-[Pen]-NTW-[Lys(Ac)]-[Pen]-[Phe[4-(2-aminoethoxy)]-[2-Nal]-[α-MeLeu]-[Lys(Ac-Phe)]-NN-
[(D)Ile]-NH2 (SEQ ID NO:215);
 
Ac-[Pen]-NTW-[Lys(Ac)]-[Pen]-[Phe[4-(2-aminoethoxy)]-[2-Nal]-[α-MeLeu]-[Lys(Ac-(D)Arg)]-
NN-[(D)Ile]-NH₂ (SEQ ID NO:216);
 
Ac-[Pen]-NTW-[Lys(Ac)]-[Pen]-[Phe[4-(2-aminoethoxy)]-[2-Nal]-[α-MeLeu]-[Lys(Ac-(D)Tyr)]-
NN-[(D)Ile]-NH2 (SEQ ID NO:217);
 
Ac-[Pen]-NTW-[Lys(Ac)]-[Pen]-[Phe[4-(2-aminoethoxy)]-[2-Nal]-[α-MeLeu]-[Lys(Ac-(D)Thr)]-
NN-[(D)Ile]-NH2 (SEQ ID NO:219);
 
Ac-[Pen]-NTW-[Lys(Ac)]-[Pen]-[Phe[4-(2-aminoethoxy)]-[2-Nal]-[α-MeLeu]-[Lys(Ac-(D)Asp)]-
NN-[(D)Ile]-NH2 (SEQ ID NO:220);
 or

Ac-[Pen]-NTW-[Lys(Ac)]-[Pen]-[Phe[4-(2-aminoethoxy)]-[2-Nal]-[α-MeLeu]-[Lys(Ac-(D)Phe)]-
NN-[(D)Ile]-NH2 (SEQ ID NO:222).

### Additional Characteristics of Peptide Inhibitors

Any of the peptide inhibitors of the present invention may be further defined, e.g., as described below. It is understood that each of the further defining features described herein may be applied to any peptide inhibitors where the amino acids designated at particular positions allow the presence of the further defining feature. In particular embodiments, these features may be present in any of the peptides of Formula (I), (II), (III), (IV), (V), or (XII)-(XVIIIh).

In various embodiments, R¹ is a bond, hydrogen, a C1-C6 alkyl, a C6-C12 aryl, a C6-C12 aryl C1-C6 alkyl, or a C1-C20 alkanoyl, and including PEGylated versions alone or as spacers of any of the foregoing, e.g., acetyl. It is understood that the R¹ may replace or be present in addition to the typical amine group located at the amino terminus of a peptide. It is further understood that R¹ may be absent. In certain embodiments, the peptide inhibitor comprises an N-terminus selected from hydrogen, a C1-C6 alkyl, a C6-C12 aryl, a C6-C12 aryl C1-C6 alkyl, or a C1-C20 alkanoyl, and including PEGylated versions alone or as spacers of any of the foregoing, e.g., acetyl. In particular embodiments of any of the peptide inhibitors described herein, R¹ or the N-terminal moiety is hydrogen. In certain embodiments, R¹ is a bond, e.g., a covalent bond.

In certain embodiments of any of the peptide inhibitors having any of the various Formulas set forth herein, R¹ or the N-terminal moiety is selected from methyl, acetyl, formyl, benzoyl, trifluoroacetyl, isovaleryl, isobutyryl, octanyl, and the conjugated amides of lauric acid, hexadecanoic acid, and γ-Glu-hexadecanoic acid. In one embodiment, R¹ or the N-terminal moiety is pGlu. In certain embodiments, R¹ is hydrogen. In particular embodiments, R¹ is acetyl, whereby the peptide inhibitor is acylated at its N-terminus, e.g., to cap or protect an N-terminal amino acid residue, e.g., an N-terminal Pen or Abu residue.

In certain embodiments of any of the peptide inhibitors described herein, R¹ or the N-terminal moiety is an acid. In certain embodiments, R¹ or the N-terminal moiety is an acid selected from acetic acid, formic acid, benzoic acid, trifluoroacetic acid, isovaleric acid, isobutyric acid, octanoic acid, lauric acid, hexadecanoic acid, 4-Biphenylacetic acid, 4-fluorophenylacetic acid, gallic acid, pyroglutamic acid, cyclopentanepropionic acid, glycolic acid, oxalic acid, pyruvic acid, lactic acid, malonic acid, succinic acid, malic acid, maleic acid, fumaric acid, tartaric acid, citric acid, palmitic acid, benzoic acid, 3-(4-hydroxybenzoyl) benzoic acid, cinnamic acid, mandelic acid, 4-methylbicyclo(2.2.2)-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, an alkylsulfonic acid and an arylsulfonic acid.

In particular embodiments, R¹ or the N-terminal moiety is an alkylsulfonic acid selected from methanesulfonic acid, ethanesulfonic acid, 1,2-ethane-disulfonic acid, and 2-hydroxyethanesulfonic acid.

In particular embodiments, R¹ or the N-terminal moiety is an arylsulfonic acid selected from benzenesulfonic acid, 4- chlorobenzenesulfonic acid, 2-naphthalenesulfonic acid, 4-toluenesulfonic acid, and camphorsulfonic acid.

In some embodiments, wherein a peptide of the present invention comprises a conjugation to an acidic compound such as, e.g., isovaleric acid, isobutyric acid, valeric acid, and the like, the presence of such a conjugation is referenced in the acid form. So, for example, but not to be limited in any way, instead of indicating a conjugation of isovaleric acid to a peptide by referencing isovaleroyl (e.g., isovaleroyl-[Pen]-QTWQ[Pen]-[Phe(4-OMe)]-[2-Nal]-[α-MeLys]-[Lys(Ac)]-NG-NH₂ (SEQ ID NO:307), in some embodiments, the present application references such a conjugation as isovaleric acid-[Pen]-QTWQ[Pen]-[Phe(4-OMe)]-[2-Nal]-[α-MeLys]-[Lys(Ac)]-NG-NH₂ (SEQ ID NO:307). Reference to the conjugation in its acid form is intended to encompass the form present in the peptide inhibitor.

In certain embodiments, the peptide inhibitor comprises a C-terminus (e.g., R² or the C-termial moiety) selected from a bond, OH or NH₂. In certain embodiments, R² is a bond. In various embodiments of any of the peptide inhibitors having any of the various Formulas set forth herein, R² or the C-terminal moiety is OH or NH₂. It is understood that the R² or the C-terminal moiety may replace or be present in addition to the carboxyl group typically located at the carboxy terminus of a peptide. It is further understood that R² may be absent.

### Peptide Dimers

In certain embodiments, the present invention includes dimers of the monomer peptide inhibitors described herein, including dimers of any of the monomer peptide inhibitors described herein or in the accompanying tables. These dimers fall within the scope of the general term "peptide inhibitors" as used herein. Illustrative dimers of the present invention are also shown in the accompanying tables, which indicate the dimerized monomer subnits in brackets followed by the linker. Unless otherwise indicated, the subunits are linked via their C-termini. The term "dimer," as in a peptide dimer, refers to compounds in which two peptide monomer subinits are linked. A peptide dimer inhibitor of the present invention may comprise two identical monomer subunits, resulting in a homodimer, or two non-identical monomer subunits, resulting in a heterodimer. A cysteine dimer comprises two peptide monomer subunits linked through a disulfide bond between a cysteine residue in one monomer subunit and a cysteine residue in the other monomer subunit.

In some embodiments, the peptide inhibitors of the present invention may be active in a dimer conformation, in particular when free cysteine residues are present in the peptide. In certain embodiments, this occurs either as a synthesized dimer or, in particular, when a free cysteine monomer peptide is present and under oxidizing conditions, dimerizes. In some embodiments, the dimer is a homodimer. In other embodiments, the dimer is a heterodimer.

In certain embodiments, monomer subunits of the present invention may be dimerized by a suitable linking moiety, e.g., a disulphide bridge between two cysteine residues, one in each peptide monomer subunit or by another suitable linker moiety, including but not limited to those defined herein. Some of the monomer subunits are shown having C- and N-termini that both comprise free amine. Thus, to produce a peptide dimer inhibitor, the monomer subunit may be modified to eliminate either the C- or N-terminal free amine, thereby permitting dimerization at the remaining free amine. Further, in some instances, a terminal end of one or more monomer subunits is acylated with an acylating organic compound selected from the group consisting of: Trifluoropentyl, Acetyl, Octonyl, Butyl, Pentyl, Hexyl, Palmityl, Trifluoromethyl butyric, cyclopentane carboxylic, cyclopropylacetic, 4-fluorobenzoic, 4-fluorophenyl acetic, 3-Phenylpropionic, tetrahedro-2H-pyran-4carboxylic, succinic acid, and glutaric acid. In some instances, monomer subunits comprise both a free carboxy terminal and a free amino terminal, whereby a user may selectively modify the subunit to achieve dimerization at a desired terminus. One having skill in the art therefore, will appreciate that the monomer subunits of the instant invention may be selectively modified to achieve a single, specific amine for a desired dimerization.

It is further understood that the C-terminal residues of the monomer subunits disclosed herein are optionally amides. Further, it is understood that, in certain embodiments, dimerization at the C-terminus is facilitated by using a suitable amino acid with a side chain having amine functionality, as is generally understood in the art. Regarding the N-terminal residues, it is generally understood that dimerization may be achieved through the free amine of the terminal residue, or may be achieved by using a suitable amino acid side chain having a free amine, as is generally understood in the art.

The linker moieties connecting monomer subunits may include any structure, length, and/or size that is compatible with the teachings herein. In at least one embodiment, a linker moiety is selected from the non-limiting group consisting of cysteine, lysine, DIG, PEG4, PEG4-biotin, PEG13, PEG25, PEG1K, PEG2K, PEG3.4K, PEG4K, PEG5K, IDA, ADA, Boc-IDA, Glutaric acid, Isophthalic acid, 1,3-phenylenediacetic acid, 1,4-phenylenediacetic acid, 1,2-phenylenediacetic acid, Triazine, Boc-Triazine, IDA-biotin, PEG4-Biotin, AADA, suitable aliphatics, aromatics, heteroaromatics, and polyethylene glycol based linkers having a molecular weight from approximately 400Da to approximately 40,000Da. In certain embodiments, PEG2 is HO₂CCH₂CH₂OCH₂CH₂OCH₂CH₂CO₂H. Non-limiting examples of suitable linker moieties are provided in Table 7.

**Table 7. Illustrative Linker Moieties**

| Abbrivation | Discription | Structure |
|---|---|---|
| DIG | DIGlycolic acid, | 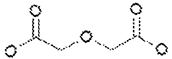 |
| PEG4 | Bifunctional PEG linker with 4 PolyEthylene Glycol units | 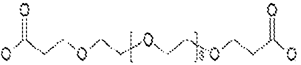 |
| PEG13 | Bifunctional PEG linker with 13 PolyEthylene Glycol units | 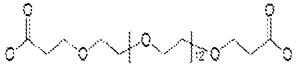 |
| PEG25 | Bifunctional PEG linker with 25 PolyEthylene Glycol units | 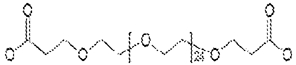 |
| PEG1K | Bifunctional PEG linker with PolyEthylene Glycol Mol wt of 1000Da | |
| PEG2K | Bifunctional PEG linker with PolyEthylene Glycol Mol wt of 2000Da | |
| PEG3.4K | Bifunctional PEG linker with PolyEthylene Glycol Mol wt of 3400Da | |
| PEG5K | Bifunctional PEG linker with PolyEthylene Glycol Mol wt of 5000Da | |
| DIG | DIGlycolic acid | 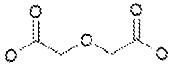 |
| β-Ala-IDA | β-Ala-Iminodiacetic acid | 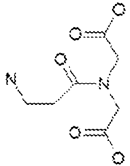 |
| Boc-β - Ala-IDA | Boc- β -Ala-Iminodiacetic acid | 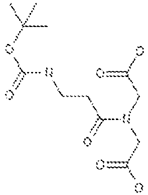 |
| Ac-β -Ala - IDA | Ac- β -Ala-Iminodiacetic acid | 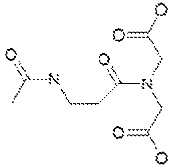 |
| IDA-β-Ala-Palm | Palmityl- β -Ala-Iminodiacetic acid | 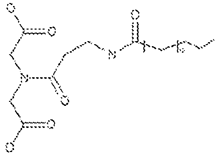 |
| GTA | Glutaric acid | 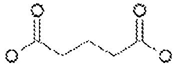 |
| PMA | Pemilic acid | 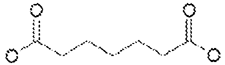 |
| AZA | Azelaic acid | 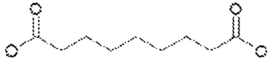 |
| DDA | Dodecanedioic acid | 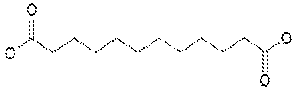 |
| IPA | Isopthalic aicd | 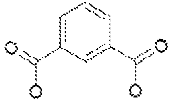 |
| 1,3-PDA | 1,3- Phenylenediacetic acid | 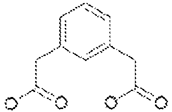 |
| 1,4-PDA | *1,4*- Phenylenediacetic acid | 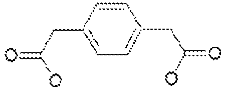 |
| 1,2-PDA | 1,2 - Phenylenediacetic acid | 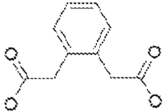 |
| Triazine | Amino propyl Triazine di-acid | 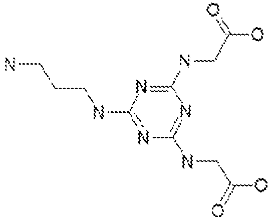 |
| Boc-Triazine | Boc-Triazine di-acid | 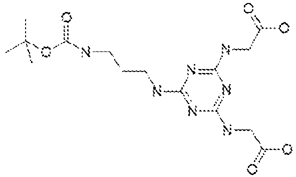 |
| ADA | Amino diacetic acid (which may also referred to as Iminodiacetic acid) | 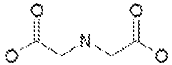 |
| AADA | n-Acetyl amino acetic acid (which may also referred to as N-acetyl Iminodiacetic acid) | 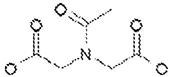 |
| PEG4-Biotin | PEG4-Biotin (Product number 10199, QuantaBioDesign) | 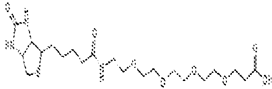 |
| IDA-Biotin | N-Biotin- β -Ala-Iminodiacetic acid | 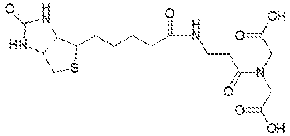 |
| Lys | Lysine | 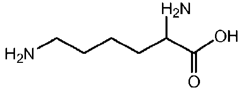 |

In some embodiments, a peptide dimer inhibitor is dimerized via a linker moiety. In some embodiments, a peptide dimer inhibitor is dimerized via an intermolecular disulfide bond formed between two cysteine residues, one in each monomer subunit. In some embodiments, a peptide dimer inhibitor is dimerized via both a linker moiety and an intermolecular disulfide bond formed between two cysteine residues. In some embodiments, the intramolecular bond is a thioether, lactam, triazole, selenoether, diselenide or olefin, instead of the disulfide bond.

One having skill in the art will appreciate that the linker (e.g., C- and N-terminal linker) moieties disclosed herein are non-limiting examples of suitable linkers, and that the present invention may include any suitable linker moiety. Thus, some embodiments of the present invention comprises a homo- or heterodimer peptide inhibitor comprised of two monomer subunits selected from the peptides shown in any of tables herein or comprising or consisting of a sequence presented in any of tables herein, wherein the C- or N-termini of the respective monomer subunits (or internal amino acid residues) are linked by any suitable linker moiety to provide a dimer peptide inhibitor having IL-23R inhibitory activity. In certain embodiments, a linker binds to the N- or C-terminus of one monomer subunit and an internal amino acid residue of the other monomer subunit making up the dimer. In certain embodiments, a linker binds to an internal amino acid residue of one monomer subunit and an internal amino acid residue of the other monomer subunit making up the dimer. In further embodiments, a linker binds to the N-or C-terminus of both subunits.

In particular embodiments, one or both of the monomer subunits comprise the sequence or structure of any one of Formula I, II, III, IV, V, XII-XVIIIh, or any of the peptides described herein, e.g., in Tables 2-6.

In certain embodiments, a peptide dimer inhibitor has the structure of Formula VI:

(R¹-X-R²)₂-L (VI)

or a pharmaceutically acceptable salt or solvate thereof,
wherein each R¹ is independently absent, a bond (e.g., a covalent bond), or R¹ is selected from hydrogen, a C1-C6 alkyl, a C6-C12 aryl, a C6-C 12 aryl C1-C6 alkyl, a C1-C20 alkanoyl, and including PEGylated versions alone or as spacers of any of the foregoing;
each R² is independently absent, a bond (e.g., a covalent bond), or selected from OH or NH₂; L is a linker moiety; andeach X is an independently selected peptide monomer subunit comprising a sequence of Formula (I), (II), (V), (III), (IV), or (XII)-(XVIIIh), as described herein. In certain embodiments, one or both peptide monomer subunit of a peptide dimer inhibitor is cyclized, e.g., via an intramolecular bond between X4 and X9. In certain embodiments, one or both peptide monomer subunits is linear or not cyclized.

In particular embodiments, of the peptide dimer inhibitors, each X7 and each X11 are both W. In certain embodiments of the peptide dimer inhibitors, one or both peptide monomer subunit has a structure shown herein, e.g., in Tables 2, 3, 4, 5, or 6.

In particular embodiments, each R¹ is independently a bond (e.g., a covalent bond), or selected from hydrogen, a C1-C6 alkyl, a C6-C12 aryl, a C6-C12 aryl C1-C6 alkyl, a C1-C20 alkanoyl, and including PEGylated versions alone or as spacers of any of the foregoing. In particular embodimetns, the N-terminus of each subunit includes a moiety selected from hydrogen, a C1-C6 alkyl, a C6-C12 aryl, a C6-C12 aryl C1-C6 alkyl, a C1-C20 alkanoyl, and including PEGylated versions alone or as spacers of any of the foregoing.

In certain embodiments of any of the peptide inhibitors having any of the various Formulae set forth herein, each R¹ (or N-terminal moiety) is selected from methyl, acetyl, formyl, benzoyl, trifluoroacetyl, isovaleryl, isobutyryl, octanyl, and the conjugated amides of lauric acid, hexadecanoic acid, and γ-Glu-hexadecanoic acid.

In particular embodiments, each R² (or C-terminal moiety) is independently a bond (e.g., a covalent bond), or selected from OH or NH_{2.}

In particular embodiments of any of the peptide dimer inhibitors described herein, either or both R¹ is hydrogen.

In particular embodiments of peptide dimer inhibitors of the present invention, the linker moiety (L) is any of the linkers described herein or shown in Table 1 or 7. In certain embodiments, L is a lysine linker, a diethylene glycol linker, an iminodiacetic acid (IDA) linker, a β-Ala-iminodiaceticacid (β-Ala-IDA) linker, or a PEG linker.

In various embodiments of any of the peptide dimer inhibitors, each of the peptide monomer subunits is attached to a linker moiety via its N-terminus, C-terminus, or an internal amino acid residue. In certain embodiments of any of the peptide dimer inhibitors, the N-terminus of each peptide monomer subunit is connected by a linker moiety.In certain embodiments of any of the peptide dimer inhibitors, the C-terminus of each peptide monomer subunit is connected by a linker moiety.In certain embodiments of any of the peptide dimer inhibitors, each peptide monomer subunit is connected by a linker moiety attached to an internal amino acid.

### Peptide Inhibitor Conjugates and Biopolymers

In certain embodiments, peptide inhibitors of the present invention, including both monomers and dimers, comprise one or more conjugated chemical substituents, such as lipophilic substituents and polymeric moieties, which may be referred to herein as half-life extension moieties. Without wishing to be bound by any particular theory, it is believed that the lipophilic substituent binds to albumin in the bloodstream, thereby shielding the peptide inhibitor from enzymatic degradation, and thus enhancing its half-life. In addition, it is believed that polymeric moieties enhance half-life and reduce clearance in the bloodstream.

In additional embodiments, any of the peptide inhibitors, e.g. peptides of Formula (I), (II), (V), (III), (IV), or (XI) further comprise a linker moiety attached to an amino acid residue present in the inhibitor, e.g., a linker moiety may be bound to a side chain of any amino acid of the peptide inhibitor, to the N-terminal amino acid of the peptide inhibitor, or to the C-terminal amino acid of the peptide inhibitor.

In additional embodiments, any of the peptide inhibitors e.g. peptides of Formulas (I)-(VI) or (XI), further comprise half-life extension moiety attached to an amino acid residue present in the inhibitor, e.g., a half-life extension moiety may be bound to a side chain of any amino acid of the peptide inhibitor, to the N-terminal amino acid of the peptide inhibitor, or to the C-terminal amino acid of the peptide inhibitor.

In additional embodiments, any of the peptide inhibitors e.g. peptides of Formulas (I)-(VI) or (XI), further comprise half-life extension moiety attached to a linker moiety that is attached to an amino acid residue present in the inhibitor, e.g., a half-life extension moiety may be bound to a linker moiety that is bound to a side chain of any amino acid of the peptide inhibitor, to the N-terminal amino acid of the peptide inhibitor, or to the C-terminal amino acid of the peptide inhibitor.

In particular embodiments, a peptide inhibitor comprises a half-life extension moiety having the structure shown below, wherein n=0 to 24 or n=14 to 24: 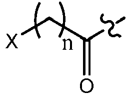
n=0 to 24
X=CH₃, CO₂H, NH₂, OH

In certain embodiments, a peptide inhibitor of the present invention comprises a half-life extension moiety shown in Table 8.

**Table 8. Illustrative Half-Life Extension Moieties**

| # | **Half-Life Extension Moietys** |
|---|---|
| C1 | 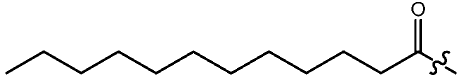 C12 (Lauric acid) |
| C2 | 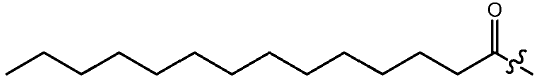 C14 (Mysteric acid) |
| C3 | 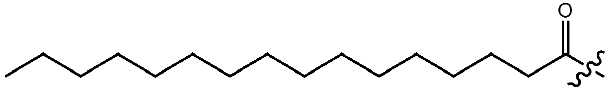 C16 (Palm or Palmitic acid) |
| C4 | 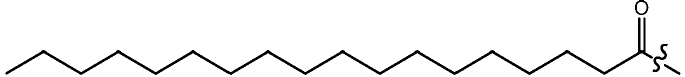 C18 (Stearic acid) |
| C5 | 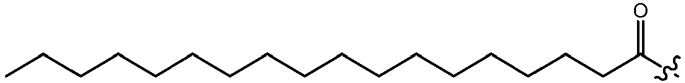 20 |
| C6 | 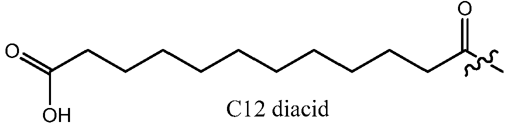 |
| C7 | 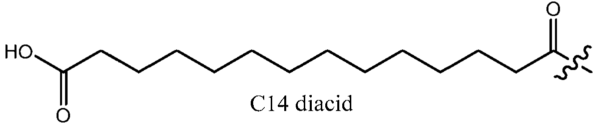 |
| C8 | 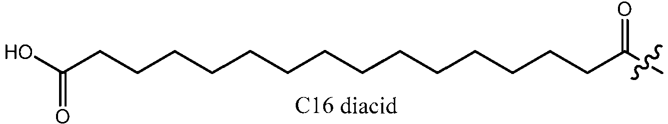 |
| C9 | 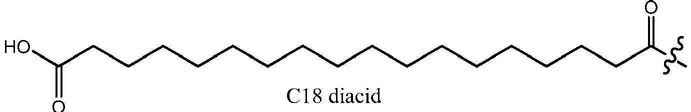 |
| C10 | 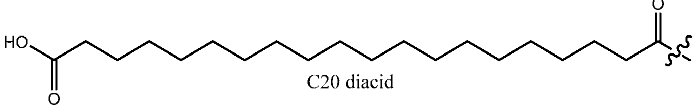 |

In certain embodiments, a half-life extension moiety is bound directly to a peptide inhibitor, while in other embodiments, a half-life extension moiety is bound to the peptide inhibitor via a linker moiety, e.g., any of those depicted in Tables 1, 7 or 9.

**Table 9. Illustrative Linker Moieties**

| # | **Linker Moiety** |
|---|---|
| L1 | 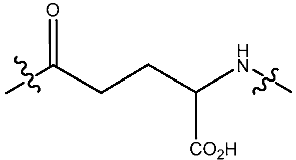 |
| | IsoGlu |
| L2 | 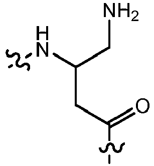 Dapa |
| L3 | 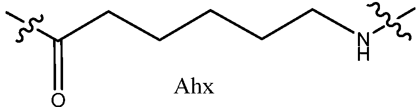 |
| L4 | Lipidic based linkers: 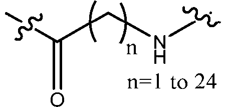 |
| L5 | 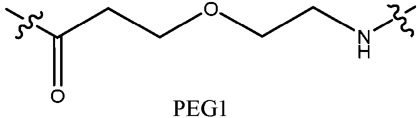 |
| L6 | 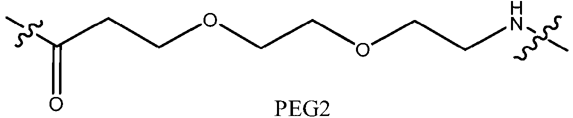 |
| L7 | 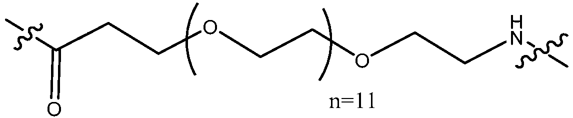 PEG11 (40 atoms) also known as PEG12 |
| L8 | 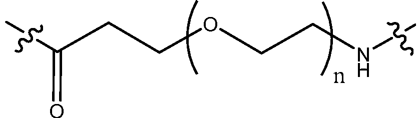 n=1 to 25 |
| | PEG based linkers |
| L9 | 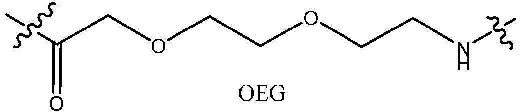 |
| L10 | 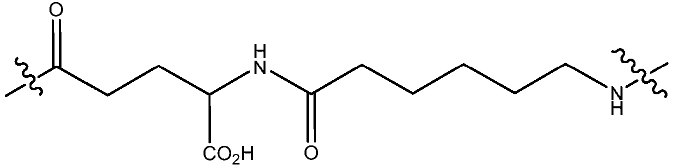 IsoGlu-Ahx |
| L11 | 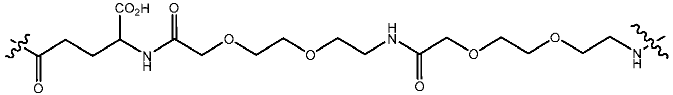 IsoGlu-OEG-OEG |
| L12 | 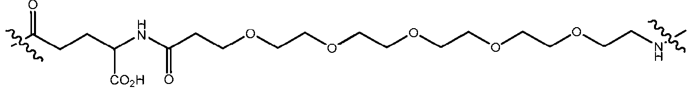 IsoGlu-PEG5 |
| L13 | 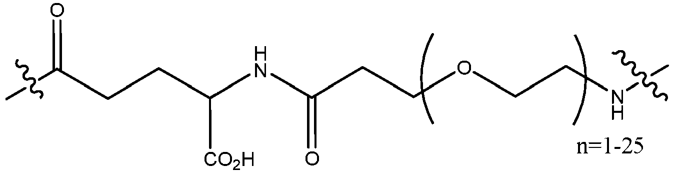 IsoGlu-PEGn |
| L14 | 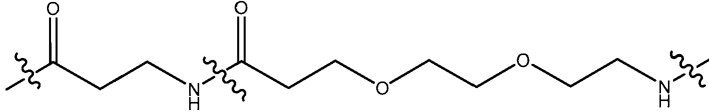 βAla-PEG2 |
| L15 | 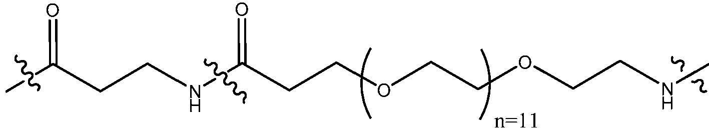 βAla-PEG11 (40 atoms) |

In particular embodiments, a peptide inhibitor of the present invention comprises any of the linker moieties shown in Tables 7 or 9 and any of the half-life extension moieties shown in Table 8, including any of the following combinations shown in Table 10.

**Table 10. Illustrative Combinations of Linkers and Half-Life Extension Moieties in Peptide Inhibitors**

| Linker | Half-Life Extension Moiety | | Linker | Half-Life Extension Moiety | | Linker | Half-Life Extension Moiety |
|---|---|---|---|---|---|---|---|
| L1 | C1 | | L1 | C2 | | L1 | C3 |
| L2 | C1 | | L2 | C2 | | L2 | C3 |
| L3 | C1 | | L3 | C2 | | L3 | C3 |
| L4 | C1 | | L4 | C2 | | L4 | C3 |
| L5 | C1 | | L5 | C2 | | L5 | C3 |
| L6 | C1 | | L6 | C2 | | L6 | C3 |
| L7 | C1 | | L7 | C2 | | L7 | C3 |
| L8 | C1 | | L8 | C2 | | L8 | C3 |
| L9 | C1 | | L9 | C2 | | L9 | C3 |
| L10 | C1 | | L10 | C2 | | L10 | C3 |
| L11 | C1 | | L11 | C2 | | L11 | C3 |
| L12 | C1 | | L12 | C2 | | L12 | C3 |
| L13 | C1 | | L13 | C2 | | L13 | C3 |
| L14 | C1 | | L14 | C2 | | L14 | C3 |
| L15 | C1 | | L15 | C2 | | L15 | C3 |
| | | | | | | | |

| Linker | Half-Life Extension Moiety | | Linker | Half-Life Extension Moiety | | Linker | Half-Life Extension Moiety |
|---|---|---|---|---|---|---|---|
| L1 | C4 | | L1 | C5 | | L1 | C6 |
| L2 | C4 | | L2 | C5 | | L2 | C6 |
| L3 | C4 | | L3 | C5 | | L3 | C6 |
| L4 | C4 | | L4 | C5 | | L4 | C6 |
| L5 | C4 | | L5 | C5 | | L5 | C6 |
| L6 | C4 | | L6 | C5 | | L6 | C6 |
| L7 | C4 | | L7 | C5 | | L7 | C6 |
| L8 | C4 | | L8 | C5 | | L8 | C6 |
| L9 | C4 | | L9 | C5 | | L9 | C6 |
| L10 | C4 | | L10 | C5 | | L10 | C6 |
| L11 | C4 | | L11 | C5 | | L11 | C6 |
| L12 | C4 | | L12 | C5 | | L12 | C6 |
| L13 | C4 | | L13 | C5 | | L13 | C6 |
| L14 | C4 | | L14 | C5 | | L14 | C6 |
| L15 | C4 | | L15 | C5 | | L15 | C6 |
| | | | | | | | |

| Linker | Half-Life Extension Moiety | | Linker | Half-Life Extension Moiety | | Linker | Half-Life Extension Moiety |
|---|---|---|---|---|---|---|---|
| L1 | C7 | | L1 | C8 | | L1 | C9 |
| L2 | C7 | | L2 | C8 | | L2 | C9 |
| L3 | C7 | | L3 | C8 | | L3 | C9 |
| L4 | C7 | | L4 | C8 | | L4 | C9 |
| L5 | C7 | | L5 | C8 | | L5 | C9 |
| L6 | C7 | | L6 | C8 | | L6 | C9 |
| L7 | C7 | | L7 | C8 | | L7 | C9 |
| L8 | C7 | | L8 | C8 | | L8 | C9 |
| L9 | C7 | | L9 | C8 | | L9 | C9 |
| L10 | C7 | | L10 | C8 | | L10 | C9 |
| L11 | C7 | | L11 | C8 | | L11 | C9 |
| L12 | C7 | | L12 | C8 | | L12 | C9 |
| L13 | C7 | | L13 | C8 | | L13 | C9 |
| L14 | C7 | | L14 | C8 | | L14 | C9 |
| L15 | C7 | | L15 | C8 | | L15 | C9 |
| | | | | | | | |
| L1 | C10 | | L6 | C10 | | L11 | C10 |
| L2 | C10 | | L7 | C10 | | L12 | C10 |
| L3 | C10 | | L8 | C10 | | L13 | C10 |
| L4 | C10 | | L9 | C10 | | L14 | C10 |
| L5 | C10 | | L10 | C10 | | L15 | C10 |

In some embodiments there may be multiple linkers present between the peptide the conjugated moiety, e.g., half-life extension moiety, e.g., as depicted in Table 11.

**Table 11. Illustrative Combinations of Linkers and Half-Life Extension Moieties in Peptide Inhibitors**

| Linker | Half-Life Extension Moiety | | Linker | Half-Life Extension Moiety |
|---|---|---|---|---|
| L1-L2 | C10 | | L1-L2 | C8 |
| L2-L5-L3 | C10 | | L2-L5-L3 | C8 |
| L3-L8 | C10 | | L3-L8 | C8 |
| L1-L2-L3 | C10 | | L1-L2-L3 | C8 |
| L5-L3-L3-L3 | C10 | | L5-L3-L3-L3 | C8 |

In certain embodiments, the half-life of a peptide inhibitor of the invention that includes a conjugated chemical substituent, i.e., a half-life extension moiety, is at least 100%, at least 120%, at least 150%, at least 200%, at least 250%, at least 300%, at least 400%, or at least 500% of the half-life of the same peptide inhibitor but without the conjugated chemical substituent. In certain embodiments, the lipophilic substituents and/or polypermic moieties enhance the permeability of the peptide inhibitor through the epithelium and/or its retention in the lamina propria. In certain embodiments, the permeability through the epithelium and/or the retention in the lamina propria of a peptide inhibitor of the invention that includes a conjugated chemical substituent is at 100%, at least 120%, at least 150%, at least 200%, at least 250%, at least 300%, at least 400%, or at least 500% of the half-life of the same peptide inhibitor but without the conjugated chemical substituent.

In one embodiment, a side chain of one or more amino acid residues (e.g., Lys residues) in a peptide inhibitor of the invention is conjugated (e.g., covalently attached) to a lipophilic substituent. The lipophilic substituent may be covalently bonded to an atom in the amino acid side chain, or alternatively may be conjugated to the amino acid side chain via one or more spacers. The spacer, when present, may provide spacing between the peptide analogue and the lipophilic substituent. In particular embodiments, the peptide inhibitor comprises any of the conjugated moieties shown in peptides disclosed in Tables 2-6.

In certain embodiments, the lipophilic substituent may comprise a hydrocarbon chain having from 4 to 30 C atoms, for example at least 8 or 12 C atoms, and preferably 24 C atoms or fewer, or 20 C atoms or fewer. The hydrocarbon chain may be linear or branched and may be saturated or unsaturated. In certain embodiments, the hydrocarbon chain is substituted with a moiety which forms part of the attachment to the amino acid side chain or the spacer, for example an acyl group, a sulfonyl group, an N atom, an O atom or an S atom. In some embodiments, the hydrocarbon chain is substituted with an acyl group, and accordingly the hydrocarbon chain may form part of an alkanoyl group, for example palmitoyl, caproyl, lauroyl, myristoyl or stearoyl.

A lipophilic substituent may be conjugated to any amino acid side chain in a peptide inhibitor of the invention. In certain embodiment, the amino acid side chain includes a carboxy, hydroxyl, thiol, amide or amine group, for forming an ester, a sulphonyl ester, a thioester, an amide or a sulphonamide with the spacer or lipophilic substituent. For example, the lipophilic substituent may be conjugated to Asn, Asp, Glu, Gln, His, Lys, Arg, Ser, Thr, Tyr, Trp, Cys or Dbu, Dpr or Om. In certain embodiments, the lipophilic substituent is conjugated to Lys. An amino acid shown as Lys in any of the Formula provided herein may be replaced by, e.g., Dbu, Dpr or Orn where a lipophilic substituent is added.

In certain embodiments, the peptide inhibitors of the present invention may be modified, e.g., to enhance stability, increase permeability, or enhance drug like characteristics, through conjugation of a chemical moiety to one or more amino acid side chain within the peptide. For example, the N(epsilon) of lysine N(epsilon), the β-carboxyl of aspartic, or the γ-carboxyl of glutamic acid may be appropriately functionalized. Thus, to produce the modified peptide, an amino acid within the peptide may be appropriately modified. Further, in some instances, the side chain is acylated with an acylating organic compound selected from the group consisting of: Trifluoropentyl, Acetyl, Octonyl, Butyl, Pentyl, Hexyl, Palmityl, Trifluoromethyl butyric, cyclopentane carboxylic, cyclopropylacetic, 4-fluorobenzoic, 4-fluorophenyl acetic, 3-Phenylpropionic, tetrahedro-2H-pyran-4carboxylic, succinic acid glutaric acid or bile acids. One having skill is the art will appreciate that a series of conjugates can be linked, e.g., for example PEG4, isoglu and combinations thereof. One having skill is the art will appreciate that an amino acid with the peptide can be isosterically replaced, for example, Lys may be replaced for Dap, Dab, α-MeLys orOrn. Examples of modified residues within a peptide are shown in Table 12.

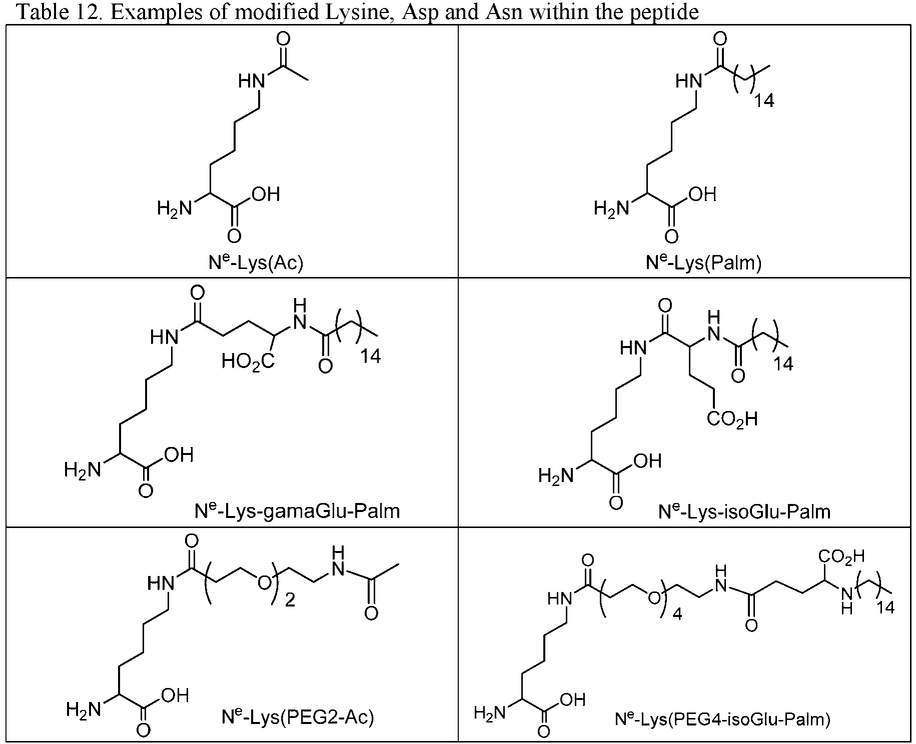

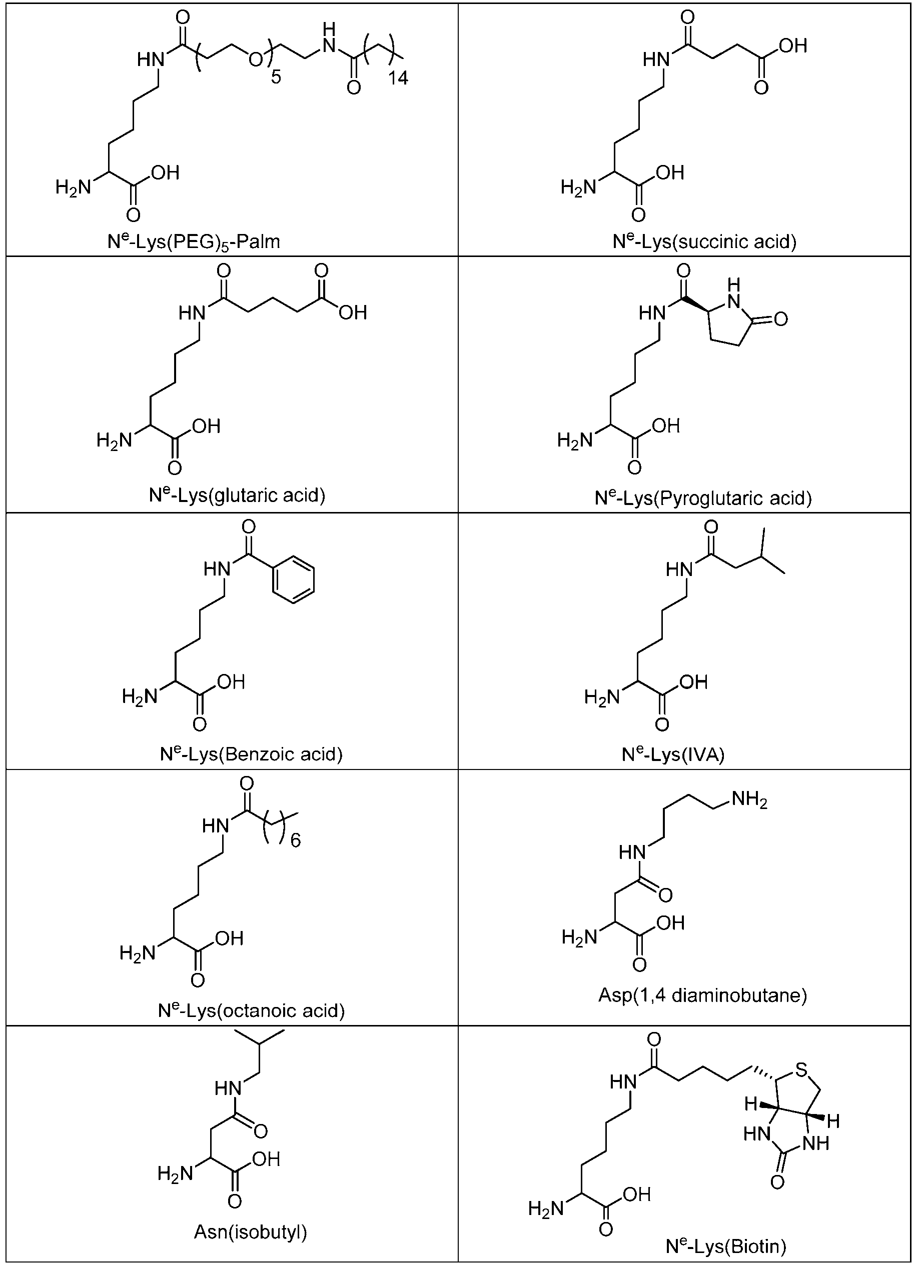

In further embodiments of the present invention, alternatively or additionally, a side-chain of one or more amino acid residues in a peptide inhibitor of the invention is conjugated to a polymeric moiety, for example, in order to increase solubility and/or half-life *in vivo* (e.g. in plasma) and/or bioavailability. Such modifications are also known to reduce clearance (e.g. renal clearance) of therapeutic proteins and peptides.

As used herein, "Polyethylene glycol" or "PEG" is a polyether compound of general Formula H-(O-CH2-CH2)n-OH. PEGs are also known as polyethylene oxides (PEOs) or polyoxyethylenes (POEs), depending on their molecular weight PEO, PEE, or POG, as used herein, refers to an oligomer or polymer of ethylene oxide. The three names are chemically synonymous, but PEG has tended to refer to oligomers and polymers with a molecular mass below 20,000 Da, PEO to polymers with a molecular mass above 20,000 Da, and POE to a polymer of any molecular mass. PEG and PEO are liquids or low-melting solids, depending on their molecular weights. Throughout this disclosure, the 3 names are used indistinguishably. PEGs are prepared by polymerization of ethylene oxide and are commercially available over a wide range of molecular weights from 300 Da to 10,000,000 Da. While PEG and PEO with different molecular weights find use in different applications, and have different physical properties (e.g. viscosity) due to chain length effects, their chemical properties are nearly identical. The polymeric moiety is preferably water-soluble (amphiphilic or hydrophilic), non-toxic, and pharmaceutically inert. Suitable polymeric moieties include polyethylene glycols (PEG), homo- or co-polymers of PEG, a monomethyl-substituted polymer of PEG (mPEG), or polyoxyethylene glycerol (POG). See, for example, Int. J. Hematology 68:1 (1998): Bioconjugate Chem. 6:150 (1995); and Crit. Rev. Therap. Drug Carrier Sys. 9:249 (1992). Also encompassed are PEGs that are prepared for purpose of half life extension, for example, mono-activated, alkoxy-terminated polyalkylene oxides (POA's) such as mono-methoxy-terminated polyethyelene glycols (mPEG's); bis activated polyethylene oxides (glycols) or other PEG derivatives are also contemplated. Suitable polymers will vary substantially by weights ranging from about 200 Da to about 40,000 Da or from about 200 Da to about 60,000 Da are usually selected for the purposes of the present invention. In certain embodiments, PEGs having molecular weights from 200 to 2,000 or from 200 to 500 are used. Different forms of PEG may also be used, depending on the initiator used for the polymerization process - a common common initiator is a monofunctional methyl ether PEG, or methoxypoly(ethylene glycol), abbreviated mPEG.

Lower-molecular-weight PEGs are also available as pure oligomers, referred to as monodisperse, uniform, or discrete. These are used in certain embodiments of the present invention.

PEGs are also available with different geometries: branched PEGs have three to ten PEG chains emanating from a central core group; star PEGs have 10 to 100 PEG chains emanating from a central core group; and comb PEGs have multiple PEG chains normally grafted onto a polymer backbone. PEGs can also be linear. The numbers that are often included in the names of PEGs indicate their average molecular weights (e.g. a PEG with n = 9 would have an average molecular weight of approximately 400 daltons, and would be labeled PEG 400.

As used herein, "PEGylation" is the act of covalently coupling a PEG structure to the peptide inhibitor of the invention, which is then referred to as a "PEGylated peptide inhibitor". In certain embodiments, the PEG of the PEGylated side chain is a PEG with a molecular weight from about 200 to about 40,000. In some embodiments, a spacer of a peptide of Formula I, Formula I', or Formula I" is PEGylated. In certain embodiments, the PEG of a PEGylated spacer is PEG3, PEG4, PEG5, PEG6, PEG7, PEG8, PEG9, PEG10, or PEG11. In certain embodiments, the PEG of a PEGylated spacer is PEG3 or PEG8.

Other suitable polymeric moieties include poly-amino acids such as poly-lysine, poly-aspartic acid and poly-glutamic acid (see for example Gombotz, et al. (1995), Bioconjugate Chem., vol. 6: 332-351; Hudecz, et al. (1992), Bioconjugate Chem., vol. 3, 49-57 and Tsukada, et al. (1984), J. Natl. Cancer Inst., vol. 73, : 721-729. The polymeric moiety may be straight-chain or branched. In some embodiments, it has a molecular weight of 500-40,000 Da, for example 500-10,000 Da, 1000-5000 Da, 10,000-20,000 Da, or 20,000-40,000 Da.

In some embodiments, a peptide inhibitor of the invention may comprise two or more such polymeric moieties, in which case the total molecular weight of all such moieties will generally fall within the ranges provided above.

In some embodiments, the polymeric moiety is coupled (by covalent linkage) to an amino, carboxyl or thiol group of an amino acid side chain. Certain examples are the thiol group of Cys residues and the epsilon amino group of Lys residues, and the carboxyl groups of Asp and Glu residues may also be involved.

The skilled worker will be well aware of suitable techniques which can be used to perform the coupling reaction. For example, a PEG moiety bearing a methoxy group can be coupled to a Cys thiol group by a maleimido linkage using reagents commercially available from Nektar Therapeutics AL. See also WO 2008/101017, and the references cited above, for details of suitable chemistry. A maleimide-functionalised PEG may also be conjugated to the side-chain sulfhydryl group of a Cys residue.

As used herein, disulfide bond oxidation can occur within a single step or is a two step process. As used herein, for a single oxidation step, the trityl protecting group is often employed during assembly, allowing deprotection during cleavage, followed by solution oxidation. When a second disulfide bond is required, one has the option of native or selective oxidation. For selective oxidation requiring orthogonal protecting groups, Acm and Trityl is used as the protecting groups for cysteine. Cleavage results in the removal of one protecting pair of cysteine allowing oxidation of this pair. The second oxidative deprotection step of the cysteine protected Acm group is then performed. For native oxidation, the trityl protecting group is used for all cysteines, allowing for natural folding of the peptide. A skilled worker will be well aware of suitable techniques which can be used to perform the oxidation step.

Several chemical moieties, including poly(ethylene)glycol, react with functional groups present in the twenty naturally occurring amino acids, such as, for example, the epsilon amino group in lysine amino acid residues, the thiol present in cysteine amino acid residues, or other nucleophilic amino acid side chains. When multiple naturally occurring amino acids react in a peptide inhibitor, these non-specific chemical reactions result in a final peptide inhibitor that contains many isomers of peptides conjugated to one or more poly(ethylene)glycol strands at different locations within the peptide inhibitor.

One advantage of certain embodiments of the present invention includes the ability to add one or more chemical moiety (such as PEG) by incorporating one or more non-natural amino acid(s) that possess unique functional groups that react with an activated PEG by way of chemistry that is unreactive with the naturally occurring amino acids present in the peptide inhibitor. For example, azide and alkyne groups are unreactive with all naturally occurring functional groups in a protein. Thus, a non-natural amino acid may be incorporated in one or more specific sites in a peptide inhibitor where PEG or another modification is desired without the undesirable non-specific reactions. In certain embodiments, the particular chemistry involved in the reaction results in a stable, covalent link between the PEG strand and the peptide inhibitor. In addition, such reactions may be performed in mild aqueous conditions that are not damaging to most peptides. In certain embodiments, the non-natural amino acid residue is AHA.

Chemical moieties attached to natural amino acids are limited in number and scope. By contrast, chemical moieties attached to non-natural amino acids can utilize a significantly greater spectrum of useful chemistries by which to attach the chemical moiety to the target molecule. Essentially any target molecule, including any protein (or portion thereof) that includes a non-natural amino acid, e.g., a non-natural amino acid containing a reactive site or side chain where a chemical moiety may attach, such as an aldehyde- or keto-derivatized amino acid, can serve as a substrate for attaching a chemical moiety.

Numerous chemical moieties may be joined or linked to a particular molecule through various known methods in the art. A variety of such methods are described in U.S. Patent No. 8,568,706. As an illustrative example, azide moieties may be useful in conjugating chemical moieties such as PEG or others described herein. The azide moiety serves as a reactive functional group, and is absent in most naturally occurring compounds (thus it is unreactive with the native amino acids of naturally occurring compounds). Azides also undergo a selective ligation with a limited number of reaction partners, and azides are small and can be introduced to biological samples without altering the molecular size of significantly. One reaction that allows incorporation or introduction of azides to molecules is the copper-mediated Huisgen [3+2] cycloaddition of an azide. This reaction can be used for the selective PEGylation of peptide inhibitors. (Tomoe et al., J. Org. Chem. 67: 3057, 2002; Rostovtsev et al., Angew. Chem., Int. Ed. 41: 596, 2002; and Wang et al., J. Am. Chem. Soc. 125: 3192, 2003, Speers et al., J. Am. Chem. Soc., 2003, 125, 4686).

### Synthesis of Peptide Inhibitors

The peptide inhibitors of the present invention may be synthesized by many techniques that are known to those skilled in the art. In certain embodiments, monomer subunits are synthesized, purified, and dimerized using the techniques described in the accompanying Examples. In certain embodiments, the present invention provides a method of producing a peptide inhibitor (or monomer subunit thereof) of the present invention, comprising chemically synthesizing a peptide comprising, consisting of, or consisting essentially of a peptide having an amino acid sequence described herein, including but not limited to any of the amino acid sequences set forth in any of Formulas I, II or tables herein. In other embodiments, the peptide is recombinantly synthesized, instead of being chemically synthesized. In certain embodiments, the peptide inhibitor is a dimer, and the method comprises synthezing both monomer subunits of the peptide dimer inhibitor and then dimerizing the two monomer subunits to produce the peptide dimer inhibitor. In various embodiments, dimerization is accomplished via any of the various methods described herein. In particular embodiments, methods of producing a peptide inhibitor (or monomer subunit thereof) further comprise cyclizing the peptide inhibitor (or monomer subunit thereof) after its synthesis. In particular embodiments, cyclization is accomplished via any of the various methods described herein. In certain embodiments, the present invention provides a method of producing a peptide inhibitor (or monomer subunit thereof) of the present invention, comprising introducing an intramolecular bond, e.g., a disulfide, an amide, or a thioether bond between two amino acids residues within a peptide comprising, consisting of, or consisting essentially of a peptide having an amino acid sequence described herein, including but not limited to any of the amino acid sequences set forth in any of Formulas (I), (II), (III), (IV) or the accompanying Examples or Tables.

In related embodiments, the present invention includes polynucleotides that encode a polypeptide having a sequence set forth in any one of Formulas (I)-(IV), or the accompanying Examples or Table.

In addition, the present invention includes vectors, e.g., expression vectors, comprising a polynucleotide of the present invention.

### Methods of Treatment

In certain embodiments, the present invention includes methods of inhibiting IL-23 binding to an IL-23R on a cell, comprising contacting the IL-23 with a peptide inhibitor of the present invention. In certain embodiments, the cell is a mammalian cell. In particular embodiments, the method is performed in vitro or in vivo. Inhibition of binding may be determined by a variety of routine experimental methods and assays known in the art.

In certain embodiments, the present invention includes methods of inhibiting IL-23 signaling by a cell, comprising contacting the IL-23 with a peptide inhibitor of the present invention. In certain embodiments, the cell is a mammalian cell. In particular embodiments, the method is performed in vitro or in vivo. In particular embodiments, the inhibition of IL-23 signalling may be determined by measuring changes in phospho-STAT3 levels in the cell.

In some embodiments, the present invention provides methods for treating a subject afflicted with a condition or indication associated with IL-21 or IL-23R (e.g., activation of the IL-23/IL-23R signaling pathway), wherein the method comprises administering to the subject a peptide inhibitor of the present invention. In one embodiment, a method is provided for treating a subject afflicted with a condition or indication characterized by inappropriate, deregulated, or increased IL-23 or IL-23R activity or signaling, comprising administering to the individual a peptide inhibitor of the present invention in an amount sufficient to inhibit (partially or fully) binding of IL-23 to IL-23R in the subject. In particular embodiments, the inhibition of IL-23 binding to IL-23R occurs in particular organs or tissues of the subject, e.g., the stomach, small intestine, large intestine/colon, intestinal mucosa, lamina propria, Peyer's Patches, mesenteric lymph nodes, or lymphatic ducts.

In some embodiments, methods of the present invention comprise providing a peptide inhibitor of the present invention to a subject in need thereof. In particular embodiments, the subject in need thereof has been diagnosed with or has been determined to be at risk of developing a disease or disorder associated with IL-23/IL-23R. In particular embodiments, the subject is a mammal.

In certain embodiments, the disease or disorder is autoimmune inflammation and related diseases and disorders, such as multiple sclerosis, asthma, rheumatoid arthritis, inflammatory bowel diseases (IBDs), juvenile IBD, adolescent IBD, Crohn's disease, sarcoidosis, Systemic Lupus Erythematosus, ankylosing spondylitis (axial spondyloarthritis), psoriatic arthritis, or psoriasis. In particular embodiments, the disease or disorder is psoriasis (e.g., plaque psoriasis, guttate psoriasis, inverse psoriasis, pustular psoriasis, Palmo-Plantar Pustulosis, psoriasis vulgaris, or erythrodermic psoriasis), atopic dermatitis, acne ectopica, ulcerative colitis, Crohn's disease, Celiac disease (nontropical Sprue), enteropathy associated with seronegative arthropathies, microscopic colitis, collagenous colitis, eosinophilic gastroenteritis/esophagitis, colitis associated with radio- or chemo-therapy, colitis associated with disorders of innate immunity as in leukocyte adhesion deficiency-1, chronic granulomatous disease, glycogen storage disease type 1b, Hermansky-Pudlak syndrome, Chediak-Higashi syndrome, Wiskott-Aldrich Syndrome, pouchitis resulting after proctocolectomy and ileoanal anastomosis, gastrointestinal cancer, pancreatitis, insulin-dependent diabetes mellitus, mastitis, cholecystitis, cholangitis, primary biliary cirrhosis, viral-associated enteropathy, pericholangitis, chronic bronchitis, chronic sinusitis, asthma, uveitis, or graft versus host disease.

In certain related embodiments, the present invention provides a method of selectively inhibiting IL-23 or IL-23R signaling (or the binding of IL-23 to IL-23R) in a subject in need thereof, comprising providing to the subject a peptide inhibitor of the present invention. In particular embodiments, the present invention includes a method of selectively inhibiting IL-23 or IL-23R signaling (or the binding of IL-23 to IL-23R) in the GI tract of a subject in need thereof, comprising providing to the subject a peptide inhibitor of the present invention by oral administration. In particular embodiments, exposure of the administered peptide inhibitor in GI tissues (e.g., small intestine or colon) is at least 10-fold, at least 20-fold, at least 50-fold, or at least 100-fold greater than the exposure in the blood. In particular embodiments, the present invention includes a method of selectively inhibiting IL23 or IL23R signaling (or the binding of IL23 to IL23R) in the GI tract of a subject in need thereof, comprising providing to the subject a peptide inhibitor, wherein the peptide inhibitor does not block the interaction between IL-6 and IL-6R or antagonize the IL-12 signaling pathway. In a further related embodiment, the present invention includes a method of inhibiting GI inflammation and/or neutrophil infiltration to the GI, comprising providing to a subject in need thereof a peptide inhibitor of the present invention.In some embodiments, methods of the present invention comprise providing a peptide inhibitor of the present invention (i.e., a first therapeutic agent) to a subject in need thereof in combination with a second therapeutic agent. In certain embodiments, the second therapeutic agent is provided to the subject before and/or simultaneously with and/or after the peptide inhibitor is administered to the subject. In particular embodiments, the second therapeutic agent is an anti-inflammatory agent. In certain embodiments, the second therapeutic agent is a non-steroidal anti-inflammatory drug, steroid, or immune modulating agent. In another embodiment, the method comprises administering to the subject a third therapeutic agent. In certain embodiments, the second therapeutic agent is an antibody that binds IL-23 or IL-23R.

In particular embodiments, the peptide inhibitor, or the pharmaceutical composition comprising a peptide inhibitor, is suspended in a sustained-release matrix. A sustained-release matrix, as used herein, is a matrix made of materials, usually polymers, which are degradable by enzymatic or acid-base hydrolysis or by dissolution. Once inserted into the body, the matrix is acted upon by enzymes and body fluids. A sustained-release matrix desirably is chosen from biocompatible materials such as liposomes, polylactides (polylactic acid), polyglycolide (polymer of glycolic acid), polylactide co-glycolide (copolymers of lactic acid and glycolic acid) polyanhydrides, poly(ortho)esters, polypeptides, hyaluronic acid, collagen, chondroitin sulfate, carboxylic acids, fatty acids, phospholipids, polysaccharides, nucleic acids, polyamino acids, amino acids such as phenylalanine, tyrosine, isoleucine, polynucleotides, polyvinyl propylene, polyvinylpyrrolidone and silicone. One embodiment of a biodegradable matrix is a matrix of one of either polylactide, polyglycolide, or polylactide co-glycolide (co-polymers of lactic acid and glycolic acid).

In certain embodiments, the present invention includes pharmacetical compositions comprising one or more peptide inhibitors of the present invention and a pharmaceutically acceptable carrier, diluent or excipient. A pharmaceutically acceptable carrier, diluent or excipient refers to a non-toxic solid, semi-solid or liquid filler, diluent, encapsulating material or Formulation auxiliary of any type. Prevention of the action of microorganisms may be ensured by the inclusion of various antibacterial and antifungal agents, for example, paraben, chlorobutanol, phenol sorbic acid, and the like. It may also be desirable to include isotonic agents such as sugars, sodium chloride, and the like.

In certain embodiments, the compositions are administered orally, parenterally, intracisternally, intravaginally, intraperitoneally, intrarectally, topically (as by powders, ointments, drops, suppository, or transdermal patch), by inhalation (such as intranasal spray), ocularly (such as intraocularly) or buccally. The term "parenteral" as used herein refers to modes of administration which include intravenous, intramuscular, intraperitoneal, intrasternal, subcutaneous, intradermal and intraarticular injection and infusion. Accordingly, in certain embodiments, the compositions are Formulated for delivery by any of these routes of administration.

In certain embodiments, pharmaceutical compositions for parenteral injection comprise pharmaceutically acceptable sterile aqueous or nonaqueous solutions, dispersions, suspensions or emulsions, or sterile powders, for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), carboxymethylcellulose and suitable mixtures thereof, β-cyclodextrin, vegetable oils (such as olive oil), and injectable organic esters such as ethyl oleate. Proper fluidity may be maintained, for example, by the use of coating materials such as lecithin, by the maintenance of the required particle size in the case of dispersions, and by the use of surfactants. Thes compositions may also contain adjuvants such as preservative, wetting agents, emulsifying agents, and dispersing agents. Prolonged absorption of an injectable pharmaceutical form may be brought about by the inclusion of agents which delay absorption, such as aluminum monostearate and gelatin.

Injectable depot forms include those made by forming microencapsule matrices of the peptide inhibitor in one or more biodegradable polymers such as polylactide-polyglycolide, poly(orthoesters), poly(anhydrides), and (poly)glycols, such as PEG. Depending upon the ratio of peptide to polymer and the nature of the particular polymer employed, the rate of release of the peptide inhibitor can be controlled. Depot injectable Formulations are also prepared by entrapping the peptide inhibitor in liposomes or microemulsions compatible with body tissues.

The injectable Formulations may be sterilized, for example, by filtration through a bacterial-retaining filter, or by incorporating sterilizing agents in the form of sterile solid compositions which can be dissolved or dispersed in sterile water or other sterile injectable medium just prior to use.

Topical administration includes administration to the skin or mucosa, including surfaces of the lung and eye. Compositions for topical lung administration, including those for inhalation and intranasal, may involve solutions and suspensions in aqueous and non-aqueous Formulations and can be prepared as a dry powder which may be pressurized or non-pressurized. In non-pressurized powder compositions, the active ingredientmay be finely divided form may be used in admixture with a larger-sized pharmaceutically acceptable inert carrier comprising particles having a size, for example, of up to 100 micrometers in diameter. Suitable inert carriers include sugars such as lactose.

Alternatively, the composition may be pressurized and contain a compressed gas, such as nitrogen or a liquefied gas propellant. The liquefied propellant medium and indeed the total composition may bey such that the active ingredient does not dissolve therein to any substantial extent. The pressurized composition may also contain a surface active agent, such as a liquid or solid non-ionic surface active agent or may be a solid anionic surface active agent. It is preferred to use the solid anionic surface active agent in the form of a sodium salt.

A further form of topical administration is to the eye. A peptide inhibitor of the invention may be delivered in a pharmaceutically acceptable ophthalmic vehicle, such that the peptide inhibitor is maintained in contact with the ocular surface for a sufficient time period to allow the peptide inhibitor to penetrate the corneal and internal regions of the eye, as for example the anterior chamber, posterior chamber, vitreous body, aqueous humor, vitreous humor, cornea, iris/ciliary, lens, choroid/retina and sclera. The pharmaceutically acceptable ophthalmic vehicle may, for example, be an ointment, vegetable oil or an encapsulating material. Alternatively, the peptide inhibitors of the invention may be injected directly into the vitreous and aqueous humour.

Compositions for rectal or vaginal administration include suppositories which may be prepared by mixing the peptide inhibitorss of this invention with suitable non-irritating excipients or carriers such as cocoa butter, polyethylene glycol or a suppository wax, which are solid at room temperature but liquid at body temperature and, therefore, melt in the rectum or vaginal cavity and release the active compound.

Peptide inhibitors of the present invention may also be administered in liposomes or other lipid-based carriers. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes can be used. The present compositions in liposome form can contain, in addition to a peptide inhibitor of the present invention, stabilizers, preservatives, excipients, and the like. In certain embodiments, the lipids comprise phospholipids, including the phosphatidyl cholines (lecithins) and serines, both natural and synthetic. Methods to form liposomes are known in the art.

Pharmaceutical compositions to be used in the invention suitable for parenteral administration may comprise sterile aqueous solutions and/or suspensions of the peptide inhibitos made isotonic with the blood of the recipient, generally using sodium chloride, glycerin, glucose, mannitol, sorbitol, and the like.

In some aspects, the invention provides a pharmaceutical composition for oral delivery. Compositions and peptide inhibitors of the instant invention may be prepared for oral administration according to any of the methods, techniques, and/or delivery vehicles described herein. Further, one having skill in the art will appreciate that the peptide inhibitors of the instant invention may be modified or integrated into a system or delivery vehicle that is not disclosed herein, yet is well known in the art and compatible for use in oral delivery of peptides.

In certain embodiments, Formulations for oral administration may comprise adjuvants (e.g. resorcinols and/or nonionic surfactants such as polyoxyethylene oleyl ether and n-hexadecylpolyethylene ether) to artificially increase the permeability of the intestinal walls, and/or enzymatic inhibitors (e.g. pancreatic trypsin inhibitors, diisopropylfluorophosphate (DFF) or trasylol) to inhibit enzymatic degradation. In certain embodiments, the peptide inhibitor of a solid-type dosage form for oral administration can be mixed with at least one additive, such as sucrose, lactose, cellulose, mannitol, trehalose, raffinose, maltitol, dextran, starches, agar, alginates, chitins, chitosans, pectins, gum tragacanth, gum arabic, gelatin, collagen, casein, albumin, synthetic or semisynthetic polymer, or glyceride. These dosage forms can also contain other type(s) of additives, e.g., inactive diluting agent, lubricant such as magnesium stearate, paraben, preserving agent such as sorbic acid, ascorbic acid, alpha-tocopherol, antioxidants such as cysteine, disintegrators, binders, thickeners, buffering agents, pH adjusting agents, sweetening agents, flavoring agents or perfuming agents.

In particular embodiments, oral dosage forms or unit doses compatible for use with the peptide inhibitors of the present invention may include a mixture of peptide inhibitor and nondrug components or excipients, as well as other non-reusable materials that may be considered either as an ingredient or packaging. Oral compositions may include at least one of a liquid, a solid, and a semi-solid dosage forms. In some embodiments, an oral dosage form is provided comprising an effective amount of peptide inhibitor, wherein the dosage form comprises at least one of a pill, a tablet, a capsule, a gel, a paste, a drink, a syrup, ointment, and suppository. In some instances, an oral dosage form is provided that is designed and configured to achieve delayed release of the peptide inhibitor in the subject's small intestine and/or colon.

In one embodiment, an oral pharmaceutical composition comprising a peptide inhibitor of the present invention comprises an enteric coating that is designed to delay release of the peptide inhibitor in the small intestine. In at least some embodiments, a pharmaceutical composition is provided which comprises a peptide inhibitor of the present invention and a protease inhibitor, such as aprotinin, in a delayed release pharmaceutical Formulation. In some instances, pharmaceutical compositions of the instant invention comprise an enteric coat that is soluble in gastric juice at a pH of about 5.0 or higher. In at least one embodiment, a pharmaceutical composition is provided comprising an enteric coating comprising a polymer having dissociable carboxylic groups, such as derivatives of cellulose, including hydroxypropylmethyl cellulose phthalate, cellulose acetate phthalate and cellulose acetate trimellitate and similar derivatives of cellulose and other carbohydrate polymers.

In one embodiment, a pharmaceutical composition comprising a peptide inhibitor of the present invention is provided in an enteric coating, the enteric coating being designed to protect and release the pharmaceutical composition in a controlled manner within the subject's lower gastrointestinal system, and to avoid systemic side effects. In addition to enteric coatings, the peptide inhibitors of the instant invention may be encapsulated, coated, engaged or otherwise associated within any compatible oral drug delivery system or component. For example, in some embodiments a peptide inhibitor of the present invention is provided in a lipid carrier system comprising at least one of polymeric hydrogels, nanoparticles, microspheres, micelles, and other lipid systems.

To overcome peptide degradation in the small intestine, some embodiments of the present invention comprise a hydrogel polymer carrier system in which a peptide inhibitor of the present invention is contained, whereby the hydrogel polymer protects the peptide inhibitor from proteolysis in the small intestine and/or colon. The peptide inhibitors of the present invention may further be Formulated for compatible use with a carrier system that is designed to increase the dissolution kinetics and enhance intestinal absorption of the peptide. These methods include the use of liposomes, micelles and nanoparticles to increase GI tract permeation of peptides.

Various bioresponsive systems may also be combined with one or more peptide inhibitor of the present invention to provide a pharmaceutical agent for oral delivery. In some embodiments, a peptide inhibitor of the instant invention is used in combination with a bioresponsive system, such as hydrogels and mucoadhesive polymers with hydrogen bonding groups (e.g., PEG, poly(methacrylic) acid [PMAA], cellulose, Eudragit^{®}, chitosan and alginate) to provide a therapeutic agent for oral administration. Other embodiments include a method for optimizing or prolonging drug residence time for a peptide inhibitor disclosed herein, wherein the surface of the peptide inhibitor surface is modified to comprise mucoadhesive properties through hydrogen bonds, polymers with linked mucins or/and hydrophobic interactions. These modified peptide molecules may demonstrate increase drug residence time within the subject, in accordance with a desired feature of the invention. Moreover, targeted mucoadhesive systems may specifically bind to receptors at the enterocytes and M-cell surfaces, thereby further increasing the uptake of particles containing the peptide inhibitor.

Other embodiments comprise a method for oral delivery of a peptide inhibitor of the present invention, wherein the peptide inhibitor is provided to a subject in combination with permeation enhancers that promote the transport of the peptides across the intestinal mucosa by increasing paracellular or transcellular permeation. Various permeation enhancers and methods for the oral delivery of therapeutic agents is described in Brayden, D.J., Mrsny, R.J., 2011. Oral peptide delivery: prioritizing the leading technologies. Ther. Delivery 2 (12), 1567-1573.

In certain embodiments, pharmaceutical compositions and Formulations of the present invention comprises a peptide inhibitor of the present invention and one or more permeation enhancer. Examples of absorption enhancers may include Bile salts, fatty acids, surfactants (anionic, cationic, and nonanionic) chelators, Zonular OT, esters, cyclodextrin, dextran sulfate, azone, crown ethers, EDTA, sucrose esters, and phosphotidyl choline, for example. Although absorption enhancers are not typically carriers by themselves, they are also widely associated with other carriers to improve oral bioavailability by transporting of peptides and proteins across the intestinal mucosa. Such substances can be added to the Formulation as excipients or incorporated to form non specific interactions with the intended peptide inhibitor.

Dietary components and/or other naturally occurring substances affirmed as enhancing tight junction permeation and as Generally Recognized As Safe (GRAS) include, e.g., asglycerides, acylcarnitines, bile salts, and medium chain fatty acids. Sodium salts of medium chain fatty acids (MCFAS) were also suggested to be permeation enhancers. The most extensively studied MCFAS is sodium caprate, a salt of capric acid, which comprises 2-3% of the fatty acids in the milk fat fraction. To date, sodium caprate is mainly used as an excipient in a suppository Formulation (Doktacillin^{™}) for improving rectal ampicillin absorption. The permeation properties of another dietary MCFAS, sodium caprylate (8-carbon), were shown in vitro to be lower when compared to sodium caprate. Sodium caprylate and a peptidic drug were Formulated in an admixture with other excipients in oil to generate an oily suspension (OS) that enhanced permeability (Tuvia, S. et al., Pharmaceutical Research, Vol. 31, No. 8, pp. 2010-2021 (2014).

For example, in one embodiment, a permeation enhancer is combined with a peptide inhibitor, wherein the permeation enhancer comprises at least one of a medium-chain fatty acid, a long-chain fatty acid, a bile salt, an amphiphilic surfactant, and a chelating agent. In certain embodiments, medium-chain fatty acid salts promote absorption by increasing paracellular permeability of the intestinal epithelium. In one embodiment, a permeation enhancer comprising sodium N-[hydroxybenzoyl)amino] caprylate is used to form a weak noncovalent association with the peptide inhibitor of the instant invention, wherein the permeation enhancer favors membrane transport and further dissociation once reaching the blood circulation. In another embodiment, a peptide inhibitor of the present invention is conjugated to oligoarginine, thereby increasing cellular penetration of the peptide into various cell types. Further, in at least one embodiment a noncovalent bond is provided between a peptide inhibibitor of the present invention and a permeation enhancer selected from the group consisting of a cyclodextrin (CD) and a dendrimers, wherein the permeation enhancer reduces peptide aggregation and increasing stability and solubility for the peptide inhibitor molecule.

In certain embodiments, a pharmaceutical composition or Formulation comprises a peptide inhibitor of the present invention and a transient permeability enhancers (TPEs). Permeation enhancers and TPEs may be used to increase orally bioavailability or the peptide inhibitor. One example of a TPE that may be used is an oily suspension Formulation that disperses a powder containing sodioum caprylate and a therapeutic agent (Tuvia, S. et al., Pharmaceutical Research, Vol. 31, No. 8, pp. 2010-2021 (2014).

In certain embodiments, pharmaceutical composition and Formulations may include a peptide inhibitor of the present invention and one or more absorption enhancers, enzyme inhibitors, or mucoso adhesive polymers.

In particular embodiments, peptide inhibors of the present invention are Formulated in a Formulation vehicle, such as, e.g., emulsions, liposomes, microsphere or nanoparticles.

Other embodiments of the invention provide a method for treating a subject with a peptide inhibitor of the present invention having an increased half-life. In one aspect, the present invention provides a peptide inhibitor having a half-life of at least several hours to one day *in vitro* or *in vivo* (e.g., when administered to a human subject) sufficient for daily (q.d.) or twice daily (b.i.d.) dosing of a therapeutically effective amount. In another embodiment, the peptide inhibitor has a half-life of three days or longer sufficient for weekly (q.w.) dosing of a therapeutically effective amount. Further, in another embodiment, the peptide inhibitor has a half-life of eight days or longer sufficient for bi-weekly (b.i.w.) or monthly dosing of a therapeutically effective amount. In another embodiment, the peptide inhibitor is derivatized or modified such that is has a longer half-life as compared to the underivatized or unmodified peptide inhibitor. In another embodiment, the peptide inhibitor contains one or more chemical modifications to increase serum half-life.

When used in at least one of the treatments or delivery systems described herein, a peptide inhibitor of the present invention may be employed in pure form or, where such forms exist, in pharmaceutically acceptable salt form.

The total daily usage of the peptide inhibitors and compositions of the present invention can be decided by the attending physician within the scope of sound medical judgment. The specific therapeutically effective dose level for any particular subject will depend upon a variety of factors including: a) the disorder being treated and the severity of the disorder; b) activity of the specific compound employed; c) the specific composition employed, the age, body weight, general health, sex and diet of the patient; d) the time of administration, route of administration, and rate of excretion of the specific peptide inhibitor employed; e) the duration of the treatment; f) drugs used in combination or coincidental with the specific peptide inhibitor employed, and like factors well known in the medical arts.

In particlar embodiments, the total daily dose of the peptide inhibitors of the invention to be administered to a human or other mammal host in single or divided doses may be in amounts, for example, from 0.0001 to 300 mg/kg body weight daily or 1 to 300 mg/kg body weight daily.

### Non-invasive Detection of Intestinal Inflammation

The peptide inhibitors of the invention may be used for detection, assessment and diagnosis of intestinal inflammation by microPET imaging, wherein the peptide inhibitor is labeled with a chelating group or a detectable label, as part of a a non-invasive diagnostic procedure. In one embodiment, a peptide inhibitor is conjugated with a bifunctional chelator. In another embodiment, a peptide inhibitor is radiolabeled. The labeled peptide inhibitor is then administered to a subject orally or rectally. In one embodiment, the labeled peptide inhibitor is included in drinking water. Following uptake of the peptide inhibitor, microPET imaging may be used to visualize inflammation throughout the subject's bowels and digestive track.

### EXAMPLES

### EXAMPLE 1

### SYNTHESIS OF PEPTIDE MONOMERS

Peptide monomers of the present invention were synthesized using the Merrifield solid phase synthesis techniques on Protein Technology's Symphony multiple channel synthesizer. The peptides were assembled using HBTU (O-Benzotriazole-N,N,N',N'-tetramethyl-uronium-hexafluoro-phosphate), Diisopropylethylamine(DIEA) coupling conditions. For some amino acid couplings PyAOP(7-Azabenzotriazol-1-yloxy)tripyrrolidinophosponium hexafluorophosphate) and DIEA conditions were used. Rink Amide MBHA resin (100-200 mesh, 0.57 mmol/g) was used for peptide with C-terminal amides and pre-loaded Wang Resin with N-α-Fmoc protected amino acid was used for peptide with C-terminal acids. The coupling reagents (HBTU and DIEA premixed) were prepared at 100mmol concentration. Similarly amino acids solutions were prepared at 100 mmol concentration. Peptide inhibitors of the present invention were identified based on medical chemistry optimization and/or phage display and screened to identify those having superior binding and/or inhibitory properties.

### Assembly

The peptides were assembled using standard Symphony protocols. The peptide sequences were assembled as follows: Resin (250 mg, 0.14 mmol) in each reaction vial was washed twice with 4ml of DMF followed by treatment with 2.5ml of 20% 4-methyl piperidine (Fmoc de-protection) for 10min. The resin was then filtered and washed two times with DMF (4ml) and re-treated with N-methyl piperifine for additional 30 minute. The resin was again washed three times with DMF (4 ml) followed by addition 2.5ml of amino acid and 2.5ml of HBTU-DIEA mixture. After 45min of frequent agitations, the resin was filtered and washed three timed with DMF (4 ml each). For a typical peptide of the present invention, double couplings were performed. After completing the coupling reaction, the resin was washed three times with DMF (4 ml each) before proceeding to the next amino acid coupling.

### Ring Closing Metathesis to form Olefins

The resin (100 µmol) was washed with 2 ml of DCM (3 × 1 min) and then with 2 ml of DCE (3 × 1 min) before being treated with a solution of 2 ml of a 6 mM solution of Grubbs' first-generation catalyst in DCE (4.94 mg ml-1; 20 mol% with regard to the resin substitution). The solution was refluxed overnight (12 h) under nitrogenbefore being drained. The resin was washed three times with DMF (4 ml each); DCM (4 ml) before being dried and cleavaed.

### Cleavage

Following completion of the peptide assembly, the peptide was cleaved from the resin by treatment with cleavage reagent, such as reagent K (82.5% trigluoroacetic acid, 5% water, 5% thioanisole, 5% phenol, 2.5% 1,2-ethanedithiol). The cleavage reagent was able to successfully cleave the peptide from the resin, as well as all remaining side chain protecting groups.

The cleaved peptides were precipitated in cold diethyl ether followed by two washings with ethyl ether. The filtrate was poured off and a second aliquot of cold ether was added, and the procedure repeated. The crude peptide was dissolved in a solution of acetonitrile:water (7:3 with 1% TFA) and filtered. The quality of linear peptide was then verified using electrospray ionization mass spectrometry (ESI-MS) (Micromass/Waters ZQ) before being purified.

### Disulfide Bond Formation via Oxidation

The peptide containing the free thiol (for example diPen) was assembled on a Rink Amide-MBHA resin following general Fmoc-SPPS procedure. The peptide was cleaved from the resin by treatment with cleavage reagent 90% trifluoroacetic acid, 5% water, 2.5% 1,2-ethanedithiol, 2.5% tri-isopropylsilane). The cleaved peptides were precipitated in cold diethyl ether followed by two washings with ethyl ether. The filtrate was poured off and a second aliquot of cold ether was added, and the procedure repeated. The crude peptide was dissolved in a solution of acetonitrile:water (7:3 with 1% TFA) and filtered giving the wanted unoxidized peptide crude peptide

The crude, cleaved peptide with X4 and X9 possessing either Cys, Pen, hCys, (D)Pen, (D)Cys or (D)hCys, was dissolved in 20ml of water : acetonitrile. Saturated Iodine in acetic acid was then added drop wise with stirring until yellow color persisted. The solution was stirred for 15 minutes, and the reaction was monitored with analytic HPLC and LCMS. When the reaction was completed, solid ascorbic acid was added until the solution became clear. The solvent mixture was then purified by first being diluted with water and then loaded onto a reverse phase HPLC machine (Luna C18 support, 10u, 100A, Mobile phase A: water containing 0.1% TFA, mobile phase B: Acetonitrile (ACN) containing 0.1% TFA, gradient began with 5% B, and changed to 50% B over 60 minutes at a flow rate of 15ml/min). Fractions containing pure product were then freeze-dried on a lyophilyzer.

### Thioether Bond Formation

The peptide containing the free thiol (eg Cys) and hSer(OTBDMS) was assembled on a Rink Amide-MBHA resin following general Fmoc-SPPS procedure. Chlorination was carried out by treating the resin with PPh₃ (10 equiv.) and Cl₃CCN (10 equiv.) in DCM for 2 h. The peptide was cleaved from the resin by treatment with cleavage reagent 90% trifluoroacetic acid, 5% water, 2.5% 1,2-ethanedithiol, 2.5% tri-isopropylsilane). The cleaved peptides were precipitated in cold diethyl ether followed by two washings with ethyl ether. The filtrate was poured off and a second aliquot of cold ether was added, and the procedure repeated. The crude peptide was dissolved in a solution of acetonitrile: water (7:3 with 1% TFA) and filtered giving the wanted uncyclized crude peptide

The crude peptide possessing a free thiol (eg Cys, Pen, hCys, (D)Pen, (D)Cys or (D)hCys) and the alkyl halide (hSer(Cl)) at either the X4 and X9 position or X9 and X4 position was dissolved in 0.1 M TRIS buffer pH 8.5. Cyclization was allowed to take place overnight at RT. The solvent mixture was then purified by first being diluted two-fold with water and then loaded onto a reverse phase HPLC machine (Luna C18 support, 10u, 100A, Mobile phase A: water containing 0.1% TFA, mobile phase B: Acetonitrile (ACN) containing 0.1% TFA, gradient began with 5% B, and changed to 50% B over 60 minutes at a flow rate of 15ml/min). Fractions containing pure product were then freeze-dried on a lyophilyzer.

### Purification

Analytical reverse-phase, high performance liquid chromatography (HPLC) was performed on a Gemini C18 column (4.6 mm x 250 mm) (Phenomenex). Semi-Preparative reverse phase HPLC was performed on a Gemini 10 µm C18 column (22 mm x 250 mm) (Phenomenex) or Jupiter 10 µm, 300 A ° C18 column (21.2 mm x 250 mm) (Phenomenex). Separations were achieved using linear gradients of buffer B in A (Mobile phase A: water containing 0.15% TFA, mobile phase B: Acetonitrile (ACN) containing 0.1% TFA), at a flow rate of 1 mL/min (analytical) and 15 mL/min (preparative). Separations were achieved using linear gradients of buffer B in A (Mobile phase A: water containing 0.15% TFA, mobile phase B: Acetonitrile (ACN) containing 0.1% TFA), at a flow rate of 1 mL/min (analytical) and 15mL/min (preparative).

### EXAMPLE 2

### PEPTIDE INHIBITION OF BINDING OF INTERLEUKIN-23 TO THE INTERLEUKIN-23 RECEPTOR

Peptide optimization was performed to identify peptide inhibitors of IL-23 signalling that were active at low concentrations (e.g., IC50 <10 nM). Peptides were tested to identify peptides that inhibit the binding of IL-23 to human IL-23R and inhibit IL-23/IL-23R functional activity, as described below.

Assays were performed to determine peptide activity as described below, and the results of these assays are provided in Tables E1 and E2. Human ELISA indicates the IL23-IL23R competitive binding assay described below, Rat ELISA indicates the rat IL-23R competitive binding ELISA assay described below, and pStat3HTRF indicates the DB cells IL-23R pSTAT3 cell assay described below. The peptides depicted in Table E1 are cyclized via a disulfide bridge formed between two Pen residues in these peptides. The peptides depicted in Table E2 are cyclized via a thioether bond between the indicated amino acid residues. Table E2 provides an illustrative structure depicting thioether cyclization, which is indicated in the table by the term "cyclo," with the cyclic region bracketed immediately following the term "cyclo." For certain peptides, the residue Abu is present where indicated, whereas in other embodiments, e.g., those related to the non-cyclized form, the Abu may be referred to as a hSer(Cl) or homoSer residue.

### IL23-IL23R Competitive Binding ELISA

An Immulon^{®} 4HBX plate was coated with 50 ng/well of IL23R_huFC and incubated overnight at 4°C. The wells were washed four times with PBST, blocked with PBS containing 3% Skim Milk for 1 hour at room temperature, and washed again four times with PBST. Serial dilutions of test peptides and IL-23 at a final concentration of 2 nM diluted in Assay Buffer (PBS containing 1% Skim Milk) were added to each well, and incubated for 2 hours at room temperature. After the wells were washed, bound IL-23 was detected by incubation with 50 ng/well of goat anti-p40 polyclonal antibodies (R&D Systems #AF309) diluted in Assay Buffer for 1 hour at room temperature. The wells were again washed four times with PBST. The secondary antibodies, HRP conjugated donkey anti-goat IgG (Jackson ImmunoResearch Laboratories #705-035-147) diluted 1:5000 in Assay Buffer was then added, and incubated for 30 minutes at room temperature. The plate was finally washed as above. Signals were visualized with TMB One Component HRP Membrane Substrate, quenched with 2 M sulfuric acid and read spectrophotometrically at 450 nm. IC50 values for various test peptides determined from these data are shown in Tables E1 and E2.

### Rat IL-23R Competitive Binding ELISA

An assay plate was coated with 300 ng/well of Rat IL-23R_huFC and incubated overnight at 4°C. The wells were washed, blocked, and washed again. Serial dilutions of test peptides and IL-23 at a final concentration of 7 nM were added to each well, and incubated for 2 hours at room temperature. After the wells were washed, bound IL-23 was detected with goat anti-p40 polyclonal antibodies, followed by an HRP conjugated donkey anti-goat IgG. Signals were visualized with TMB One Component HRP Membrane Substrate and quenched with 2 M sulfuric acid. IC50 values for various test peptides determined from these data are shown in Tables E1 and E2.

### DB Cells IL23R pSTAT3 Cell Assay

IL-23 plays a central role in supporting and maintaining Th17 differentiation *in vivo.* This process is thought to mediated primarily through the Signal Transducer and Activator of Transcription 3 (STAT3), with phosphorylation of STAT3 (to yield pSTAT3) leading to upregulation of RORC and pro-inflammatory IL-17. This cell assay examines the levels of pSTAT3 in IL-23R-expressing DB cells when stimulated with IL-23 in the presence of test compounds. DB cells (ATCC #CRL-2289), cultured in RPMI-1640 medium (ATCC #30-2001) supplemented with 10% FBS and 1% Glutamine, were seeded at 5 X 10E5 cells/well in a 96 well tissue culture plate. Serial dilutions of test peptides and IL-23 at a final concentration of 0.5 nM were added to each well, and incubated for 30 minutes at 37°C in a 5% CO₂ humidified incubator. Changes in phospho-STAT3 levels in the cell lysates were detected using the Cisbio HTRF pSTAT3 Cellular Assay Kit, according to manufacturer's Two Plate Assay protocol. IC50 values determined from these data are shown in Tables E1, E2, and E3. * = ≤ 1 nM: ** = 1 nM - 10 nM; *** = 10 nM - 100 nM; **** = >100 nM. Where not shown, data was not yet determined.

**Table E1. IC₅₀s of Illustrative Peptides Containing the Ac-[Pen]-XXWX-[Pen]-XXXX Motif (SEQ ID NO:269) and Analogues**

| **SEQ ID No.** | **Sequence** | **pStat3 HTRF (nM)** |
|---|---|---|
| 1 | Ac-[(D)Arg]-[Pen]-QTWQ-[Pen]-[Phe[4-(2-aminoethoxy)]-[2-Nal]-[ 4- amino-4-carboxy-tetrahydropyran]-ENN-NH2 | ** |
| 2 | Ac-[Pen]-N-DWQ-[Pen]-[Phe[4-(2-aminoethoxy)]-[2-Nal]-[4-amino-4- carboxy-tetrahydropyran]-ENN-NH2 | *** |
| 3 | Ac-[Pen]-N-[Dab]-WQ-[Pen]-[Phe[4-(2-aminoethoxy)]-[2-Nal]-[ 4-amino- 4-carboxy-tetrahydropyran]-ENN-NH2 | ** |
| 4 | Ac-[Pen]-NT-[1-Nal]-Q-[Pen]-[Phe[4-(2-aminoethoxy)]-[2-Nal]-[4-amino- 4-carboxy-tetrahydropyran]-[Lys(Ac)]-NN-NH2 | *** |
| 5 | Ac-[Pen]-NT-[2-Nal]-Q-[Pen]-[Phe[4-(2-aminoethoxy)]-[2-Nal]- [4- amino-4-carboxy-tetrahydropyran]-[Lys(Ac)]-NN-NH2 | ** |
| 6 | Ac-[Pen]-NTWE-[Pen]-[Phe[4-(2-aminoethoxy)]-[2-Nal]-[ 4-amino-4- carboxy-tetrahydropyran]-[Lys(Ac)]-NN-NH2 | ** |
| 7 | Ac-[Pen]-NTWF-[Pen]-[Phe[4-(2-aminoethoxy)]-[2-Nal]-[ 4-amino-4- carboxy-tetrahydropyran]-[Lys(Ac)]-NN-NH2 | *** |
| 8 | Ac-[Pen]-NTWQ-[Pen]-[Phe[4-(2-aminoethoxy)]-W-[4-amino-4-carboxy- tetrahydropyran]- [Lys(Ac)]-NN-NH2 | ** |
| 9 | Ac-[Pen]-NT-[Trp(5-F)]-Q-[Pen]-[Phe[4-(2-aminoethoxy)]-W-[α-MeLeu]- [Lys(Ac)]-NN-NH2 | ** |

**Table E2. IC₅₀s of Illustrative Peptide Inhibitors (Thioethers)**

| 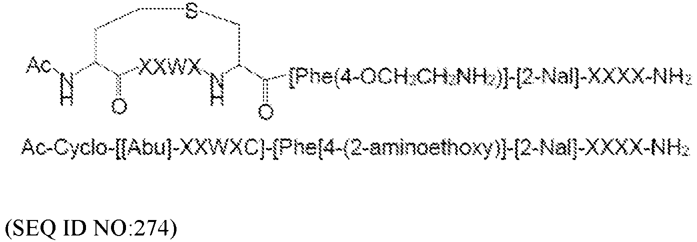 | | |
|---|---|---|
| **SEQ ID No.** | **Sequence** | **pStat3 HTRF (nM)** |
| 71 | Alexa488-[PEG4]-[(D)Arg]-cyclo[[Abu]-QTWQC]-[Phe[4-(2- aminoethoxy)]-[2-Nal]-[4-amino-4-carboxy-tetrahydropyran]- ENN-NH2 | * |
| 72 | Alexa647-[PEG4]-[(D)Arg]-cyclo[[Abu]-QTWQC]-[Phe[4-(2- aminoethoxy)]-[2-Nal]-[4-amino-4-carboxy-tetrahydropyran]- ENN-NH2 | ** |
| 73 | Ac-[(D)Arg]-cyclo[[Abu]-QTWQC]-[Phe[4-(2-aminoethoxy)]-[2- Nal]-[4-amino-4-carboxy-tetrahydropyran]-END-NH2 | ** |
| 74 | Ac-[(D)Arg]-cyclo[[Abu]-QTWQC]-[Phe[4-(2-aminoethoxy)]-[2- Nal]-[4-amino-4-carboxy-tetrahydropyran]-EDN-NH2 | *** |
| 75 | Ac-[(D)Arg]-cyclo[[Abu]-QTWEC]-[Phe[4-(2-aminoethoxy)]-[2- Nal]-[4-amino-4-carboxy-tetrahydropyran]-ENN-NH2 | ** |
| 76 | Ac-[(D)Arg]-cyclo[[Abu]-ETWQC]-[Phe[4-(2-aminoethoxy)]-[2- Nal]-[4-amino-4-carboxy-tetrahydropyran]-ENN-NH2 | ** |
| 77 | Ac-[(D)Arg]-cyclo[[Abu]-QTWQC]-[Phe[4-(2-aminoethoxy)]-[2- Nal]-[4-amino-4-carboxy-tetrahydropyran]-EDD-NH2 | *** |
| 78 | Ac-[(D)Arg]-cyclo[[Abu]-QTWEC]-[Phe[4-(2-aminoethoxy)]-[2- Nal]-[4-amino-4-carboxy-tetrahydropyran]-END-NH2 | ** |
| 79 | Ac-[(D)Arg]-cyclo[[Abu]-ETWQC]-[Phe[4-(2-aminoethoxy)]-[2- Nal]-[4-amino-4-carboxy-tetrahydropyran]-END-NH2 | ** |
| 80 | Ac-[(D)Arg]-cyclo[[Abu]-QTWEC]-[Phe[4-(aminoethoxy)]-[2- Nal]-[4-amino-4-carboxy-tetrahydropyran]-EDN-NH2 | *** |
| 81 | Ac-[(D)Arg]-cyclo[[Abu]-ETWQC]-[Phe[4-(2-aminoethoxy)]-[2- Nal]-[4-amino-4-carboxy-tetrahydropyran]-EDN-NH2 | *** |
| 82 | Ac-[(D)Arg]-cyclo[[Abu]-ETWEC]-[Phe[4-(2-aminoethoxy)]-[2- Nal]-[4-amino-4-carboxy-tetrahydropyran]-ENN-NH2 | ** |
| 83 | Ac-[(D)Arg]-cyclo[[Abu]-QTWQC]-[Phe[4-(2-aminoethoxy)]-[2- Nal]-[4-amino-4-carboxy-tetrahydropyran]-ENN-OH | ** |
| 84 | Ac-[(D)Arg]-cyclo[[Abu]-QTWQC]-[Phe[4-(2-aminoethoxy)]-[2- Nal]-[4-amino-4-carboxy-tetrahydropyran]-END-OH | *** |
| 85 | Ac-[(D)Arg]-cyclo[[Abu]-QTWQC]-[Phe[4-(2-aminoethoxy)]-[2- Nal]-[4-amino-4-carboxy-tetrahydropyran]-EDN-OH | *** |
| 86 | Ac-[(D)Arg]-cyclo[[Abu]-QTWEC]-[Phe[4-(2-aminoethoxy)]-[2- Nal]-[4-amino-4-carboxy-tetrahydropyran]-ENN-OH | ** |
| 87 | [Ac-[(D)Arg]-cyclo[[Abu]-ETWQC]-[Phe[4-(2-aminoethoxy)]-[2- Nal]-[4-amino-4-carboxy-tetrahydropyran]-ENN-OH | ** |
| 88 | [NH2-PEG4]-[(D)Arg]-cyclo[[Abu]-QTWQC]-[Phe[4-(2- aminoethoxy)]-[2-Nal]-[4-amino-4-carboxy-tetrahydropyran]- ENN-NH2 | ** |
| 89 | [Biotin]-[PEG4]-[(D)Arg]-cyclo[[Abu]-QTWQC]-[Phe[4-(2- aminoethoxy)]-[2-Nal]-[4-amino-4-carboxy-tetrahydropyran]- ENN-NH2 | * |
| 90 | [NH2-PEG4]-[(D)Arg]-cyclo[[Abu]-QTWQC]-[Phe[4-(2- aminoethoxy)-(PEG4-NH2)]-[2-Nal]-[4-amino-4-carboxy- tetrahydropyran]-ENN-NH2 | ** |
| 91 | [NH2-PEG4]-[(D)Arg]-cyclo[[Abu]-QTWQC]-[Phe[4-(2- aminoethoxy)-(PEG4)-(Biotin)]-[2-Nal]-[4-amino-4-carboxy- tetrahydropyran]-ENN-NH2 | ** |
| 92 | Ac-[(D)Arg]-cyclo[[Abu]-QTWQ-[Pen]]-[Phe[4-(2-aminoethoxy)]- [2-Nal]-[4-amino-4-carboxy-tetrahydropyran]-ENN-NH2 | *** |
| 93 | Ac-[(D)Arg]-cyclo[[Abu]-QTW-[α-MeLeu]-C]-[Phe[4-(2- aminoethoxy)]-[2-Nal]-[4-amino-4-carboxy-tetrahydropyran]- ENN-NH₂ | ** |
| 94 | Ac-[(D)Arg]-cyclo[[Abu]-QTW-[α-MeLys]-C]-[Phe[4-(2- aminoethoxy)]-[2-Nal]-[4-amino-4-carboxy-tetrahydropyran]- ENN-NH₂ | *** |
| 95 | Ac-[(D)Arg]-cyclo[[Abu]-QTW-[α-MeLys(Ac)]-C]-[Phe[4-(2- aminoethoxy)]-[2-Nal]-[4-amino-4-carboxy-tetrahydropyran]- ENN-NH2 | ** |
| 96 | Ac-[(D)Arg]-cyclo[[Abu]-QTW-[(D)Gln]-C]-[Phe[4-(2- aminoethoxy)]-[2-Nal]-[4-amino-4-carboxy-tetrahydropyran]- ENN-NH2 | *** |
| 97 | Ac-[(D)Arg]-cyclo[[Abu]-QTW-[β–homoGln]-C]-[Phe[4-(2- aminoethoxy)]-[2-Nal]-[4-amino-4-carboxy-tetrahydropyran]- ENN-NH2 | **** |
| 98 | Ac-[(D)Arg]-cyclo[[Abu]-QTW-[α-MeLys(Ac)]-C]-[Phe[4-(2- acetylaminoethoxy]-[2-Nal]-[4-amino-4-carboxy-tetrahydropyran]- ENN-NH2 | *** |
| 99 | Ac-[(D)Arg]-cyclo[[Abu]-QTWFC]-[Phe[4-(2-aminoethoxy)]-[2- Nal]-[4-amino-4-carboxy-tetrahydropyran]-ENN-NH2 | *** |
| 100 | Ac-[(D)Arg]-cyclo[[Abu]-QTWWC]-[Phe[4-(2-aminoethoxy)]-[2- Nal]-[4-amino-4-carboxy-tetrahydropyran]-ENN-NH2 | ** |
| 101 | Ac-[(D)Arg]-cyclo[[Abu]-QTW-[Aib]-C]-[Phe[4-(2- aminoethoxy)]-[2-Nal]-[4-amino-4-carboxy-tetrahydropyran]- ENN-NH2 | *** |
| 102 | Ac-[(D)Arg]-cyclo[[Abu]-QTWTC]-[Phe[4-(2-aminoethoxy)]-[2- Nal]-[4-amino-4-carboxy-tetrahydropyran]-ENN-NH2 | *** |
| 103 | Ac-[(D)Arg]-cyclo[[Abu]-QTWVC]-[Phe[4-(2-aminoethoxy)]-[2- Nal]-[4-amino-4-carboxy-tetrahydropyran]-ENN-NH2 | *** |
| 104 | Ac-[(D)Arg]-cyclo[[Abu]-QT-[Trp(5-F)]-QC]-[Phe[4-(2- aminoethoxy)]-[2-Nal]-[4-amino-4-carboxy-tetrahydropyran]- ENN-NH2 | ** |
| 105 | Ac-[(D)Arg]-cyclo[[Abu]-QT-[Trp(5-F)]-QC]-[Phe[4-(2- aminoethoxy)]-[2-Nal]-[α-MeLeu]-ENN-NH2 | * |
| 106 | Ac-cyclo[[Abu]-QT-[Trp(5-F)]-QC]-[Phe[4-(2-aminoethoxy)]-[2- Nal]-[α-MeLeu]-ENN-NH2 | * |
| 107 | Ac-[(D)Arg]-cyclo[[Abu]-QTWQC]-[Phe[4-(2-aminoethoxy)]-[2- Nal]-[α-MeLeu]-ENN-NH2 | * |
| 108 | Ac-[(D)Arg]-cyclo[[Abu]-QTW-[2-Nal]-C]-[Phe[4-(2- aminoethoxy)]-[2-Nal]-[ 4-amino-4-carboxy- tetrahydropyran]-ENN-NH2 | ** |
| 109 | Ac-[(D)Arg]-cyclo[[Abu]-QTW-[1-Nal]-C]-[Phe[4-(2- aminoethoxy)]-[2-Nal]-[ 4-amino-4-carboxy- tetrahydropyran]-ENN-NH2 | ** |
| 110 | Ac-[(D)Arg]-cyclo[[Abu]-QTW-[Trp(5-F)]-C]-[Phe[4-(2- aminoethoxy)]-[2-Nal]-[ 4-amino-4-carboxy- tetrahydropyran]-ENN-NH2 | ** |

**Table E3. IC₅₀s of Additional Illustrative Peptide Inhibitors**

| **SEQ ID No.** | **Sequence** | **pStat3 HTRF (nM)** |
|---|---|---|
| 135 | Ac-cyclo[[(D)Abu]-QTWQC]-[Phe[4-(2-aminoethoxy)]-[2- Nal]-[α-MeLeu]-ENN-NH2 | *** |
| 136 | Ac-cyclo[[(D)Abu]-NTWQ-[Pen]]-[Phe[4-(2-aminoethoxy)]- [2-Nal]-[α-MeLeu]-[Lys(Ac)]-NN-NH2 | ** |
| 137 | Ac-cyclo[Pen]-NTWQ-[(D)Abu]]-[Phe[4-(2-aminoethoxy)]-[2- Nal]-[α-MeLeu]-[Lys(Ac)]-NN-NH2 | **** |
| 138 | Ac-[(D)Arg]-cyclo[[Abu]-QTWQC]-[Phe[4-(2-aminoethoxy)]- [2-Nal]-[α-MeLeu]-ENN-NH2 | * |
| 139 | Ac-[(D)Arg]-cyclo[[Abu]-QTW-[2-Nal]-C]-[Phe[4-(2- aminoethoxy)]-[2-Nal]-[4-amino-4-carboxy-tetrahydropyran]-ENN- NH2 | ** |
| 140 | Ac-[(D)Arg]-cyclo[[Abu]-QTW-[1-Nal]-C]-[Phe[4-(2- aminoethoxy)]-[2-Nal]-[4-amino-4-carboxy-tetrahydropyran]-ENN- NH2 | ** |
| 141 | Ac-[(D)Arg]-cyclo[[Abu]-QTW-[5-fluor-Trp]-C]-[Phe[4-(2- aminoethoxy)]-[2-Nal]-[4-amino-4-carboxy-tetrahydropyran]-ENN- NH2 | ** |
| 142 | Ac-[Pen]-NTWQ-[Pen]-[Phe[4-(2-aminoethoxy)]-[2-Nal]-[α- MeLeu]-[Lys(Ac)]-NN-OH | ** |
| 143 | Ac-[Pen]-NTWQ-[Pen]-[Phe[4-(2-aminoethoxy)]-[2-Nal]-[α- MeLeu]-[Lys(Ac)]-ND-NH2 | ** |
| 144 | Ac-[Pen]-NTWQ-[Pen]-[Phe[4-(2-aminoethoxy)]-[2-Nal]-[α- MeLeu]-[Lys(Ac)]-DN-NH2 | *** |
| 145 | Ac-[Pen]-NTWE-[Pen]-[Phe[4-(2-aminoethoxy)]-[2-Nal]-[α- MeLeu]-[Lys(Ac)]-NN-NH2 | ** |
| 146 | Ac-[Pen]-DTWQ-[Pen]-[Phe[4-(2-aminoethoxy)]-[2-Nal]-[α- MeLeu]-[Lys(Ac)]-NN-NH₂ | ** |
| 147 | Ac-[(D)Arg]-cyclo[[Abu]-QTWQC]-[Phe[4-(2-aminoethoxy)]-[2- Nal]-[3-cyclohexyl-Ala]-ENN-NH2 | * |
| 148 | Ac-[(D)Arg]-cyclo[[Abu]-QTWQC]-[Phe[4-(2-aminoethoxy)]-[2- Nal]-[Aib]-ENN-NH2 | * |
| 150 | Ac-[(D)Arg]-cyclo[[(D)Pen(sulfoxide)]-QTWQ-[Abu]]-[Phe[4-(2- aminoethoxy)]-[2-Nal]-[4-amino-4-carboxy-tetrahydropyran]-ENN- NH2 | **** |
| 151 | Ac-cyclo[[(D)Pen]-QTWQ-[Abu]]-[Phe[4-(2-aminoethoxy)]-[2-Nal]- [4-amino-4-carboxy-tetrahydropyran]-ENN-NH2 | **** |
| 152 | Ac-cyclo[[(D)Pen(sulfoxide)]-QTWQ-[Abu]]-[Phe[4-(2- aminoethoxy)]-[2-Nal]-[4-amino-4-carboxy-tetrahydropyran]-ENN- NH2 | **** |
| 153 | Ac-[(D)Arg]-cyclo[[Abu]-QTW-Lys(isoGlu-Palm)-C]-[Phe[4-(2- aminoethoxy)]-[2-Nal]-[4-amino-4-carboxy-tetrahydropyran]-ENN- NH2 | **** |
| 154 | Ac-[(D)Arg]-cyclo[[Abu]-QTW-[Lys(PEG12)]-C]-[Phe[4-(2- aminoethoxy)]-[2-Nal]-[4-amino-4-carboxy-tetrahydropyran]-ENN- NH2 | ** |
| 156 | Ac-[(D)Arg]-cyclo[[Abu]-QTWQC]-[Phe[4-(2-aminoethoxy)]-[2- Nal]-[4-amino-4-carboxy-tetrahydropyran]-[Lys(PEG12)]-NN-NH2 | ** |
| 158 | Ac-[Pen]-NTWQ-[Pen]-[Phe[4-(2-aminoethoxy)]-[2-Nal]-[α- MeLeu]-[Lys(Ac)]-NND-NH2 | ** |
| 159 | Ac-[Pen]-NTWQ-[Pen]-[Phe[4-(2-aminoethoxy)]-[2-Nal]-[α- MeLeu]-[Lys(Ac)]-NNY-NH2 | * |
| 160 | Ac-[Pen]-NTWQ-[Pen]-[Phe[4-(2-aminoethoxy)]-[2-Nal]-[α- MeLeu]-[Lys(Ac)]-NNW-NH2 | ** |
| 161 | Ac-[Pen]-NTWQ-[Pen]-[Phe[4-(2-aminoethoxy)]-[2-Nal]-[α- MeLeu]-[Lys(Ac)]-NNR-NH2 | * |
| 162 | Ac-[Pen]-NTWQ-[Pen]-[Phe[4-(2-aminoethoxy)]-[2-Nal]-[α- MeLeu]-[Lys(Ac)]-NNL-NH2 | ** |
| 163 | Ac-[Pen]-NTWQ-[Pen]-[Phe[4-(2-aminoethoxy)]-[2-Nal]-[α- MeLeu]-[Lys(Ac)]-NNG-NH2 | ** |
| 164 | Ac-[Pen]-NTWQ-[Pen]-[Phe[4-(2-aminoethoxy)]-[2-Nal]-[α- MeLeu]-[Lys(Ac)]-NNQ-NH2 | * |
| 165 | Ac-[Pen]-NTWQ-[Pen]-[Phe[4-(2-aminoethoxy)]-[2-Nal]-[α- MeLeu]-[Lys(Ac)]-NNS-NH2 | * |
| 166 | Ac-[Pen]-NTWQ-[Pen]-[Phe[4-(2-aminoethoxy)]-[2-Nal]-[α- MeLeu]-[Lys(Ac)]-N-[Lys(Ac)]-NH2 | ** |
| 168 | Ac-[Pen]-NTWQ-[Pen]-[Phe[4-(2-aminoethoxy)]-[2-Nal]-[α- MeLeu]-ENN-NH2 | ** |
| 169 | Ac-[Pen]-[Lys(Ac)]-TWQ-[Pen]-[Phe[4-(2-aminoethoxy)]-[2-Nal]- [α-MeLeu]-[Lys(Ac)]-NN-NH2 | ** |
| 170 | Ac-[Pen]-NTW-[Lys(Ac)]-[Pen]-[Phe[4-(2-aminoethoxy)]-[2-Nal]- [α-MeLeu]-[Lys(Ac)]-NN-NH2 | * |
| 171 | Ac-[Pen]-NTWQ-[Pen]-[Phe[4-(2-aminoethoxy)]-[2-Nal]-[α- MeLeu]-[Lys(Ac)]-NN-[(D)Asp]-NH2 | ** |
| 172 | Ac-[Pen]-NTWQ-[Pen]-[Phe[4-(2-aminoethoxy)]-[2-Nal]-[α- MeLeu]-[Lys(Ac)]-NN-[(D)Tyr]-NH2 | ** |
| 173 | Ac-[Pen]-NTWQ-[Pen]-[Phe[4-(2-aminoethoxy)]-[2-Nal]-[α- MeLeu]-[Lys(Ac)]-NN-[2-Nal]-NH2 | ** |
| 174 | Ac-[Pen]-NTWQ-[Pen]-[Phe[4-(2-aminoethoxy)]-[2-Nal]-[α- MeLeu]-[Lys(Ac)]-NN-[(D)Arg]-NH2 | * |
| 175 | Ac-[Pen]-NTWQ-[Pen]-[Phe[4-(2-aminoethoxy)]-[2-Nal]-[α- MeLeu]-[Lys(Ac)]-NN-[(D)Ile]-NH2 | * |
| 176 | Ac-[Pen]-NTWQ-[Pen]-[Phe[4-(2-aminoethoxy)]-[2-Nal]-[α- MeLeu]-[Lys(Ac)]-NN-[(D)Ala]-NH2 | ** |
| 177 | Ac-[Pen]-NTWQ-[Pen]-[Phe[4-(2-aminoethoxy)]-[2-Nal]-[α- MeLeu]-[Lys(Ac)]-NN-[(D)Gln]-NH2 | * |
| 178 | Ac-[Pen]-NTWQ-[Pen]-[Phe[4-(2-aminoethoxy)]-[2-Nal]-[α- MeLeu]-[Lys(Ac)]-NN-[(D)Ser]-NH2 | ** |
| 179 | Ac-[Lys(Ac)]-[Pen]-NTWQ-[Pen]-[Phe[4-(2-aminoethoxy)]-[2-Nal]- [α-MeLeu]-[Lys(Ac)]-NN-NH2 | ** |
| 180 | Ac-[Lys(Ac)]-[Pen]-NTWQ-[Pen]-[Phe(4-CONH2)]-[2-Nal]-[α- MeLeu]-[Lys(Ac)]-NN-NH2 | ** |
| 181 | Ac-[Pen]-NTWQ-[Pen]-[Phe[4-(2-aminoethoxy)]-[2-Nal]-[α- MeLeu]-[Lys(Ac)]-NN-[Lys(Ac)]-NH2 | ** |
| 182 | Ac-[Pen]-NTWQ-[Pen]-[Phe(4-CONH2)]-[2-Nal]-[α-MeLeu]- [Lys(Ac)]-NN-[Lys(Ac)]-NH2 | ** |
| 183 | Ac-[Pen]-NTWQ-[Pen]-[Phe(4-CONH2)]-[2-Nal]-[α-MeLeu]- [Lys(Ac)]-NN-[(D)Lys]-NH2 | ** |
| 184 | (Ac-[Pen]-NTWQ-[Pen]-[Phe(4-CONH2)]-[2-Nal]-[α-MeLeu]- [Lys(Ac)]-NN-[(D)Lys]-NH2)2 -DIG | * |
| 185 | Ac-[Pen]-NT-[5-Fluro-Trp]-Q-[Pen]-[Phe[4-(2-aminoethoxy)]-[2- Nal]-[α-MeLeu]-[Lys(Ac)]-NN-NH2 | ** |
| 186 | Ac-[Pen]-NTWQ-[Pen]-[Phe[4-(2-aminoethoxy)]-[2-Nal]-[α- MeLeu]-[β-homo-Glu]-NN-NH2 | ** |
| 187 | Ac-[Pen]-NTWQ-[Pen]-[Phe[4-(2-aminoethoxy)]-[2-Nal]-[α- MeLeu]-DNN-NH2 | *** |
| 188 | Ac-[Pen]-NTWQ-[Pen]-[Phe[4-(2-aminoethoxy)]-[2-Nal]-[α- MeLeu]-[Lys(Ac)]-[Cit]-N-NH2 | ** |
| 189 | Ac-[Pen]-NTWQ-[Pen]-[Phe[4-(2-aminoethoxy)]-[2-Nal]-[α- MeLeu]-[Lys(Ac)]-N-[Cit]-NH2 | ** |
| 190 | Ac-[Pen]-NTWQ-[Pen]-[Phe[4-(2-aminoethoxy)]-[2-Nal]-[α- MeLeu]-[Lys(Ac)]-NG-NH2 | ** |
| 191 | Ac-[Pen]-NTW-[Cit]-[Pen]-[Phe[4-(2-aminoethoxy)]-[2-Nal]-[α- MeLeu]-[Lys(Ac)]-NN-NH2 | ** |
| 192 | Ac-[Pen]-NTWQ-[Pen]-[Phe[4-(2-aminoethoxy)]-[2-Nal]-[α- MeLeu]-[Lys(Ac)]-N-[Sarc]-NH2 | ** |
| 193 | Ac-[Pen]-[Cit]-TW-[Cit]-[Pen]-[Phe[4-(2-aminoethoxy)]-[2-Nal]-[α- MeLeu]-[Lys(Ac)]-NN-NH2 | ** |
| 194 | Ac-[Pen]-NTWQ[Pen]-[Phe[4-(2-aminoethoxy)]-[2-Nal]-[Deg ]- [Lys(Ac)]-NN-NH2 | ** |
| 195 | Ac-[Pen]-NTW-[Lys(Ac)]-[Pen]-[Phe[4-(2-aminoethoxy)]-[2-Nal]- [α-MeLeu]-[Lys(Ac)]-NNG-NH2 | * |
| 196 | Ac-[Pen]-NTW-[Lys(Ac)]-[Pen]-[Phe[4-(2-aminoethoxy)]-[2-Nal]- [α-MeLeu]-[Lys(Ac)]-NN-[β-Ala]-NH2 | * |
| 197 | Ac-[Pen]-NTW-[Lys(Ac)]-[Pen]-[Phe[4-(2-aminoethoxy)]-[2-Nal]- [α-MeLeu]-[Lys(Ac)]-NN-[Ahx]-NH2 | * |
| 198 | Ac-[Pen]-NTW-[Lys(Ac)]-[Pen]-[Phe[4-(2-aminoethoxy)]-[2-Nal]- [α-MeLeu]-[Lys(Ac)]-NN-[Sar]-NH2 | * |
| 199 | Ac-[Pen]-NTW-[Lys(Ac)]-[Pen]-[Phe[4-(2-aminoethoxy)]-[2-Nal]- [α-MeLeu]-[Lys(Ac)]-NN-[(D)Arg]-NH2 | ** |
| 200 | Ac-[Pen]-NTW-[Lys(Ac)]-[Pen]-[Phe[4-(2-aminoethoxy)]-[2-Nal]- [α-MeLeu]-[Lys(Ac)]-NN-[(D)Ile]-NH2 | ** |
| 202 | Ac-[Pen]-NT-[Trp(5-F)]-[Lys(Ac)]-[Pen]-[Phe[4-(2-aminoethoxy)]- [2-Nal]-[α-MeLeu]-[Lys(Ac)]-NN-NH2 | ** |
| 203 | Ac-[Pen]-NT-[Trp(5-F)]-[Lys(Ac)]-[Pen]-[Phe[4-(2-aminoethoxy)]- [2-Nal]-[α-MeLeu]-[Lys(Ac)]-NNG-NH2 | ** |
| 204 | (Ac-[Pen]-NTW-[Lys(Ac)]-[Pen]-[Phe[4-(2-aminoethoxy)]-[2-Nal]- [α-MeLeu]-[Lys(Ac)]-NN-NH₂)₂-DIG | ** |
| 205 | (Ac-[Pen]-NTW-[Lys(Ac)]-[Pen]-[Phe[4-(2-aminoethoxy)]-[2-Nal]- [α-MeLeu]-[Lys(Ac)]-NN-NH₂)₂-IDA | *** |
| 206 | (Ac-[Pen]-NTW-[Lys(Ac)]-[Pen]-[Phe[4-(2-aminoethoxy)]-[2-Nal]- [α-MeLeu]-[Lys(Ac)]-NN-NH₂)₂-PEG2 | *** |
| 207 | (Ac-[Pen]-NTW-[Lys(Ac)]-[Pen]-[Phe[4-(2-aminoethoxy)]-[2-Nal]- [α-MeLeu]-[Lys(Ac)]-NN-NH₂)₂-PEG4 | ** |
| 208 | (Ac-[Pen]-NTW-[Lys(Ac)]-[Pen]-[Phe[4-(2-aminoethoxy)]-[2-Nal]- [α-MeLeu]-[Lys(Ac)]-NN-NH2)2 -PEG13 | ** |
| 209 | Ac-[Pen]-NTW-[Lys(Ac)]-[Pen]-[Phe[4-(2-aminoethoxy)]-[2-Nal]- [α-MeLeu]-[Lys(Ac-Arg)]- NN-[(D)Ile]-NH2 | ** |
| 210 | Ac-[Pen]-NTW[-Lys(Ac)]-[Pen]-[Phe[4-(2-aminoethoxy)]-[2-Nal]- [α-MeLeu]-[Lys(Ac-Tyr)]-NN-[(D)Ile]-NH2 | ** |
| 211 | Ac-[Pen]-NTW[-Lys(Ac)]-[Pen]-[Phe[4-(2-aminoethoxy)]-[2-Nal]- [α-MeLeu]-[Lys(Ac-Asn)]-NN-[(D)Ile]-NH2 | ** |
| 212 | Ac-[Pen]-NTW-[Lys(Ac)]-[Pen]-[Phe[4-(2-aminoethoxy)]-[2-Nal]- [α-MeLeu]-[Lys(Ac-Thr)]-NN-[(D)Ile]-NH2 | ** |
| 213 | Ac-[Pen]-NTW-[Lys(Ac)]-[Pen]-[Phe[4-(2-aminoethoxy)]-[2-Nal]- [α-MeLeu]-[Lys(Ac-Asp)]-NN-[(D)Ile]-NH2 | ** |
| 214 | Ac-[Pen]-NTW-[Lys(Ac)]-[Pen]-[Phe[4-(2-aminoethoxy)]-[2-Nal]- [α-MeLeu]-[Lys(Ac-Leu)]-NN-[(D)Ile]-NH2 | ** |
| 215 | Ac-[Pen]-NTW-[Lys(Ac)]-[Pen]-[Phe[4-(2-aminoethoxy)]-[2-Nal]- [α-MeLeu]-[Lys(Ac-Phe)]-NN-[(D)Ile]-NH2 | ** |
| 216 | Ac-[Pen]-NTW-[Lys(Ac)]-[Pen]-[Phe[4-(2-aminoethoxy)]-[2-Nal]- [α-MeLeu]-[Lys(Ac-(D)Arg)]-NN-[(D)Ile]-NH2 | ** |
| 217 | Ac-[Pen]-NTW-[Lys(Ac)]-[Pen]-[Phe[4-(2-aminoethoxy)]-[2-Nal]- [α-MeLeu]-[Lys(Ac-(D)Tyr)]-NN-[(D)Ile]-NH2 | ** |
| 218 | Ac-[Pen]-NTW-[Lys(Ac)]-[Pen]-[Phe[4-(2-aminoethoxy)]-[2-Nal]- [α-MeLeu]-[Lys(Ac-(D)Asn)]-NN-[(D)Ile]-NH2 | * |
| 219 | Ac-[Pen]-NTW-[Lys(Ac)]-[Pen]-[Phe[4-(2-aminoethoxy)]-[2-Nal]- [α-MeLeu]-[Lys(Ac-(D)Thr)]-NN-[(D)Ile]-NH2 | ** |
| 220 | Ac-[Pen]-NTW-[Lys(Ac)]-[Pen]-[Phe[4-(2-aminoethoxy)]-[2-Nal]- [α-MeLeu]-[Lys(Ac-(D)Asp)]-NN-[(D)Ile]-NH2 | ** |
| 221 | Ac-[Pen]-NTW-[Lys(Ac)]-[Pen]-[Phe[4-(2-aminoethoxy)]-[2-Nal]- ;α-MeLeu;-;Lys(Ac-((D)Leu);-NN-;(D)Ile;-NH2 | *** |
| 222 | Ac-[Pen]-NTW-[Lys(Ac)]-[Pen]-[Phe[4-(2-aminoethoxy)]-[2-Nal]- [α-MeLeu]-[Lys(Ac-(D)Phe)]-NN-[(D)Ile]-NH2 | ** |
| 223 | Ac-[Lys(Ac-Glu)]-[Pen]-NTW-[Lys(Ac)]-[Pen]-[Phe[4-(2- aminoethoxy)]-[2-Nal]-[α-MeLeu]-[Lys(Ac)]-NN-NH2 | * |
| 224 | Ac-[Lys(Ac-Phe)]-[Pen]-NTW-[Lys(Ac)]-[Pen]-[Phe[4-(2- aminoethoxy)]-[2-Nal]-[α-MeLeu]-[Lys(Ac)]-NN-NH2 | * |
| 225 | Ac-[Lys(Ac-Tyr)]-[Pen]-NTW-[Lys(Ac)]-[Pen]-[Phe[4-(2- aminoethoxy)]-[2-Nal]-[α-MeLeu]-[Lys(Ac)]-NN-NH2 | * |
| 226 | Ac-[Lys(Ac-Ser)]-[Pen]-NTW-[Lys(Ac)]-[Pen]-[Phe[4-(2- aminoethoxy)]-[2-Nal]-[α-MeLeu]-[Lys(Ac)]-NN-NH2 | * |
| 227 | Ac-[Lys(Ac-Arg)]-[Pen]-NTW-[Lys(Ac)]-[Pen]-[Phe[4-(2- aminoethoxy)]-[2-Nal]-[α-MeLeu]-[Lys(Ac)]-NN-NH2 | * |
| 228 | Ac-[Lys(Ac-Leu)]-[Pen]-NTW-[Lys(Ac)]-[Pen]-[Phe[4-(2- aminoethoxy)]-[2-Nal]-[α-MeLeu]-[Lys(Ac)]-NN-NH2 | * |
| 229 | Ac-[Lys(Ac-Pro)]-[Pen]-NTW-[Lys(Ac)]-[Pen]-[Phe[4-(2- aminoethoxy)]-[2-Nal]-[α-MeLeu]-[Lys(Ac)]-NN-NH2 | * |
| 230 | Ac-[(D)Lys(Ac-Glu)]-[Pen]-NTW-[Lys(Ac)]-[Pen]-[Phe[4-(2- aminoethoxy)]-[2-Nal]-[α-MeLeu]-[Lys(Ac)]-NN-NH2 | * |
| 231 | Ac-[(D)Lys(Ac-Phe)]-[Pen]-NTW-[Lys(Ac)]-[Pen]-[Phe[4-(2- aminoethoxy)]-[2-Nal]-[α-MeLeu]-[Lys(Ac)]-NN-NH2 | * |
| 232 | Ac-[(D)Lys(Ac-Tyr)]-[Pen]-NTW-[Lys(Ac)]-[Pen]-[Phe[4-(2- aminoethoxy)]-[2-Nal]-[α-MeLeu]-[Lys(Ac)]-NN-NH₂ | * |
| 233 | Ac-[(D)Lys(Ac-Ser)]-[Pen]-NTW-[Lys(Ac)]-[Pen]-[Phe[4-(2- aminoethoxy)]-[2-Nal]-[α-MeLeu]-[Lys(Ac)]-NN-NH2 | * |
| 234 | Ac-[(D)Lys(Ac-Arg)]-[Pen]-NTW-[Lys(Ac)]-[Pen]-[Phe[4-(2- aminoethoxy)]-[2-Nal]-[α-MeLeu]-[Lys(Ac)]-NN-NH2 | * |
| 235 | Ac-[(D)Lys(Ac-Leu)]-[Pen]-NTW-[Lys(Ac)]-[Pen]-[Phe[4-(2- aminoethoxy)]-[2-Nal]-[α-MeLeu]-[Lys(Ac)]-NN-NH2 | * |
| 236 | Ac-[(D)Lys(Ac-Pro)]-[Pen]-NTW-[Lys(Ac)]-[Pen]-[Phe[4-(2- aminoethoxy)]-[2-Nal]-[α-MeLeu]-[Lys(Ac)]-NN-NH2 | * |

All of the above U.S. patents, U.S. patent application publications, U.S. patent applications, foreign patents, foreign patent applications and non-patent publications referred to in this specification and/or listed in the Application Data Sheet, are incorporated herein by reference, in their entirety.

From the foregoing it will be appreciated that, although specific embodiments of the invention have been described herein for purposes of illustration, various modifications may be made without deviating from the spirit and scope of the invention. Accordingly, the invention is not limited except as by the appended claims.

### EMBODIMENTS

1. A peptide inhibitor of an interleukin-23 receptor, or a pharmaceutically acceptable salt or solvate thereof, wherein the peptide inhibitor comprises an amino acid sequence of Formula (V): 
X0-X1-X2-X3-X4-X5-X6-X7-X8-X9-X10-X11-X12-X13-X14-X15-X16-X17-X18-X19-
X20-X21-X22-X23 (V) (SEQ ID NO:238)
 wherein
   X0 is Gly, Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, or absent;
   X1 is Gly, Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, or absent;
   X2 is (D)Asp, Arg, (D)Arg, Phe, (D)Phe, 2-Nal, Thr, Leu, (D)Gln, (D)Asn, IsoGlu, Gly, Arg, Phe, Glu, Gln, Thr, (D)Glu, (D)Thr, (D)Leu, alpha-MeArg, alpha-MePhe, alpha-MeLeu, alpha-MeLys, alpha-MeAsn, alpha-MeTyr, or absent;
   X3 is (D)Arg, (D)Tyr, Gly, alpha-MeArg, alpha-MePhe, alpha-MeLeu, alpha-MeLys, alpha-MeAsn, alpha-MeTyr, Lys(Ac), Lys(Y1-Ac), or absent, wherein Y1 is an amino acid;
   X4 is Abu, Cys, (D)Cys), alpha-MeCys, (D)Abu, (D)Pen, Pen, or Pen(sulfoxide);
   X5 is Cit, Glu, Gly, Lys, Asn, Pro, alpha-MeGln, alpha-MeLys, alpha-MeLeu, alpha-MeAsn, Lys(Ac), alpha-MeLys(Ac), Dab(Ac), Dap(Ac), homo-Lys(Ac), Gln, Asp, or Cys;
   X6 is Thr, Aib, Asp, Dab, Gly, Pro, Ser, alpha-MeGln, alpha-MeLys, alpha-MeLeu, alpha-MeAsn, alpha-MeThr, alpha-MeSer, or Val;
   X7 is Trp, Trp(5-F), 1-Nal, 2-Nal, Phe(2-Me), Phe(3-Me), Phe(4-Me), Trp(7-Aza), or Phe(3,4-dimethoxy);
   X8 is Gln, alpha-Me-Lys, alpha-MeLeu, alpha-MeLys(Ac), beta-homoGln, Cit, Glu, Phe, Asn, Thr, Val, Aib, alpha-MeGln, alpha-MeAsn, Lys(Ac), alpha-MeLys(Ac), Dab(Ac), Dap(Ac), homo-Lys(Ac), 1-Nal, 2-Nal, or Trp;
   X9 is Cys, (D)Cys), alpha-MeCys, (D)Abu, (D)Pen, Pen, or Abu;
   X10 is Phe, Phe[4-(2-aminoethoxy)], Phe[4-(2-acetylaminoethoxy)], alpha-MeTyr, or Phe(4-CONH₂);
   X11 is 2-Nal, Trp, Trp(5-F), Trp(7-Aza), Phe(2-Me), Phe(3-Me), Phe(4-Me), Phe(3,4-dimethoxy), or 1-Nal;
   X12 is 4-amino-4-carboxy-tetrahydropyran (THP), alpha-MeLys, alpha-MeLeu, alpha-MeArg, alpha-MePhe, alpha-MeLeu, alpha-MeLys, alpha-MeAsn, alpha-MeTyr, Ala, cyclohexylAla, Lys, or Aib;
   X13 is Glu, Cit, Gln, Lys(Ac), alpha-MeArg, alpha-MeGlu, alpha-MeLeu, alpha-MeLys, alpha-Me-Asn, alpha-MeLys(Ac), Dab(Ac), Dap(Ac), homo-Lys(Ac), Lys, pegylated Lys, b-homoGlu, or Lys(Y2-Ac), wherein Y2 is an amino acid;
   X14 is Asn, 2-Nap, Aib, Arg, Cit, Asp, Phe, Gly, Lys, Leu, Asn, n-Leu, Gln, Ser, Tic, Trp, alpha-MeGln, alpha-MeAsn, alpha-MeLys(Ac), Dab(Ac), Dap(Ac), homo-Lys(Ac), or Lys(Ac);
   X15 is Asn, Aib, beta-Ala, Cit, Gln, Asp, alpha-MeGln, alpha-MeAsn, Lys(Ac), alpha-MeLys(Ac), Dab(Ac), Dap(Ac), homo-Lys(Ac), or absent;
   X16 is Glu, Phe, Lys, Asn, Trp, Gly, Thr, Pro, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, Ala, Asp, Tyr, Arg, Leu, Gln, Ser, Ile, 1-Nal, 2-Nal, (D)Ala, (D)Asp, (D)Tyr, (D)Arg, (D)Leu, (D)Ser, (D)Ile, or absent;
   X17 is Lys, Gly, Pro, The, Phe, Trp, Gln, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent;
   X18 is Gly, Lys, Glu, Phe, Thr, Arg, Gln, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent;
   X19 is Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent;
   X20 is Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent;
   X21 is Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent;
   X22 is Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent; and
   X23 is Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent,
   wherein the peptide inhibitor is cyclized via a bond between X4 and X9, and wherein the peptide inhibitor inhibits the binding of an interleukin-23 (IL-23) to an IL-23 receptor.
2. The peptide inhibitor of embodiment 1, wherein the bond between X4 and X9 is a disulfide bond or a thioether bond.
3. The peptide inhibitor of embodiment 1, wherein X4 is Pen and X9 is Pen, and the bond is a disulfide bond.
4. The peptide inhibitor of embodiment 3, wherein the peptide inhibitor has a structure of Formula (III).
5. The peptide inhibitor of embodiment 3, wherein the peptide inhibitor comprises an amino acid sequence set forth in Formula (IIIa) or Table E1.
6. The peptide inhibitor of embodiment 1, wherein X4 is Abu and X9 is Cys, and the bond is a thioether bond.
7. The peptide inhibitor of embodiment 6, wherein the peptide inhibitor has a structure of Formula (IV).
8. The peptide inhibitor of embodiment 6, wherein the peptide inhibitor comprises an amino acid sequence set forth in Formula (IVa) or Table E2.
9. The peptide inhibitor of any one of embodiments 1-8, further comprising one or more half-life extension moiety and/or one or more linker moiety conjugated to the peptide inhibitor.
10. The peptide inhibitor of embodiment 9, wherein the half-life extension moiety is conjugated to the peptide inhibitor via one or more linker moieties.
11. The peptide inhibitor of any one of embodiments 1-10, wherein the peptide inhibitor comprises the structure of Formula (Z):

   R¹-X-R² (Z)

   or a pharmaceutically acceptable salt or solvate thereof, wherein
   R¹ is a bond, hydrogen, a C1-C6 alkyl, a C6-C12 aryl, a C6-C12 aryl, a C1-C6 alkyl, a C1-C20 alkanoyl, and including PEGylated versions alone or as spacers of any of the foregoing;
   X is the amino acid sequence of Formula (I), Formula (II), Formula (IIIa), Formula (IVa), Formula (V), Formula (XII)-(XVIIIh), or an amino acid sequence set forth in any of Tables E1, E2, or E3; and R² is OH or NH₂.
12. A peptide dimer inhibitor of an interleukin-23 receptor, wherein the peptide dimer inhibitor comprises two peptide monomer subunits connected via one or more linker moieties, wherein each peptide monomer subunit comprises an amino acid sequence or structure of Formula (I), (II), (IIIa), (IVa), Formula (V), Formula (XII)-(XVIIIh), or an amino acid sequence set forth in any of Tables E1, E2, and E3.
13. The peptide dimer inhibitor of embodiment 12, wherein the one or more linker moiety is a diethylene glycol linker, an iminodiacetic acid (IDA) linker, a β-Ala-iminodiaceticacid (β-Ala-IDA) linker, or a PEG linker.
14. The peptide dimer inhibitor of embodiment 12 or embodiment 13, wherein the N-terminus of each peptide monomer subunit is connected by the linker moiety or wherein the C-terminus of each peptide monomer subunit is connected by the linker moiety.
15. The peptide inhibitor of embodiment 11 or the peptide dimer inhibitor of embodiment 12, wherein X comprises or consists of the sequence of Formula XII:
   X2-X3-X4-X5-T-X7-X8-X9-X10-X11-X12-X13-X14-X15-X16 (XII) (SEQ ID NO:275) wherein
   X2 is Arg, (D)Arg, Gln, or absent;
   X3 is (D)Arg, Phe, (D)Phe, Lys, (D)Lys, Lys(Y1-Ac), (D)Lys(Y1-Ac), or absent, wherein Y1 is an amino acid or Y1 is absent;
   X4 is Cys, (D)Cys), alpha-MeCys, Abu, (D)Pen, Pen, (D)Pensulfoxide or Pensulfoxide;
   X5 is Cit, Lys, Asn, Asp, Glu, Lys(Ac), or Gln;
   X7 is Trp, substituted Trp, or 1-Nal, wherein substituted Trp is Trp substituted with halo, or azaTrp;
   X8 is Gln, Lys, Lys(Ac), a-MeLeu, Cit, Glu, 1-Nal, 2-Nal, Trp, substituted Trp, or Lys(Peg12);
   X9 is Cys, Abu, or Pen;
   X10 is Phe, Phe[4-(2-aminoethoxy)], Phe(Cmd), or Phe[4-(2-acetylaminoethoxy)];
   X11 is 2-Nal, Phe(2-Me), Phe(3-Me), Phe(4-Me), Phe(3,4-dimethoxy), or 1-Nal;
   X12 is alpha-MeLeu, Aib, Lys, cyclohexylAla, tetrahydropyranAla, Lys(Peg12), or Deg;
   X13 is Glu, b-homoGlu, Lys, (D)Lys, Lys(Y2-Ac), or (D)Lys(Y2-Ac); wherein Y2 is an amino acid, or Y2 is absent;
   X14 is Asn, Asp, Cit, or Lys(Ac);
   X15 is Asn, Lys, Lys(Ac), Cit, Asp, Gly, Ala, b-Ala, or Sarc;
   X16 is an amino acid or absent; and
   wherein X4 and X9 are capable of forming a disulfide bond or a thioether bond.
16. The peptide inhibitor or the peptide dimer inhibitor of embodiment 15, wherein X is according to Formula XIII:
   X3-X4-X5-T-Trp-X8-X9-Phe[4-(2-aminoethoxy)]-(2-Nal)-X12-X13-X14-Asn-X16 (XIII) (SEQ ID NO:276).
17. The peptide inhibitor or the peptide dimer inhibitor of embodiment 15, wherein X4 and X9 are joined together to form a disulfide bond or a thioether bond.
18. The peptide inhibitor or the peptide dimer inhibitor of embodiment 15, wherein X is according to Formula XIVa or XIVb:
   X3-Abu-X5-T-Trp-X8-X9-Phe[4-(2-aminoethoxy)]-(2-Nal)-X12-X13-X14-Asn-X16 (XIVa) (SEQ ID NO:277); or
   X3-Pen-X5-T-Trp-X8-X9-Phe[4-(2-aminoethoxy)]-(2-Nal)-X12-X13-X14-Asn-X16 (XIVb) (SEQ ID NO:278).
19. The peptide inhibitor or the peptide dimer inhibitor of embodiment 15, wherein X is according to Formula XVa, XVb, XVc, or XVd:
   X3-Abu-Asn-T-Trp-X8-X9-Phe[4-(2-aminoethoxy)]-(2-Nal)-X12-X13-X14-Asn-X16 (XVa) (SEQ ID NO:279);
   X3-Pen-Asn-T-Trp-X8-X9-Phe[4-(2-aminoethoxy)]-(2-Nal)-X12-X13-X14-Asn-X16 (XVb) (SEQ ID NO:280);
   X3-Abu-Gln-T-Trp-X8-X9-Phe[4-(2-aminoethoxy)]-(2-Nal)-X12-X13-X14-Asn-X16 (XVc) (SEQ ID NO:281); or
   X3-Pen-Gln-T-Trp-X8-X9-Phe[4-(2-aminoethoxy)]-(2-Nal)-X12-X13-X14-Asn-X16 (XVd) (SEQ ID NO:282).
20. The peptide inhibitor or the peptide dimer inhibitor of embodiment 15, wherein X is according to Formula XVIa, XVIb, XVIc, XVId, XVIe, XVIf, XVIg, or XVIh:
   X3-Abu-Asn-T-Trp-X8-Cys-Phe[4-(2-aminoethoxy)]-(2-Nal)-X12-X13-X14-Asn-X16 (XVIa) (SEQ ID NO:283);
   X3-Pen-Asn-T-Trp-X8-Cys-Phe[4-(2-aminoethoxy)]-(2-Nal)-X12-X13-X14-Asn-X16 (XVIb) (SEQ ID NO:284);
   X3-Abu-Gln-T-Trp-X8-Cys-Phe[4-(2-aminoethoxy)]-(2-Nal)-X12-X13-X14-Asn-X16 (XVIC) (SEQ ID NO:285);
   X3-Pen-Gln-T-Trp-X8-Cys-Phe[4-(2-aminoethoxy)]-(2-Nal)-X12-X13-X14-Asn-X16 (XVId) (SEQ ID NO:286);
   X3-Abu-Asn-T-Trp-X8-Pen-Phe[4-(2-aminoethoxy)]-(2-Nal)-X12-X13-X14-Asn-X16 (XVIe) (SEQ ID NO:287);
   X3-Pen-Asn-T-Trp-X8-Pen-Phe[4-(2-aminoethoxy)]-(2-Nal)-X12-X13-X14-Asn-X16 (XVIf) (SEQ ID NO:288);
   X3-Abu-Gln-T-Trp-X8-Pen-Phe[4-(2-aminoethoxy)]-(2-Nal)-X12-X13-X14-Asn-X16 (XVIg) (SEQ ID NO:289); or
   X3-Pen-Gln-T-Trp-X8-Pen-Phe[4-(2-aminoethoxy)]-(2-Nal)-X12-X13-X14-Asn-X16 (XVIh) (SEQ ID NO:290).
21. The peptide inhibitor or the peptide dimer inhibitor of embodiment 15, wherein X is according to Formula XVIIa, XVIIb, XVIIc, XVIId, XVIIe, XVIIf, XVIIg, or XVIIh:
   X3-Abu-Asn-T-Trp-X8-Cys-Phe[4-(2-aminoethoxy)]-(2-Nal)-[THP-Ala]-X13-Asn-Asn-X16 (XVIIa) (SEQ ID NO:291);
   X3-Pen-Asn-T-Trp-X8-Cys-Phe[4-(2-aminoethoxy)]-(2-Nal)-[THP-Ala]-X13-Asn-Asn-X16 (XVIIb) (SEQ ID NO:292);
   X3-Abu-Gln-T-Trp-X8-Cys-Phe[4-(2-aminoethoxy)]-(2-Nal)-[THP-Ala]-X13-Asn-Asn-X16 (XVIIc) (SEQ ID NO:293);
   X3-Pen-Gln-T-Trp-X8-Cys-Phe[4-(2-aminoethoxy)]-(2-Nal)-[THP-Ala]-X13-Asn-Asn-X16 (XVIId) (SEQ ID NO:294);
   X3-Abu-Asn-T-Trp-X8-Pen-Phe[4-(2-aminoethoxy)]-(2-Nal)-[THP-Ala]-X13-Asn-Asn-X16 (XVIIe) (SEQ ID NO:295);
   X3-Pen-Asn-T-Trp-X8-Pen-Phe[4-(2-aminoethoxy)]-(2-Nal)-[THP-Ala]-X13-Asn-Asn-X16 (XVIIf) (SEQ ID NO:296);
   X3-Abu-Gln-T-Trp-X8-Pen-Phe[4-(2-aminoethoxy)]-(2-Nal)-[THP-Ala]-X13-Asn-Asn-X16 (XVIIg) (SEQ ID NO:297); or
   X3-Pen-Gln-T-Trp-X8-Pen-Phe[4-(2-aminoethoxy)]-(2-Nal)-[THP-Ala]-X13-Asn-Asn-X16 (XVIIh) (SEQ ID NO:298).
22. The peptide inhibitor or the peptide dimer inhibitor of embodiment 15, wherein X is according to Formula XVIIIa, XVIIIb, XVIIIc, XVIIId, XVIIIe, XVIIIf, XVIIIg, or XVIIIh:
   X3-Abu-Asn-T-Trp-X8-Cys-Phe[4-(2-aminoethoxy)]-(2-Nal)-[a-MeLeu]-X13-Asn-Asn-X16 (XVIIIa) (SEQ ID NO:299);
   X3-Pen-Asn-T-Trp-X8-Cys-Phe[4-(2-aminoethoxy)]-(2-Nal)-[a-MeLeu]-X13-Asn-Asn-X16 (XVIIIb) (SEQ ID NO:300);
   X3-Abu-Gln-T-Trp-X8-Cys-Phe[4-(2-aminoethoxy)]-(2-Nal)-[a-MeLeu]-X13-Asn-Asn-X16 (XVIIIc) (SEQ ID NO:301);
   X3-Pen-Gln-T-Trp-X8-Cys-Phe[4-(2-aminoethoxy)]-(2-Nal)-[a-MeLeu]-X13-Asn-Asn-X16 (XVIIId) (SEQ ID NO:302);
   X3-Abu-Asn-T-Trp-X8-Pen-Phe[4-(2-aminoethoxy)]-(2-Nal)-[a-MeLeu]-X13-Asn-Asn-X16 (XVIIIe) (SEQ ID NO:303);
   X3-Pen-Asn-T-Trp-X8-Pen-Phe[4-(2-aminoethoxy)]-(2-Nal)-[a-MeLeu]-X13-Asn-Asn-X16 (XVIIIf) (SEQ ID NO:304);
   X3-Abu-Gln-T-Trp-X8-Pen-Phe[4-(2-aminoethoxy)]-(2-Nal)-[a-MeLeu]-X13-Asn-Asn-X16 (XVIIIg) (SEQ ID NO:305); or
   X3-Pen-Gln-T-Trp-X8-Pen-Phe[4-(2-aminoethoxy)]-(2-Nal)-[a-MeLeu]-X13-Asn-Asn-X16 (XVIIIh) (SEQ ID NO:306).
23. The peptide inhibitor or the peptide dimer inhibitor of any one of embodiments 15-22, wherein X3 is Gln, Glu, Lys(Ac) or a-MeLeu.
24. The peptide inhibitor or the peptide dimer inhibitor of any one of embodiments 15-23, wherein X8 is Lys(Y1-Ac) or (D)Lys(Y1-Ac); and Y1 is Glu, Phe, Trp, Pro, or Arg.
25. The peptide inhibitor or the peptide dimer inhibitor of any one of embodiments 15-24, wherein X13 is Glu, b-homoGlu, Lys, (D)Lys, Lys(Y2-Ac), or (D)Lys(Y2-Ac); and Y2 is an amino acid or absent.
26. The peptide inhibitor or the peptide dimer inhibitor of any one of embodiments 15-25, wherein X16 is Sar, Lys, (D)Lys, Ahx, b-Ala, Gly, Arg, (D)Arg, Ile, Gln, (D)Gln, Tyr, Ser, (D)Ser, (D)Tyr, Ala, Trp, Asp, or (D)Asp.
27. The peptide inhibitor or the peptide dimer inhibitor of any one of embodiments 15-26, wherein abu and Pen; abu and Cys; Pen and Pen; or Pen and Cys are joined together to form a disulfide bond.
28. The peptide inhibitor of any one of embodiments 1-11 or the peptide dimer inhibitor of any of embodiments 12-14 further comprising a conjugated chemical substituent.
29. The peptide inhibitor or the peptide dimer of embodiment 15, wherein the conjugated chemical substituent is a lipophilic substituent or a polymeric moiety.
30. The peptide inhibitor or the peptide dimer of embodiment 15, wherein the conjugated chemical substituent is Ac, Palm, gamaGlu-Palm, isoGlu-Palm, PEG2-Ac, PEG4-isoGlu-Palm, (PEG)₅-Palm, succinic acid, glutaric acid, pyroglutaric acid, benzoic acid, IVA, octanoic acid, 1,4 diaminobutane, isobutyl, or biotin.
31. The peptide inhibitor or the peptide dimer of embodiment 15, wherein the conjugated chemical substituent is a polyethylene glycol with a molecular mass of 400 Da to 40,000 Da.
32. A polynucleotide comprising a sequence encoding the peptide inhibitor of any one of embodiments 1-11 or one or both peptide monomer subunit of the peptide dimer inhibitor of any one of embodiments 12-14.
33. A vector comprising the polynucleotide of embodiment 32.
34. A pharmaceutical composition comprising the peptide inhibitor or the peptide dimer inhibitor of any one of embodiments 1-33, and a pharmaceutically acceptable carrier, excipient, or diluent.
35. The pharmaceutical composition of embodiment 34, further comprising an enteric coating.
36. The pharmaceutical composition of embodiment 35, wherein the enteric coating protects and releases the pharmaceutical composition within a subject's lower gastrointestinal system.
37. A method for treating an Inflammatory Bowel Disease (IBD), ulcerative colitis, Crohn's disease, Celiac disease (*nontropical Sprue),* enteropathy associated with seronegative arthropathies, microscopic colitis, collagenous colitis, eosinophilic gastroenteritis, colitis associated with radio- or chemo-therapy, colitis associated with disorders of innate immunity as in leukocyte adhesion deficiency-1, chronic granulomatous disease, glycogen storage disease type 1b, Hermansky-Pudlak syndrome, Chediak-Higashi syndrome, and Wiskott-Aldrich Syndrome, pouchitis resulting after proctocolectomy and ileoanal anastomosis, gastrointestinal cancer, pancreatitis, insulin-dependent diabetes mellitus, mastitis, cholecystitis, cholangitis, pericholangitis, chronic bronchitis, chronic sinusitis, asthma, psoriasis, psoriatic arthritis, or graft versus host disease in a subject, comprising providing to the subject an effective amount of the peptide inhibitor or peptide dimer inhibitor of any one of embodiments 1-32, or the pharmaceutical composition of any one of embodiments 34-36.
38. The method of embodiment 37, wherein the pharmaceutical composition is provided to the subject by an oral, parenteral, intravenous, peritoneal, intradermal, subcutaneous, intramuscular, intrathecal, inhalation, vaporization, nebulization, sublingual, buccal, parenteral, rectal, intraocular, inhalation, topically, vaginal, or topical route of administration.
39. The method of embodiment 37 for treating Inflammatory Bowel Disease (IBD), ulcerative colitis, Crohn's disease, wherein the pharmaceutical composition is provided to the subject orally.
40. The method of embodiment 37 for treating psoriasis, wherein the pharmaceutical composition is provided to the subject orally, topically, parenterally, intravenously, subcutaneously, peritonealy, or intravenously.

## Claims

1. A peptide inhibitor of an interleukin-23 receptor, or a pharmaceutically acceptable salt or solvate thereof, wherein the peptide inhibitor comprises an amino acid sequence of Formula (IVa) or Formula (IIIa): 
X0-X1-X2-X3-[Abu]-X5-X6-X7-X8-[Cys]-[Phe[4-(2-aminoethoxy)]-[2-Nal]-[4-amino-4-
carboxy-tetrahydropyran]-X13-X14-X15-X16-X17-X18-X19-X20-X21-X22-X23 (IVa)
 
X0-X1-X2-X3-[Pen]-X5-X6-X7-X8-[Pen]-[Phe[4-(2-aminoethoxy)]-[2-Nal]-[4-amino-4-
carboxy-tetrahydropyran]-X13-X14-X15-X16-X17-X18-X19-X20-X21-X22-X23 (IIIa)
 wherein
X0 is Gly, Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, or absent;
X1 is Gly, Arg, Phe, Glu, Gln, Thr, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, or absent;
X2 is (D)Asp, Arg, (D)Arg, Phe, (D)Phe, 2-Nal, Thr, Leu, (D)Gln, (D)Asn, IsoGlu, Gly, Arg, Phe, Glu, Gln, Thr, (D)Glu, (D)Thr, (D)Leu, alpha-MeArg, alpha-MePhe, alpha-MeLeu, alpha-MeLys, alpha-MeAsn, alpha-MeTyr, or absent;
X3 is (D)Arg, (D)Tyr, Gly, alpha-MeArg, alpha-MePhe, alpha-MeLeu, alpha-MeLys, alpha-MeAsn, alpha-MeTyr, or absent;
X5 is Cit, Glu, Gly, Lys, Asn, Pro, alpha-MeGln, alpha-MeLys, alpha-MeLeu, alpha-MeAsn, Lys(Ac), alpha-MeLys(Ac), Dab(Ac), Dap(Ac), homo-Lys(Ac), or Gln;
X6 is Thr, Aib, Asp, Dab, Gly, Pro, Ser, alpha-MeGln, alpha-MeLys, alpha-MeLeu, alpha-MeAsn, alpha-MeThr, alpha-MeSer, or Val;
X7 is Trp(5-F), Trp, 1-Nal, 2-Nal, Phe(2-Me), Phe(3-Me), Phe(4-Me), Trp(7-Aza), or Phe(3,4-dimethoxy);
X8 is Gln, alpha-Me-Lys, alpha-MeLeu, alpha-MeLys(Ac), beta-homoGln, Cit, Glu, Phe, Asn, Thr, Val, Aib, alpha-MeGln, alpha-MeAsn, Lys(Ac), alpha-MeLys(Ac), Dab(Ac), Dap(Ac), homo-Lys(Ac),or Trp;
X13 is Glu, Cit, Gln, alpha-MeArg, alpha-MeGlu, alpha-MeLeu, alpha-MeLys, alpha-MeAsn, Lys(Ac), alpha-MeLys(Ac), Dab(Ac), Dap(Ac), homo-Lys(Ac), or Lys;
X14 is Asn, 2-Nap, Aib, Arg, Cit, Asp, Phe, Gly, Lys, Leu, Asn, n-Leu, Gln, Ser, Tic, Trp, alpha-MeGln, alpha-MeAsn, alpha-MeLys(Ac), Dab(Ac), Dap(Ac), homo-Lys(Ac), or Lys(Ac);
X15 is Asn, Aib, beta-Ala, Cit, Gln, Asp, alpha-MeGln, alpha-MeAsn, Lys(Ac), alpha-MeLys(Ac), Dab(Ac), Dap(Ac), homo-Lys(Ac),or absent;
X16 is Glu, Phe, Lys, Asn, Trp, Gly, Thr, Pro, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent;
X17 is Lys, Gly, Pro, The, Phe, Trp, Gln, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent;
X18 is Gly, Lys, Glu, Phe, Thr, Arg, Gln, (D)Arg, (D)Phe, (D)Glu, (D)Thr, (D)Leu, (D)Gln, alpha-MeArg, alpha-MePhe, alpha-MeLeu, alpha-MeLys, alpha-MeAsn, alpha-MeTyr, alpha-MeAsp, or absent;
X19 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent;
X20 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent;
X21 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent;
X22 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent; and
X23 is Arg, Phe, Glu, Gln, Thr, (D)Arg, (D)Phe, (D)Glu, (D)Thr, (D)Leu, (D)Gln , alpha-MeArg, alpha-MePhe, alpha-MeLeu, alpha-MeLys, alpha-MeAsn, alpha-MeTyr, alpha-MeAsp, or absent;
wherein the peptide inhibitor comprising an amino acid sequence of Formula (IVa) is cyclized via a thioether bond between Abu and Cys and wherein the peptide inhibitor comprising an amino acid sequence of Formula (IIIa) is cyclized via a disulfide bond between Pen and Pen;
wherein at least one of X0 to X3 is not absent; and
wherein at least three of X13 to X23 are not absent.

2. The peptide inhibitor of claim 1, or a pharmaceutically acceptable salt or solvate thereof, wherein X7 is Trp(5-F).

3. The peptide inhibitor of claim 1, or a pharmaceutically acceptable salt or solvate thereof, wherein:
(a) the peptide inhibitor comprises an amino acid sequence of Formula (IVa) and wherein X5-X8 are selected from: QT-(Trp(5-F))-Q (SEQ ID NO:264), QTWQ (SEQ ID NO:242), QTWE (SEQ ID NO:251), ETWQ (SEQ ID NO:252), ETWE (SEQ ID NO:253), QTW-(alpha-MeLeu) (SEQ ID NO:254), QTW-(alpha-MeLys) (SEQ ID NO:255), QTW-(alpha-MeLys(Ac)) (SEQ ID NO:256), QTW-((D)Gln) (SEQ ID NO:257), QTW-(B-homoGln) (SEQ ID NO:258), QTWF (SEQ ID NO:259), QTWW (SEQ ID NO:260), QTWAib (SEQ ID NO:261), QTWT (SEQ ID NO:262), and QTWV (SEQ ID NO:263); or
(b) the peptide inhibitor comprises an amino acid sequence of Formula (IIIa) and wherein X5-X8 are selected from: NT[Trp(5-F)]Q (SEQ ID NO:250), QTWQ (SEQ ID NO:242), NDWQ (SEQ ID NO:243), N(Dab)WQ (SEQ ID NO:244), NT(1-Nal)Q (SEQ ID NO:245), NT(2-Nal)Q (SEQ ID NO:246), NTWE (SEQ ID NO:247), NTWF (SEQ ID NO:248), and NTWQ (SEQ ID NO:249).

4. The peptide inhibitor of any one of claims 1-3, or a pharmaceutically acceptable salt or solvate thereof, wherein X14 is Asn and X15 is Asn.

5. The peptide inhibitor of claim 1, or a pharmaceutically acceptable salt or solvate thereof, wherein the peptide inhibitor comprises an amino acid sequence selected from the group consisting of:
| **SEQ ID No.** | **Sequence** |
|---|---|
| 104 | Ac-[(D)Arg]-cyclo[[Abu]-QT-[Trp(5-F)]-QC]-[Phe[4-(2-aminoethoxy)]-[2-Nal]- [4-amino-4-carboxy-tetrahydropyran]-ENN-NH2 |
| 310 | Ac-[(D)Arg]-cyclo[[Abu]-QT-[Trp(5-F)]-QC]-[Phe[4-(2-aminoethoxy)]-[2-Nal]- [4-amino-4-carboxy-tetrahydropyran]-ENN-NH2 |
| 71 | Alexa488-[PEG4]-[(D)Arg]-cyclo[[Abu]-QTWQC]-[Phe[4-(2-aminoethoxy)]- [2-Nal]-[4-amino-4-carboxy-tetrahydropyran]-ENN-NH2 |
| 72 | Alexa647-[PEG4]-[(D)Arg]-cyclo[[Abu]-QTWQC]-[Phe[4-(2-aminoethoxy)]- [2-Nal]-[4-amino-4-carboxy-tetrahydropyran]-ENN-NH2 |
| 73 | Ac-[(D)Arg]-cyclo[[Abu]-QTWQC]-[Phe[4-(2-aminoethoxy)]-[2-Nal]-[4- amino-4-carboxy-tetrahydropyran]-END-NH2 |
| 74 | Ac-[(D)Arg]-cyclo[[Abu]-QTWQC]-[Phe[4-(2-aminoethoxy)]-[2-Nal]-[4- amino-4-carboxy-tetrahydropyran]-EDN-NH2 |
| 75 | Ac-[(D)Arg]-cyclo[[Abu]-QTWEC]-[Phe[4-(2-aminoethoxy)]-[2-Nal]-[4-amino- 4-carboxy-tetrahydropyran]-ENN-NH2 |
| 76 | Ac-[(D)Arg]-cyclo[[Abu]-ETWQC]-[Phe[4-(2-aminoethoxy)]-[2-Nal]-[4-amino- 4-carboxy-tetrahydropyran]-ENN-NH2 |
| 77 | Ac-[(D)Arg]-cyclo[[Abu]-QTWQC]-[Phe[4-(2-aminoethoxy)]-[2-Nal]-[4- amino-4-carboxy-tetrahydropyran]-EDD-NH2 |
| 78 | Ac-[(D)Arg]-cyclo[[Abu]-QTWEC]-[Phe[4-(2-aminoethoxy)]-[2-Nal]-[4-amino- 4-carboxy-tetrahydropyran]-END-NH2 |
| 79 | Ac-[(D)Arg]-cyclo[[Abu]-ETWQC]-[Phe[4-(2-aminoethoxy)]-[2-Nal]-[4-amino- 4-carboxy-tetrahydropyran]-END-NH2 |
| 81 | Ac-[(D)Arg]-cyclo[[Abu]-ETWQC]-[Phe[4-(2-aminoethoxy)]-[2-Nal]-[4-amino- 4-carboxy-tetrahydropyran]-EDN-NH2 |
| 82 | Ac-[(D)Arg]-cyclo[[Abu]-ETWEC]-[Phe[4-(2-aminoethoxy)]-[2-Nal]-[4-amino- 4-carboxy-tetrahydropyran]-ENN-NH2 |
| 83 | Ac-[(D)Arg]-cyclo[[Abu]-QTWQC]-[Phe[4-(2-aminoethoxy)]-[2-Nal]-[4- amino-4-carboxy-tetrahydropyran]-ENN-OH |
| 84 | Ac-[(D)Arg]-cyclo[[Abu]-QTWQC]-[Phe[4-(2-aminoethoxy)]-[2-Nal]-[4- amino-4-carboxy-tetrahydropyran]-END-OH |
| 85 | Ac-[(D)Arg]-cyclo[[Abu]-QTWQC]-[Phe[4-(2-aminoethoxy)]-[2-Nal]-[4- amino-4-carboxy-tetrahydropyran]-EDN-OH |
| 86 | Ac-[(D)Arg]-cyclo[[Abu]-QTWEC]-[Phe[4-(2-aminoethoxy)]-[2-Nal]-[4-amino- 4-carboxy-tetrahydropyran]-ENN-OH |
| 87 | [Ac-[(D)Arg]-cyclo[[Abu]-ETWQC]-[Phe[4-(2-aminoethoxy)]-[2-Nal]-[4- amino-4-carboxy-tetrahydropyran]-ENN-OH |
| 88 | [NH2-PEG4]-[(D)Arg]-cyclo[[Abu]-QTWQC]-[Phe[4-(2-aminoethoxy)]-[2- Nal]-[4-amino-4-carboxy-tetrahydropyran]-ENN-NH2 |
| 89 | [Biotin]-[PEG4]-[(D)Arg]-cyclo[[Abu]-QTWQC]-[Phe[4-(2-aminoethoxy)]-[2- Nal]-[4-amino-4-carboxy-tetrahydropyran]-ENN-NH2 |
| 93 | Ac-[(D)Arg]-cyclo[[Abu]-QTW-[α-MeLeu]-C]-[Phe[4-(2-aminoethoxy)]-[2- Nal]-[4-amino-4-carboxy-tetrahydropyran]-ENN-NH2 |
| 94 | Ac-[(D)Arg]-cyclo[[Abu]-QTW-[α-MeLys]-C]-[Phe[4-(2-aminoethoxy)]-[2- Nal]-[4-amino-4-carboxy-tetrahydropyran]-ENN-NH2 |
| 95 | Ac-[(D)Arg]-cyclo[[Abu]-QTW-[α-MeLys(Ac)]-C]-[Phe[4-(2-aminoethoxy)]- [2-Nal]-[4-amino-4-carboxy-tetrahydropyran]-ENN-NH2 |
| 96 | Ac-[(D)Arg]-cyclo[[Abu]-QTW-[(D)Gln]-C]-[Phe[4-(2-aminoethoxy)]-[2-Nal]- [4-amino-4-carboxy-tetrahydropyran]-ENN-NH2 |
| 97 | Ac-[(D)Arg]-cyclo[[Abu]-QTW-[β-homoGln]-C]-[Phe[4-(2-aminoethoxy)]-[2- Nal]-[4-amino-4-carboxy-tetrahydropyran]-ENN-NH2 |
| 99 | Ac-[(D)Arg]-cyclo[[Abu]-QTWFC]-[Phe[4-(2-aminoethoxy)]-[2-Nal]-[4-amino- 4-carboxy-tetrahydropyran]-ENN-NH2 |
| 100 | Ac-[(D)Arg]-cyclo[[Abu]-QTWWC]-[Phe[4-(2-aminoethoxy)]-[2-Nal]-[4- amino-4-carboxy-tetrahydropyran]-ENN-NH2 |
| 101 | Ac-[(D)Arg]-cyclo[[Abu]-QTW-[Aib]-C]-[Phe[4-(2-aminoethoxy)]-[2-Nal]-[4- amino-4-carboxy-tetrahydropyran]-ENN-NH2 |
| 102 | Ac-[(D)Arg]-cyclo[[Abu]-QTWTC]-[Phe[4-(2-aminoethoxy)]-[2-Nal]-[4-amino- 4-carboxy-tetrahydropyran]-ENN-NH2 |
| 103 | Ac-[(D)Arg]-cyclo[[Abu]-QTWVC]-[Phe[4-(2-aminoethoxy)]-[2-Nal]-[4- amino-4-carboxy-tetrahydropyran]-ENN-NH2 |
| 108 | Ac-[(D)Arg]-cyclo[[Abu]-QTW-[2-Nal]-C]-[Phe[4-(2-aminoethoxy)]-[2-Nal]- [4-amino-4-carboxy-tetrahydropyran]-ENN-NH2 |
| 109 | Ac-[(D)Arg]-cyclo[[Abu]-QTW-[1-Nal]-C]-[Phe[4-(2-aminoethoxy)]-[2-Nal]- [4-amino-4-carboxy-tetrahydropyran]-ENN-NH2 |
| 308 | Ac-[(D)Arg]-cyclo[[Abu]-QT-[2-Nal]-QC]-[Phe[4-(2-aminoethoxy)]-[2-Nal]-[4- amino-4-carboxy-tetrahydropyran]-ENN-NH2 |
| 309 | Ac-[(D)Arg]-cyclo[[Abu]-QT-[1-Nal]-QC]-[Phe[4-(2-aminoethoxy)]-[2-Nal]-[4- amino-4-carboxy-tetrahydropyran]-ENN-NH2 |
| 139 | Ac-[(D)Arg]-cyclo[[Abu]-QTW-[2-Nal]-C]-[Phe[4-(2-aminoethoxy)]-[2-Nal]- [4-amino-4-carboxy-tetrahydropyran]-ENN-NH2 |
| 140 | Ac-[(D)Arg]-cyclo[[Abu]-QTW-[1-Nal]-C]-[Phe[4-(2-aminoethoxy)]-[2-Nal]- [4-amino-4-carboxy-tetrahydropyran]-ENN-NH2 |
| 1 | Ac-[(D)Arg]-[Pen]-QTWQ-[Pen]-[Phe[4-(2-aminoethoxy)]-[2-Nal]-[4-amino-4- carboxy-tetrahydropyran]-ENN-NH2 |

6. The peptide inhibitor of any one of claims 1-5, or a pharmaceutically acceptable salt or solvate thereof, further comprising one or more half-life extension moiety and/or one or more linker moiety conjugated to the peptide inhibitor.

7. The peptide inhibitor of claim 6, or a pharmaceutically acceptable salt or solvate thereof, wherein the half-life extension moiety is conjugated to the peptide inhibitor via one or more linker moieties.

8. The peptide inhibitor of any one of claims 1-7, wherein the peptide inhibitor comprises the structure of Formula (Z):
R¹-X-R² (Z)
or a pharmaceutically acceptable salt or solvate thereof, wherein
R¹ is a bond, hydrogen, a C1-C6 alkyl, a C6-C12 aryl, a C6-C12 aryl, a C1-C6 alkyl, a C1-C20 alkanoyl, and including PEGylated versions alone or as spacers of any of the foregoing; X is the amino acid sequence of Formula (IVa) or Formula (IIIa); and R² is OH or NH₂.

9. A polynucleotide comprising a sequence encoding the peptide inhibitor of any one of claims 1-8.

10. A pharmaceutical composition comprising the peptide inhibitor of any one of claims 1-8, or a pharmaceutically acceptable salt or solvate thereof, and a pharmaceutically acceptable carrier, excipient, or diluent.

11. The pharmaceutical composition of claim 10, further comprising an enteric coating, optionally wherein the enteric coating protects and releases the pharmaceutical composition within a subject's lower gastrointestinal system.

12. The peptide inhibitor of any one of claims 1-8, or a pharmaceutically acceptable salt or solvate thereof, or the pharmaceutical composition of claim 10 or claim 11, for use in a method for treating an Inflammatory Bowel Disease (IBD), ulcerative colitis, Crohn's disease, Celiac disease (*nontropical Sprue)*, enteropathy associated with seronegative arthropathies, microscopic colitis, collagenous colitis, eosinophilic gastroenteritis, colitis associated with radio- or chemo-therapy, colitis associated with disorders of innate immunity as in leukocyte adhesion deficiency-1, chronic granulomatous disease, glycogen storage disease type 1b, Hermansky-Pudlak syndrome, Chediak-Higashi syndrome, and Wiskott-Aldrich Syndrome, pouchitis resulting after proctocolectomy and ileoanal anastomosis, gastrointestinal cancer, pancreatitis, insulin-dependent diabetes mellitus, mastitis, cholecystitis, cholangitis, pericholangitis, chronic bronchitis, chronic sinusitis, asthma, psoriasis, psoriatic arthritis, or graft versus host disease in a subject, the method comprising providing to the subject an effective amount of the peptide inhibitor, salt, solvate, or pharmaceutical composition.

13. The pharmaceutical composition for use according to claim 12, wherein the pharmaceutical composition is provided to the subject by an oral, parenteral, intravenous, peritoneal, intradermal, subcutaneous, intramuscular, intrathecal, inhalation, vaporization, nebulization, sublingual, buccal, parenteral, rectal, intraocular, inhalation, topically, vaginal, or topical route of administration.

14. The pharmaceutical composition for use according to claim 12, wherein the method is a method for treating Inflammatory Bowel Disease (IBD), ulcerative colitis, Crohn's disease, wherein the pharmaceutical composition is provided to the subject orally.

15. The pharmaceutical composition for use according to claim 12, wherein the method is a method for treating psoriasis, wherein the pharmaceutical composition is provided to the subject orally, topically, parenterally, intravenously, subcutaneously, peritoneally, or intravenously.
